(19) Europäisches Patentamt
European Patent Office
Office européen des brevets

(11) EP 3 402 503 B1

(12) EUROPEAN PATENT SPECIFICATION

(45) Date of publication and mention of the grant of the patent:
**21.10.2020 Bulletin 2020/43**

(21) Application number: **17701596.3**

(22) Date of filing: **13.01.2017**

(51) Int Cl.:
*A61K 38/00* (2006.01) *A61K 39/00* (2006.01)
*A61P 35/00* (2006.01) *A61K 31/522* (2006.01)
*A61K 31/52* (2006.01) *A61K 31/519* (2006.01)
*A61K 31/517* (2006.01) *A61K 31/4985* (2006.01)
*A61K 31/454* (2006.01) *A61K 45/06* (2006.01)
*A61P 19/02* (2006.01)

(86) International application number:
**PCT/IB2017/050198**

(87) International publication number:
**WO 2017/122175 (20.07.2017 Gazette 2017/29)**

# (54) THERAPEUTIC COMBINATIONS OF AN ANTIFOLATE AND A BTK INHIBITOR

THERAPEUTISCHE KOMBINATIONEN AUS EINEM ANTIFOLAT UND EINEM BTK-HEMMER

ASSOCIATIONS THÉRAPEUTIQUES D’UN ANTIFOLATE ET D’UN INHIBITEUR DE BTK

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.01.2016 US 201662278374 P**
**05.08.2016 US 201662371626 P**

(43) Date of publication of application:
**21.11.2018 Bulletin 2018/47**

(73) Proprietor: **Acerta Pharma B.V.**
**5349 AB Oss (NL)**

(72) Inventors:
- **KAPTEIN, Allard**
**5301 HB Zaltbommel (NL)**
- **ROTHBAUM, Wayne**
**Delray Beach, Florida 33483 (US)**
- **LANNUTTI, Brian**
**Solana Beach, California 92075 (US)**

(74) Representative: **D Young & Co LLP**
**120 Holborn**
**London EC1N 2DY (GB)**

(56) References cited:
**EP-A1- 2 786 996**
**WO-A1-2013/010868**
**WO-A1-2015/061752**
**WO-A2-2015/110923**
**US-A1- 2014 256 734**

- **D. XU ET AL: "RN486, a Selective Bruton’s Tyrosine Kinase Inhibitor, Abrogates Immune Hypersensitivity Responses and Arthritis in Rodents", JOURNAL OF PHARMACOLOGY AND EXPERIMENTAL THERAPEUTICS, vol. 341, no. 1, 6 January 2012 (2012-01-06), pages 90-103, XP055227121, DOI: 10.1124/jpet.111.187740**
- **Clinical trials: "A Phase 2a, 4-Week, Double-Blind, Proof-of-Concept Efficacy and Safety Study of ACP-196 Versus Placebo in Subjects With Active Rheumatoid Arthritis on Background Methotrexate", clinicaltrials.gov , 4 November 2015 (2015-11-04), XP002768714, Retrieved from the Internet: URL:https://clinicaltrials.gov/archive/NCT02387762/2015_11_04 [retrieved on 2016-03-27]**
- **AstraZeneca: "Majority-stake investment in Acerta Pharma", astrazeneca.com , 17 December 2015 (2015-12-17), XP002768715, Retrieved from the Internet: URL:https://www.astrazeneca.com/content/dam/az/PDF/Acerta_Investor_conference_call_17_December_2015.pdf [retrieved on 2016-03-27]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 402 503 B1

- DATABASE REGISTRY [Online] CHEMICAL ABSTRACTS SERVICE, COLUMBUS, OHIO, US; XP002764066, retrieved from REGISTRY; STN Database accession no. 1420477-60-6
- GIORGIO V SCAGLIOTTI ET AL: "Antimetabolites and cancer: emerging data with a focus on antifolates", EXPERT OPINION ON THERAPEUTIC PATENTS, vol. 16, no. 2, 1 February 2006 (2006-02-01), pages 189-200, XP055141364, ISSN: 1354-3776, DOI: 10.1517/13543776.16.2.189

SEQUENCE LISTING

<110> Acerta Pharma B.V.

<120> THERAPEUTIC COMBINATIONS OF AN ANTIFOLATE AND A BTK INHIBITOR

<130> 055112-5038-WO

<140> Filed herewith
<141> 2017-01-13

<160> 68

<170> PatentIn version 3.5

<210> 1
<211> 451
<212> PRT
<213> Artificial Sequence

<220>
<223> rituximab heavy chain

<400> 1

```
Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser Ala Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160
```

```
Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
```

405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 2
<211> 213
<212> PRT
<213> Artificial Sequence

<220>
<223> rituximab light chain

<400> 2

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Ile
20 25 30

His Trp Phe Gln Gln Lys Pro Gly Ser Ser Pro Lys Pro Trp Ile Tyr
35 40 45

Ala Thr Ser Asn Leu Ala Ser Gly Val Pro Val Arg Phe Ser Gly Ser
50 55 60

Gly Ser Gly Thr Ser Tyr Ser Leu Thr Ile Ser Arg Val Glu Ala Glu
65 70 75 80

Asp Ala Ala Thr Tyr Tyr Cys Gln Gln Trp Thr Ser Asn Pro Pro Thr
85 90 95

Phe Gly Gly Gly Thr Lys Leu Glu Ile Lys Arg Thr Val Ala Ala Pro
100 105 110

Ser Val Phe Ile Phe Pro Pro Ser Asp Glu Gln Leu Lys Ser Gly Thr
115 120 125

Ala Ser Val Val Cys Leu Leu Asn Asn Phe Tyr Pro Arg Glu Ala Lys
130 135 140

Val Gln Trp Lys Val Asp Asn Ala Leu Gln Ser Gly Asn Ser Gln Glu
145 150 155 160

Ser Val Thr Glu Gln Asp Ser Lys Asp Ser Thr Tyr Ser Leu Ser Ser
165 170 175

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn Arg Gly Glu Cys
210

<210> 3
<211> 449
<212> PRT
<213> Artificial Sequence

<220>

<223> obinutuzumab heavy chain

<400> 3

```
Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140
```

```
Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400
```

```
Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys
```

<210> 4
<211> 219
<212> PRT
<213> Artificial Sequence

<220>
<223> obinutuzumab light chain

<400> 4

```
Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140
```

```
Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

<210> 5
<211> 122
<212> PRT
<213> Artificial Sequence

<220>
<223> ofatumumab variable heavy chain

<400> 5

```
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
    50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser
        115                 120
```

<210> 6
<211> 107
<212> PRT
<213> Artificial Sequence

<220>
<223> ofatumumab variable light chain

<400> 6

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Ile
                85                  90                  95

Thr Phe Gly Gln Gly Thr Arg Leu Glu Ile Lys
            100                 105
```

<210> 7
<211> 222
<212> PRT
<213> Artificial Sequence

<220>
<223> ofatumumab Fab fragment heavy chain

<400> 7

```
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asn Asp Tyr
            20                  25                  30

Ala Met His Trp Val Arg Gln Ala Pro Gly Lys Gly Leu Glu Trp Val
        35                  40                  45

Ser Thr Ile Ser Trp Asn Ser Gly Ser Ile Gly Tyr Ala Asp Ser Val
    50                  55                  60

Lys Gly Arg Phe Thr Ile Ser Arg Asp Asn Ala Lys Lys Ser Leu Tyr
65                  70                  75                  80

Leu Gln Met Asn Ser Leu Arg Ala Glu Asp Thr Ala Leu Tyr Tyr Cys
                85                  90                  95

Ala Lys Asp Ile Gln Tyr Gly Asn Tyr Tyr Tyr Gly Met Asp Val Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Gly Ser Ser Lys Ser Thr Ser Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro
    210                 215                 220
```

<210> 8
<211> 211
<212> PRT
<213> Artificial Sequence

<220>

<223> ofatumumab Fab fragment light chain

<400> 8

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Ile
                85                  90                  95

Thr Phe Gly Gln Gly Thr Arg Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg
    210
```

<210> 9
<211> 451

<212> PRT
<213> Artificial Sequence

<220>
<223> veltuzumab heavy chain

<400> 9

```
Gln Val Gln Leu Gln Gln Ser Gly Ala Glu Val Lys Lys Pro Gly Ser
1               5                   10                  15

Ser Val Lys Val Ser Cys Lys Ala Ser Gly Tyr Thr Phe Thr Ser Tyr
            20                  25                  30

Asn Met His Trp Val Lys Gln Ala Pro Gly Gln Gly Leu Glu Trp Ile
```

```
        35                  40                  45

Gly Ala Ile Tyr Pro Gly Met Gly Asp Thr Ser Tyr Asn Gln Lys Phe
    50                  55                  60

Lys Gly Lys Ala Thr Leu Thr Ala Asp Glu Ser Thr Asn Thr Ala Tyr
65                  70                  75                  80

Met Glu Leu Ser Ser Leu Arg Ser Glu Asp Thr Ala Phe Tyr Tyr Cys
                85                  90                  95

Ala Arg Ser Thr Tyr Tyr Gly Gly Asp Trp Tyr Phe Asp Val Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285
```

```
His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450
```

<210> 10
<211> 213
<212> PRT
<213> Artificial Sequence

<220>
<223> veltuzumab light chain

<400> 10

```
Asp Ile Gln Leu Thr Gln Ser Pro Ser Ser Leu Ser Ala Ser Val Gly
1               5                   10                  15

Asp Arg Val Thr Met Thr Cys Arg Ala Ser Ser Ser Val Ser Tyr Ile
            20                  25                  30

His Trp Phe Gln Gln Lys Pro Gly Lys Ala Pro Lys Pro Trp Ile Tyr


        35                  40                  45

Ala Thr Ser Asn Leu Ala Ser Gly Val Pro Val Arg Phe Ser Gly Ser
    50                  55                  60

Gly Ser Gly Thr Asp Tyr Thr Phe Thr Ile Ser Ser Leu Gln Pro Glu
65                  70                  75                  80

Asp Ile Ala Thr Tyr Tyr Cys Gln Gln Trp Thr Ser Asn Pro Pro Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210
```

<210> 11
<211> 447
<212> PRT
<213> Artificial Sequence

<220>
<223> tositumomab heavy chain

<400> 11

```
Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30
```

```
Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Ala Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
```

275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Pro Ala Pro Ile Glu Lys Thr Ile
325 330 335

Ser Lys Ala Lys Gly Gln Pro Arg Glu Pro Gln Val Tyr Thr Leu Pro
340 345 350

Pro Ser Arg Asp Glu Leu Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 12
<211> 210
<212> PRT
<213> Artificial Sequence

<220>
<223> ositumomab light chain

<400> 12

```
Gln Ile Val Leu Ser Gln Ser Pro Ala Ile Leu Ser Ala Ser Pro Gly
1               5                   10                  15

Glu Lys Val Thr Met Thr Cys Arg Ala Ser Ser Ser Val Ser Tyr Met
            20                  25                  30

His Trp Tyr Gln Gln Lys Pro Gly Ser Ser Pro Lys Pro Trp Ile Tyr
        35                  40                  45

Ala Pro Ser Asn Leu Ala Ser Gly Val Pro Ala Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Ser Phe Asn Pro Pro Thr
                85                  90                  95

Phe Gly Ala Gly Thr Lys Leu Glu Leu Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg
    210
```

<210> 13
<211> 443
<212> PRT
<213> Artificial Sequence

<220>

<223> ibritumomab heavy chain

<400> 13

```
Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30
```

```
Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Ala Pro Ser Val Tyr Pro Leu
        115                 120                 125

Ala Pro Val Cys Gly Asp Thr Thr Gly Ser Ser Val Thr Leu Gly Cys
    130                 135                 140

Leu Val Lys Gly Tyr Phe Pro Glu Pro Val Thr Leu Thr Trp Asn Ser
145                 150                 155                 160

Gly Ser Leu Ser Ser Gly Val His Thr Phe Pro Ala Val Leu Gln Ser
                165                 170                 175

Asp Leu Tyr Thr Leu Ser Ser Ser Val Thr Val Thr Ser Ser Thr Trp
            180                 185                 190

Pro Ser Gln Ser Ile Thr Cys Asn Val Ala His Pro Ala Ser Ser Thr
        195                 200                 205

Lys Val Asp Lys Lys Ile Glu Pro Arg Gly Pro Thr Ile Lys Pro Cys
    210                 215                 220

Pro Pro Cys Lys Cys Pro Ala Pro Asn Leu Leu Gly Gly Pro Ser Val
225                 230                 235                 240

Phe Ile Phe Pro Pro Lys Ile Lys Asp Val Leu Met Ile Ser Leu Ser
                245                 250                 255

Pro Ile Val Thr Cys Val Val Val Asp Val Ser Glu Asp Asp Pro Asp
            260                 265                 270

Val Gln Ile Ser Trp Phe Val Asn Asn Val Glu Val His Thr Ala Gln
        275                 280                 285
```

Thr Gln Thr His Arg Glu Asp Tyr Asn Ser Thr Leu Arg Val Val Ser
290 295 300

Ala Leu Pro Ile Gln His Gln Asp Trp Met Ser Gly Lys Glu Phe Lys
305 310 315 320

Cys Lys Val Asn Asn Lys Asp Leu Pro Ala Pro Ile Glu Arg Thr Ile
325 330 335

Ser Lys Pro Lys Gly Ser Val Arg Ala Pro Gln Val Tyr Val Leu Pro
340 345 350

Pro Pro Glu Glu Glu Met Thr Lys Lys Gln Val Thr Leu Thr Cys Met
355 360 365

Val Thr Asp Phe Met Pro Glu Asp Ile Tyr Val Glu Trp Thr Asn Asn
370 375 380

Gly Lys Thr Glu Leu Asn Tyr Lys Asn Thr Glu Pro Val Leu Asp Ser
385 390 395 400

Asp Gly Ser Tyr Phe Met Tyr Ser Lys Leu Arg Val Glu Lys Lys Asn
405 410 415

Trp Val Glu Arg Asn Ser Tyr Ser Cys Ser Val Val His Glu Gly Leu
420 425 430

His Asn His His Thr Thr Lys Ser Phe Ser Arg
435 440

<210> 14
<211> 209
<212> PRT
<213> Artificial Sequence

<220>
<223> ibritumomab light chain

<400> 14

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Met
20 25 30

His Trp Tyr Gln Gln Lys Pro Gly Ser Ser Pro Lys Pro Trp Ile Tyr
35 40 45

Ala Pro Ser Asn Leu Ala Ser Gly Val Pro Ala Arg Phe Ser Gly Ser
50 55 60

Gly Ser Gly Thr Ser Tyr Ser Leu Thr Ile Ser Arg Val Glu Ala Glu
65 70 75 80

Asp Ala Ala Thr Tyr Tyr Cys Gln Gln Trp Ser Phe Asn Pro Pro Thr
85 90 95

Phe Gly Ala Gly Thr Lys Leu Glu Leu Lys Arg Ala Asp Ala Ala Pro
100 105 110

Thr Val Phe Ile Phe Pro Pro Ser Asp Glu Gln Leu Lys Ser Gly Thr
115 120 125

Ala Ser Val Val Cys Leu Leu Asn Asn Phe Tyr Pro Arg Glu Ala Lys
130 135 140

Val Gln Trp Lys Val Asp Asn Ala Leu Gln Ser Gly Asn Ser Gln Glu
145 150 155 160

Ser Val Thr Glu Gln Asp Ser Lys Asp Ser Thr Tyr Ser Leu Ser Ser
165 170 175

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn

<210> 15
<211> 440
<212> PRT
<213> Artificial Sequence

<220>
<223> nivolumab heavy chain

<400> 15

Gln Val Gln Leu Val Glu Ser Gly Gly Gly Val Val Gln Pro Gly Arg
1 5 10 15

Ser Leu Arg Leu Asp Cys Lys Ala Ser Gly Ile Thr Phe Ser Asn Ser
20 25 30

```
Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285
```

```
Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Lys
        435                 440
```

<210> 16
<211> 214
<212> PRT
<213> Artificial Sequence

<220>
<223> nivolumab light chain

<400> 16

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45
```

```
Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 17
<211> 113
<212> PRT
<213> Artificial Sequence

<220>
<223> nivolumab variable heavy chain

<400> 17

```
Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val

        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser
```

<210> 18
<211> 107
<212> PRT
<213> Artificial Sequence

<220>
<223> nivolumab variable light chain

<400> 18

Glu Ile Val Leu Thr Gln Ser Pro Ala Thr Leu Ser Leu Ser Pro Gly
1 5 10 15

Glu Arg Ala Thr Leu Ser Cys Arg Ala Ser Gln Ser Val Ser Ser Tyr
20 25 30

Leu Ala Trp Tyr Gln Gln Lys Pro Gly Gln Ala Pro Arg Leu Leu Ile
35 40 45

Tyr Asp Ala Ser Asn Arg Ala Thr Gly Ile Pro Ala Arg Phe Ser Gly
50 55 60

Ser Gly Ser Gly Thr Asp Phe Thr Leu Thr Ile Ser Ser Leu Glu Pro
65 70 75 80

Glu Asp Phe Ala Val Tyr Tyr Cys Gln Gln Ser Ser Asn Trp Pro Arg
85 90 95

Thr Phe Gly Gln Gly Thr Lys Val Glu Ile Lys
100 105

<210> 19
<211> 5
<212> PRT
<213> Artificial Sequence

<220>
<223> nivolumab heavy chain CDR1

<400> 19

Asn Ser Gly Met His
1 5

<210> 20
<211> 17
<212> PRT
<213> Artificial Sequence

<220>
<223> nivolumab heavy chain CDR2

<400> 20

Val Ile Trp Tyr Asp Gly Ser Lys Arg Tyr Tyr Ala Asp Ser Val Lys
1 5 10 15

Gly

<210> 21
<211> 4
<212> PRT

<213> Artificial Sequence

<220>
<223> nivolumab heavy chain CDR3

<400> 21

Asn Asp Asp Tyr
1

<210> 22
<211> 11
<212> PRT
<213> Artificial Sequence

<220>
<223> nivolumab light chain CDR1

<400> 22

Arg Ala Ser Gln Ser Val Ser Ser Tyr Leu Ala
1 5 10

<210> 23
<211> 7
<212> PRT
<213> Artificial Sequence

<220>
<223> nivolumab light chain CDR2

<400> 23

Asp Ala Ser Asn Arg Ala Thr
1 5

<210> 24
<211> 9
<212> PRT
<213> Artificial Sequence

<220>
<223> nivolumab light chain CDR3

<400> 24

Gln Gln Ser Ser Asn Trp Pro Arg Thr
1 5

<210> 25
<211> 447
<212> PRT
<213> Artificial Sequence

<220>
<223> pembrolizumab heavy chain

<400> 25

```
Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Met Tyr Trp Val Arg Gln Ala Pro Gly Gln Gly Leu Glu Trp Met
        35                  40                  45

Gly Gly Ile Asn Pro Ser Asn Gly Gly Thr Asn Phe Asn Glu Lys Phe
    50                  55                  60

Lys Asn Arg Val Thr Leu Thr Thr Asp Ser Ser Thr Thr Thr Ala Tyr
65                  70                  75                  80

Met Glu Leu Lys Ser Leu Gln Phe Asp Asp Thr Ala Val Tyr Tyr Cys
                85                  90                  95

Ala Arg Arg Asp Tyr Arg Phe Asp Met Gly Phe Asp Tyr Trp Gly Gln
            100                 105                 110

Gly Thr Thr Val Thr Val Ser Ser Ala Ser Thr Lys Gly Pro Ser Val
        115                 120                 125
```

```
Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
```

370 375 380

Gly Gln Pro Glu Asn Asn Tyr Lys Thr Thr Pro Pro Val Leu Asp Ser
385 390 395 400

Asp Gly Ser Phe Phe Leu Tyr Ser Arg Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Leu Gly Lys
435 440 445

<210> 26
<211> 218
<212> PRT
<213> Artificial Sequence

<220>
<223> pembrolizumab light chain

<400> 26

```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg
                85                  90                  95

Asp Leu Pro Leu Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 27
<211> 120
<212> PRT
<213> Artificial Sequence

<220>
<223> pembrolizumab variable heavy chain

<400> 27

```
Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Met Tyr Trp Val Arg Gln Ala Pro Gly Gln Gly Leu Glu Trp Met
        35                  40                  45

Gly Gly Ile Asn Pro Ser Asn Gly Gly Thr Asn Phe Asn Glu Lys Phe
    50                  55                  60

Lys Asn Arg Val Thr Leu Thr Thr Asp Ser Ser Thr Thr Thr Ala Tyr
65                  70                  75                  80

Met Glu Leu Lys Ser Leu Gln Phe Asp Asp Thr Ala Val Tyr Tyr Cys
                85                  90                  95

Ala Arg Arg Asp Tyr Arg Phe Asp Met Gly Phe Asp Tyr Trp Gly Gln
            100                 105                 110

Gly Thr Thr Val Thr Val Ser Ser
        115                 120
```

<210> 28
<211> 111
<212> PRT
<213> Artificial Sequence

<220>
<223> pembrolizumab variable light chain

<400> 28

Glu Ile Val Leu Thr Gln Ser Pro Ala Thr Leu Ser Leu Ser Pro Gly
1 5 10 15

Glu Arg Ala Thr Leu Ser Cys Arg Ala Ser Lys Gly Val Ser Thr Ser
20 25 30

Gly Tyr Ser Tyr Leu His Trp Tyr Gln Gln Lys Pro Gly Gln Ala Pro
35 40 45

Arg Leu Leu Ile Tyr Leu Ala Ser Tyr Leu Glu Ser Gly Val Pro Ala
50 55 60

Arg Phe Ser Gly Ser Gly Ser Gly Thr Asp Phe Thr Leu Thr Ile Ser
65 70 75 80

Ser Leu Glu Pro Glu Asp Phe Ala Val Tyr Tyr Cys Gln His Ser Arg
85 90 95

Asp Leu Pro Leu Thr Phe Gly Gly Gly Thr Lys Val Glu Ile Lys
100 105 110

<210> 29
<211> 5
<212> PRT
<213> Artificial Sequence

<220>
<223> pembrolizumab heavy chain CDR1

<400> 29

Asn Tyr Tyr Met Tyr
1 5

<210> 30
<211> 16
<212> PRT
<213> Artificial Sequence

<220>
<223> pembrolizumab heavy chain CDR2

<400> 30

Gly Ile Asn Pro Ser Asn Gly Gly Thr Asn Phe Asn Glu Lys Phe Lys
1 5 10 15

<210> 31
<211> 11
<212> PRT
<213> Artificial Sequence

<220>

<223> pembrolizumab heavy chain CDR3

<400> 31

```
Arg Asp Tyr Arg Phe Asp Met Gly Phe Asp Tyr
1               5                   10
```

<210> 32
<211> 15
<212> PRT
<213> Artificial Sequence

<220>
<223> pembrolizumab light chain CDR1

<400> 32

```
Arg Ala Ser Lys Gly Val Ser Thr Ser Gly Tyr Ser Tyr Leu His
1               5                   10                  15
```

<210> 33
<211> 7
<212> PRT
<213> Artificial Sequence

<220>
<223> pembrolizumab light chain CDR2

<400> 33

```
Leu Ala Ser Tyr Leu Glu Ser
1               5
```

<210> 34
<211> 9
<212> PRT
<213> Artificial Sequence

<220>
<223> pembrolizumab light chain CDR3

<400> 34

```
Gln His Ser Arg Asp Leu Pro Leu Thr
1               5
```

<210> 35
<211> 447
<212> PRT
<213> Artificial Sequence

<220>
<223> pidilizumab heavy chain

<400> 35

```
Gln Val Gln Leu Val Gln Ser Gly Ser Glu Leu Lys Lys Pro Gly Ala
1               5                   10                  15

Ser Val Lys Ile Ser Cys Lys Ala Ser Gly Tyr Thr Phe Thr Asn Tyr
            20                  25                  30

Gly Met Asn Trp Val Arg Gln Ala Pro Gly Gln Gly Leu Gln Trp Met
        35                  40                  45

Gly Trp Ile Asn Thr Asp Ser Gly Glu Ser Thr Tyr Ala Glu Glu Phe
    50                  55                  60

Lys Gly Arg Phe Val Phe Ser Leu Asp Thr Ser Val Asn Thr Ala Tyr
65                  70                  75                  80

Leu Gln Ile Thr Ser Leu Thr Ala Glu Asp Thr Gly Met Tyr Phe Cys
                85                  90                  95

Val Arg Val Gly Tyr Asp Ala Leu Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220
```

Thr Cys Pro Pro Cys Pro Ala Pro Glu Leu Leu Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Pro Ala Pro Ile Glu Lys Thr Ile
325 330 335

Ser Lys Ala Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 36
<211> 213
<212> PRT
<213> Artificial Sequence

<220>

<223> pidilizumab light chain

<400> 36

```
Glu Ile Val Leu Thr Gln Ser Pro Ser Ser Leu Ser Ala Ser Val Gly
1               5                   10                  15

Asp Arg Val Thr Ile Thr Cys Ser Ala Arg Ser Ser Val Ser Tyr Met
            20                  25                  30

His Trp Phe Gln Gln Lys Pro Gly Lys Ala Pro Lys Leu Trp Ile Tyr
        35                  40                  45

Arg Thr Ser Asn Leu Ala Ser Gly Val Pro Ser Arg Phe Ser Gly Ser
    50                  55                  60

Gly Ser Gly Thr Ser Tyr Cys Leu Thr Ile Asn Ser Leu Gln Pro Glu
65                  70                  75                  80

Asp Phe Ala Thr Tyr Tyr Cys Gln Gln Arg Ser Ser Phe Pro Leu Thr
                85                  90                  95

Phe Gly Gly Gly Thr Lys Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210
```

<210> 37
<211> 117

<212> PRT
<213> Artificial Sequence

<220>
<223> pidilizumab variable heavy chain

<400> 37

```
Gln Val Gln Leu Val Gln Ser Gly Ser Glu Leu Lys Lys Pro Gly Ala
1               5                   10                  15

Ser Val Lys Ile Ser Cys Lys Ala Ser Gly Tyr Thr Phe Thr Asn Tyr
            20                  25                  30

Gly Met Asn Trp Val Arg Gln Ala Pro Gly Gln Gly Leu Gln Trp Met
        35                  40                  45

Gly Trp Ile Asn Thr Asp Ser Gly Glu Ser Thr Tyr Ala Glu Glu Phe
    50                  55                  60

Lys Gly Arg Phe Val Phe Ser Leu Asp Thr Ser Val Asn Thr Ala Tyr
65                  70                  75                  80

Leu Gln Ile Thr Ser Leu Thr Ala Glu Asp Thr Gly Met Tyr Phe Cys
                85                  90                  95

Val Arg Val Gly Tyr Asp Ala Leu Asp Tyr Trp Gly Gln Gly Thr Leu
            100                 105                 110

Val Thr Val Ser Ser
        115
```

<210> 38
<211> 106
<212> PRT
<213> Artificial Sequence

<220>
<223> pidilizumab variable light chain

<400> 38

```
Glu Ile Val Leu Thr Gln Ser Pro Ser Ser Leu Ser Ala Ser Val Gly
1               5                   10                  15

Asp Arg Val Thr Ile Thr Cys Ser Ala Arg Ser Ser Val Ser Tyr Met
            20                  25                  30

His Trp Phe Gln Gln Lys Pro Gly Lys Ala Pro Lys Leu Trp Ile Tyr
        35                  40                  45

Arg Thr Ser Asn Leu Ala Ser Gly Val Pro Ser Arg Phe Ser Gly Ser
    50                  55                  60

Gly Ser Gly Thr Ser Tyr Cys Leu Thr Ile Asn Ser Leu Gln Pro Glu
65                  70                  75                  80

Asp Phe Ala Thr Tyr Tyr Cys Gln Gln Arg Ser Ser Phe Pro Leu Thr
                85                  90                  95

Phe Gly Gly Gly Thr Lys Leu Glu Ile Lys
            100                 105
```

<210> 39
<211> 451
<212> PRT
<213> Artificial Sequence

<220>
<223> durvalumab (MEDI4736) heavy chain

<400> 39

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Trp Phe Gly Glu Leu Ala Phe Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160
```

```
Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Phe Glu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Ser Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415
```

```
Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450
```

<210> 40
<211> 265
<212> PRT
<213> Artificial Sequence

<220>
<223> durvalumab (MEDI4736) light chain

<400> 40

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val
        35                  40                  45

Ala Asn Glu Ile Val Leu Thr Gln Ser Pro Gly Thr Leu Ser Leu Ser
    50                  55                  60

Pro Gly Glu Arg Ala Thr Leu Ser Cys Arg Ala Ser Gln Arg Val Ser
65                  70                  75                  80

Ser Ser Tyr Leu Ala Trp Tyr Gln Gln Lys Pro Gly Gln Ala Pro Arg
                85                  90                  95

Leu Leu Ile Tyr Asp Ala Ser Ser Arg Ala Thr Gly Ile Pro Asp Arg
            100                 105                 110

Phe Ser Gly Ser Gly Ser Gly Thr Asp Phe Thr Leu Thr Ile Ser Arg
        115                 120                 125

Leu Glu Pro Glu Asp Phe Ala Val Tyr Tyr Cys Gln Gln Tyr Gly Ser
    130                 135                 140

Leu Pro Trp Thr Phe Gly Gln Gly Thr Lys Val Glu Ile Lys Arg Thr
145                 150                 155                 160
```

```
Val Ala Ala Pro Ser Val Phe Ile Phe Pro Pro Ser Asp Glu Gln Leu
                165                 170                 175

Lys Ser Gly Thr Ala Ser Val Val Cys Leu Leu Asn Asn Phe Tyr Pro
            180                 185                 190

Arg Glu Ala Lys Val Gln Trp Lys Val Asp Asn Ala Leu Gln Ser Gly
        195                 200                 205

Asn Ser Gln Glu Ser Val Thr Glu Gln Asp Ser Lys Asp Ser Thr Tyr
    210                 215                 220

Ser Leu Ser Ser Thr Leu Thr Leu Ser Lys Ala Asp Tyr Glu Lys His
225                 230                 235                 240

Lys Val Tyr Ala Cys Glu Val Thr His Gln Gly Leu Ser Ser Pro Val
                245                 250                 255

Thr Lys Ser Phe Asn Arg Gly Glu Cys
            260                 265
```

<210> 41
<211> 121
<212> PRT
<213> Artificial Sequence

<220>
<223> durvalumab (MEDI4736) variable heavy chain

<400> 41

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Glu Gly Gly Trp Phe Gly Glu Leu Ala Phe Asp Tyr Trp Gly

                100                 105                 110

    Gln Gly Thr Leu Val Thr Val Ser Ser
            115                 120
```

<210> 42
<211> 108
<212> PRT
<213> Artificial Sequence

<220>
<223> durvalumab (MEDI4736) variable light chain

<400> 42

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Arg Val Ser Ser Ser
20 25 30

Tyr Leu Ala Trp Tyr Gln Gln Lys Pro Gly Gln Ala Pro Arg Leu Leu
35 40 45

Ile Tyr Asp Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Tyr Gly Ser Leu Pro
85 90 95

Trp Thr Phe Gly Gln Gly Thr Lys Val Glu Ile Lys
100 105

<210> 43
<211> 5
<212> PRT
<213> Artificial Sequence

<220>
<223> durvalumab (MEDI4736) heavy chain CDR1

<400> 43

Arg Tyr Trp Met Ser
1 5

<210> 44
<211> 17
<212> PRT
<213> Artificial Sequence

<220>
<223> durvalumab (MEDI4736) heavy chain CDR2

<400> 44

Asn Ile Lys Gln Asp Gly Ser Glu Lys Tyr Tyr Val Asp Ser Val Lys
1 5 10 15

Gly

<210> 45
<211> 12
<212> PRT

<213> Artificial Sequence

<220>
<223> durvalumab (MEDI4736) heavy chain CDR3

<400> 45

```
Glu Gly Gly Trp Phe Gly Glu Leu Ala Phe Asp Tyr
1               5                   10
```

<210> 46
<211> 12
<212> PRT
<213> Artificial Sequence

<220>
<223> durvalumab (MEDI4736) light chain CDR1

<400> 46

```
Arg Ala Ser Gln Arg Val Ser Ser Ser Tyr Leu Ala
1               5                   10
```

<210> 47
<211> 7
<212> PRT
<213> Artificial Sequence

<220>
<223> durvalumab (MEDI4736) light chain CDR2

<400> 47

```
Asp Ala Ser Ser Arg Ala Thr
1               5
```

<210> 48
<211> 9
<212> PRT
<213> Artificial Sequence

<220>
<223> durvalumab (MEDI4736) light chain CDR3

<400> 48

```
Gln Gln Tyr Gly Ser Leu Pro Trp Thr
1               5
```

<210> 49
<211> 448
<212> PRT
<213> Artificial sequence

<220>
<223> atezolizumab (MPDL3280A) heavy chain

<400> 49

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190
```

Ser Leu Gly Thr Gln Thr Tyr Ile Cys Asn Val Asn His Lys Pro Ser
195 200 205

Asn Thr Lys Val Asp Lys Lys Val Glu Pro Lys Ser Cys Asp Lys Thr
210 215 220

His Thr Cys Pro Pro Cys Pro Ala Pro Glu Leu Leu Gly Gly Pro Ser
225 230 235 240

Val Phe Leu Phe Pro Pro Lys Pro Lys Asp Thr Leu Met Ile Ser Arg
245 250 255

Thr Pro Glu Val Thr Cys Val Val Val Asp Val Ser His Glu Asp Pro
260 265 270

Glu Val Lys Phe Asn Trp Tyr Val Asp Gly Val Glu Val His Asn Ala
275 280 285

Lys Thr Lys Pro Arg Glu Glu Gln Tyr Ala Ser Thr Tyr Arg Val Val
290 295 300

Ser Val Leu Thr Val Leu His Gln Asp Trp Leu Asn Gly Lys Glu Tyr
305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Tyr Thr Leu
340 345 350

Pro Pro Ser Arg Glu Glu Met Thr Lys Asn Gln Val Ser Leu Thr Cys
355 360 365

Leu Val Lys Gly Phe Tyr Pro Ser Asp Ile Ala Val Glu Trp Glu Ser
370 375 380

Asn Gly Gln Pro Glu Asn Asn Tyr Lys Thr Thr Pro Pro Val Leu Asp
385 390 395 400

Ser Asp Gly Ser Phe Phe Leu Tyr Ser Lys Leu Thr Val Asp Lys Ser
405 410 415

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro Gly Lys

435 440 445

<210> 50
<211> 214
<212> PRT
<213> Artificial sequence

<220>
<223> atezolizumab (MPDL3280A) light chain

<400> 50

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Leu Tyr His Pro Ala
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

                    Phe Asn Arg Gly Glu Cys
                        210
```

<210> 51
<211> 118
<212> PRT
<213> Artificial sequence

<220>
<223> atezolizumab (MPDL3280A) variable heavy chain

<400> 51

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ala
        115
```

<210> 52
<211> 108
<212> PRT
<213> Artificial sequence

<220>
<223> atezolizumab (MPDL3280A) variable light chain

<400> 52

```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
```

20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Tyr Leu Tyr His Pro Ala
85 90 95

Thr Phe Gly Gln Gly Thr Lys Val Glu Ile Lys Arg
100 105

<210> 53
<211> 10
<212> PRT
<213> Artificial sequence

<220>
<223> atezolizumab (MPDL3280A) heavy chain CDR1

<220>
<221> variant
<222> (6).. (6)
<223> residue is D or G

<400> 53

Gly Phe Thr Phe Ser Xaa Ser Trp Ile His
1 5 10

<210> 54
<211> 18
<212> PRT
<213> Artificial sequence

<220>
<223> atezolizumab (MPDL3280A) heavy chain CDR2

<220>
<221> variant
<222> (4).. (4)
<223> residue is S or L

<220>
<221> variant
<222> (10)..(10)
<223> residue is T or S

<400> 54

Ala Trp Ile Xaa Pro Tyr Gly Gly Ser Xaa Tyr Tyr Ala Asp Ser Val
1 5 10 15

Lys Gly

<210> 55
<211> 9
<212> PRT
<213> Artificial sequence

<220>
<223> atezolizumab (MPDL3280A) heavy chain CDR3

<400> 55

Arg His Trp Pro Gly Gly Phe Asp Tyr
1 5

<210> 56
<211> 11
<212> PRT
<213> Artificial sequence

<220>
<223> atezolizumab (MPDL3280A) light chain CDR1

<220>
<221> variant
<222> (5)..(5)
<223> residue is D or V

<220>
<221> variant
<222> (6).. (6)
<223> residue is V or I

<220>
<221> variant
<222> (7).. (7)
<223> residue is S or N

<220>
<221> variant
<222> (9).. (9)
<223> residue is A or F

<220>
<221> variant
<222> (10)..(10)
<223> residue is V or L

<400> 56

Arg Ala Ser Gln Xaa Xaa Xaa Thr Xaa Xaa Ala
1 5 10

<210> 57

<211> 7
<212> PRT
<213> Artificial sequence

<220>
<223> atezolizumab (MPDL3280A) light chain CDR2

<220>
<221> variant
<222> (4).. (4)
<223> residue is F or T

<220>
<221> variant
<222> (6).. (6)
<223> residue is Y or A

<400> 57

```
Ser Ala Ser Xaa Leu Xaa Ser
1               5
```

<210> 58
<211> 9
<212> PRT
<213> Artificial sequence

<220>
<223> atezolizumab (MPDL3280A) light chain CDR3

<220>
<221> variant
<222> (3)..(3)
<223> residue is Y, G, F or S

<220>
<221> variant
<222> (4)..(4)
<223> residue is L, Y, F, or W

<220>
<221> variant
<222> (5)..(5)
<223> residue is Y, N, A, T, G, F or I

<220>
<221> variant
<222> (6).. (6)
<223> residue is H, V, P, T or I

<220>
<221> variant
<222> (8)..(8)
<223> residue is A, W, R, P or T

<400> 58

```
Gln Gln Xaa Xaa Xaa Xaa Pro Xaa Thr
1               5
```

<210> 59
<211> 450
<212> PRT
<213> Artificial Sequence

<220>
<223> avelumab (MSB0010718C) heavy chain

<400> 59

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ile Met Met Trp Val Arg Gln Ala Pro Gly Lys Gly Leu Glu Trp Val
        35                  40                  45

Ser Ser Ile Tyr Pro Ser Gly Gly Ile Thr Phe Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ile Lys Leu Gly Thr Val Thr Thr Val Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205
```

```
Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450
```

<210> 60
<211> 216
<212> PRT
<213> Artificial Sequence

<220>
<223> avelumab (MSB0010718C) light chain

<400> 60

```
Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205
```

Thr Val Ala Pro Thr Glu Cys Ser
210 215

<210> 61
<211> 120
<212> PRT
<213> Artificial Sequence

<220>
<223> avelumab (MSB0010718C) variable heavy chain

<400> 61

Glu Val Gln Leu Leu Glu Ser Gly Gly Gly Leu Val Gln Pro Gly Gly
1 5 10 15

Ser Leu Arg Leu Ser Cys Ala Ala Ser Gly Phe Thr Phe Ser Ser Tyr
20 25 30

Ile Met Met Trp Val Arg Gln Ala Pro Gly Lys Gly Leu Glu Trp Val
35 40 45

Ser Ser Ile Tyr Pro Ser Gly Gly Ile Thr Phe Tyr Ala Asp Thr Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Ile Lys Leu Gly Thr Val Thr Thr Val Asp Tyr Trp Gly Gln
100 105 110

Gly Thr Leu Val Thr Val Ser Ser
115 120

<210> 62
<211> 110
<212> PRT
<213> Artificial Sequence

<220>
<223> avelumab (MSB0010718C) variable light chain

<400> 62

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Arg Val Phe Gly Thr Gly Thr Lys Val Thr Val Leu
            100                 105                 110

<210> 63
<211> 5
<212> PRT
<213> Artificial Sequence

<220>
<223> avelumab (MSB0010718C) heavy chain CDR1

<400> 63

Ser Tyr Ile Met Met
1               5

<210> 64
<211> 17
<212> PRT
<213> Artificial Sequence

<220>
<223> avelumab (MSB0010718C) heavy chain CDR2

<400> 64

Ser Ile Tyr Pro Ser Gly Gly Ile Thr Phe Tyr Ala Asp Thr Val Lys
1               5                   10                  15

Gly

<210> 65
<211> 11
<212> PRT
<213> Artificial Sequence

<220>
<223> avelumab (MSB0010718C) heavy chain CDR3

<400> 65

Ile Lys Leu Gly Thr Val Thr Thr Val Asp Tyr
1 5 10

<210> 66
<211> 14
<212> PRT
<213> Artificial Sequence

<220>
<223> avelumab (MSB0010718C) light chain CDR1

<400> 66

Thr Gly Thr Ser Ser Asp Val Gly Gly Tyr Asn Tyr Val Ser
1 5 10

<210> 67
<211> 7
<212> PRT
<213> Artificial Sequence

<220>
<223> avelumab (MSB0010718C) light chain CDR2

<400> 67

Asp Val Ser Asn Arg Pro Ser
1 5

<210> 68
<211> 10
<212> PRT
<213> Artificial Sequence

<220>
<223> avelumab (MSB0010718C) light chain CDR3

<400> 68

```
Ser Ser Tyr Thr Ser Ser Ser Thr Arg Val
1               5                   10
```

## Description

### FIELD OF THE INVENTION

Therapeutic combinations of a Bruton's tyrosine kinase (BTK) inhibitor and an antifolate compound, and uses of the therapeutic combinations are disclosed herein. In particular, a combination of a BTK inhibitor and an antifolate compound and compositions and uses thereof are disclosed.

### BACKGROUND OF THE INVENTION

B lymphocyte activation is key in the generation of adaptive immune responses. Derailed B lymphocyte activation is a hallmark of many autoimmune disorders and modulation of this immune response is therefore of therapeutic interest. Recently the success of B cell therapies in autoimmune disorders has been established. Treatment of rheumatoid arthritis (RA) patients with rituximab (anti-CD20 therapy) is an accepted clinical therapy. More recent clinical trials show that treatment with rituximab also ameliorates disease symptoms in relapsing remitting multiple sclerosis (RRMS) and systemic lupus erythematosus (SLE) patients. This success supports the potential for future therapies in autoimmune disorders targeting B cell immunity.

Bruton's Tyrosine Kinase (BTK) is a Tec family non-receptor protein kinase expressed in B cells and myeloid cells. The function of BTK in signaling pathways activated by the engagement of the B cell receptor (BCR) and FCER1 on mast cells is well established. Functional mutations in BTK in humans result in a primary immunodeficiency disease characterized by a defect in B cell development with a block between pro- and pre-B cell stages. The result is an almost complete absence of B lymphocytes, causing a pronounced reduction of serum immunoglobulin of all classes. These findings support a key role for BTK in the regulation of the production of auto-antibodies in autoimmune disorders.

Other diseases with an important role for dysfunctional B cells are B cell malignancies. The reported role for BTK in the regulation of proliferation and apoptosis of B cells indicates the potential for BTK inhibitors in the treatment of B cell lymphomas. BTK inhibitors have thus been developed as potential therapies, as described in D'Cruz and Uckun, OncoTargets and Therapy 2013, 6, 161-176.

Antifolates represent one of the most thoroughly studied classes of antineoplastic agents, with aminopterin initially demonstrating clinical activity approximately 50 years ago. Methotrexate was developed shortly thereafter, and today is a standard component of effective chemotherapeutic regimens for malignancies such as lymphoma, breast cancer, and head and neck cancer. Bonnadonna, et al., J. Am. Med. Assoc. 1995, 273, 542-547; Bonnadonna, et al., N. Engl. J. Med. 1995, 332, 901-906; and Hong, et al., Cancer 1983, 52, 206-210. Antifolates inhibit one or several key folate-requiring enzymes of the thymidine and purine biosynthetic pathways, in particular, thymidylate synthase (TS), dihydrofolate reductase (DHFR), and glycinamide ribonucleotide formyltransferase (GARFT), by competing with reduced folates for binding sites of these enzymes. Shih, et al., Advan. Enzyme Regul. 1998, 38, 135-152 and Shih, et al., Cancer Res 1997, 57, 1116-1123. In Xu, D. et al. J. Pharmacol. Exp. Th., 2012, 341, 90 -103, 6-cyclopropyl-8-fluoro-2-(2-hydroxymethyl-3-{1-methyl-5-[5-(4-methyl-piperazin-1-yl)-pyridin-2-ylamino]-6-oxo-1,6-dihydro-pyridin-3-yl}-phenyl)-2*H*-isoquinolin-1-one (RN486), was found to inhibit the BTK enzyme and was shown to be effective for the treatment of arthritis.

The present invention provides the unexpected finding that the combination of an antifolate compound and a BTK inhibitor is synergistically effective in the treatment of rheumatoid arthritis.

### SUMMARY OF THE INVENTION

In an embodiment, the invention provides a combination comprising a BTK inhibitor having structure of Formula (2):

Formula (2)

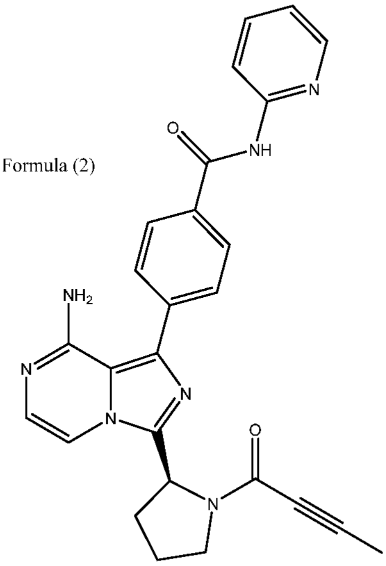

or a pharmaceutically acceptable salt thereof, and methotrexate or a pharmaceutically acceptable salt thereof for use in the treatment of rheumatoid arthritis.

In another embodiment, the invention provides a composition comprising therapeutically effective amounts of:

(1) a BTK inhibitor having structure of Formula (2):

Formula (2)

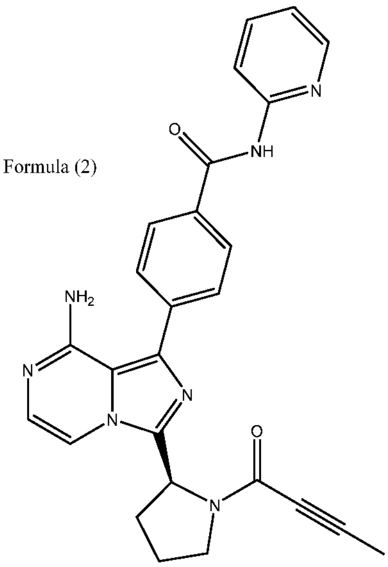

or a pharmaceutically acceptable salt thereof, and

(2) methotrexate or a pharmaceutically acceptable salt thereof, for use in the treatment of rheumatoid arthritis.

In another embodiment, the invention provides a kit comprising:

a pharmaceutical composition comprising a BTK inhibitor having structure of formula (2):

Formula (2)

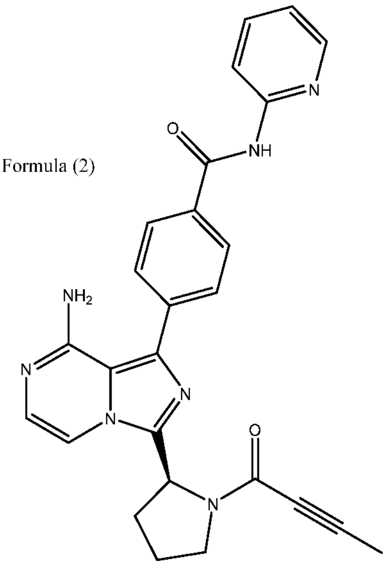

or a pharmaceutically acceptable salt thereof, and a pharmaceutical composition comprising methotrexate or a pharmaceutically acceptable salt thereof, for co-administration of the methotrexate and the compound of formula (2), either simultaneously or separately, for use in the treatment of rheumatoid arthritis.

BRIEF DESCRIPTION OF THE DRAWINGS

The foregoing summary, as well as the following detailed description of the invention, will be better understood when read in conjunction with the appended drawings.

FIG. 1 illustrates the effect of the BTK inhibitor of Formula (2) (1 mg/kg), alone or in combination with methotrexate (0.3 mg/kg) on arthritis score versus day following immunization.

FIG. 2 illustrates the effect of the BTK inhibitor of Formula (2) (1 mg/kg), alone or in combination with methotrexate (0.5 mg/kg) on arthritis score versus day following immunization.

FIG. 3 illustrates the effect of the BTK inhibitor of Formula (2) (5 mg/kg), alone or in combination with methotrexate (0.3 mg/kg) on arthritis score versus day following immunization.

FIG. 4 illustrates the effect of the BTK inhibitor of Formula (2) (5 mg/kg), alone or in combination with methotrexate (0.5 mg/kg) on arthritis score versus day following immunization.

BRIEF DESCRIPTION OF THE SEQUENCE LISTING

SEQ ID NO:1 is the heavy chain amino acid sequence of the anti-CD20 monoclonal antibody rituximab.

SEQ ID NO:2 is the light chain amino acid sequence of the anti-CD20 monoclonal antibody rituximab.

SEQ ID NO:3 is the heavy chain amino acid sequence of the anti-CD20 monoclonal antibody obinutuzumab.

SEQ ID NO:4 is the light chain amino acid sequence of the anti-CD20 monoclonal antibody obinutuzumab.

SEQ ID NO:5 is the variable heavy chain amino acid sequence of the anti-CD20 monoclonal antibody ofatumumab.

SEQ ID NO:6 is the variable light chain amino acid sequence of the anti-CD20 monoclonal antibody ofatumumab.

SEQ ID NO:7 is the Fab fragment heavy chain amino acid sequence of the anti-CD20 monoclonal antibody ofatumumab.

SEQ ID NO:8 is the Fab fragment light chain amino acid sequence of the anti-CD20 monoclonal antibody ofatumumab.

SEQ ID NO:9 is the heavy chain amino acid sequence of the anti-CD20 monoclonal antibody veltuzumab.

SEQ ID NO:10 is the light chain amino acid sequence of the anti-CD20 monoclonal antibody veltuzumab.

SEQ ID NO: 11 is the heavy chain amino acid sequence of the anti-CD20 monoclonal antibody tositumomab.

SEQ ID NO:12 is the light chain amino acid sequence of the anti-CD20 monoclonal antibody tositumomab.

SEQ ID NO:13 is the heavy chain amino acid sequence of the anti-CD20 monoclonal antibody ibritumomab.

SEQ ID NO:14 is the light chain amino acid sequence of the anti-CD20 monoclonal antibody ibritumomab.

SEQ ID NO:15 is the heavy chain amino acid sequence of the PD-1 inhibitor nivolumab.

SEQ ID NO:16 is the light chain amino acid sequence of the PD-1 inhibitor nivolumab.

SEQ ID NO: 17 is the heavy chain variable region ($V_H$) amino acid sequence of the PD-1 inhibitor nivolumab.

SEQ ID NO: 18 is the light chain variable region ($V_L$) amino acid sequence of the PD-1 inhibitor nivolumab.

SEQ ID NO:19 is the heavy chain CDR1 amino acid sequence of the PD-1 inhibitor nivolumab.

SEQ ID NO:20 is the heavy chain CDR2 amino acid sequence of the PD-1 inhibitor nivolumab.

SEQ ID NO:21 is the heavy chain CDR3 amino acid sequence of the PD-1 inhibitor nivolumab.

SEQ ID NO:22 is the light chain CDR1 amino acid sequence of the PD-1 inhibitor nivolumab.

SEQ ID NO:23 is the light chain CDR2 amino acid sequence of the PD-1 inhibitor nivolumab.

SEQ ID NO:24 is the light chain CDR3 amino acid sequence of the PD-1 inhibitor nivolumab.

SEQ ID NO:25 is the heavy chain amino acid sequence of the PD-1 inhibitor pembrolizumab.

SEQ ID NO:26 is the light chain amino acid sequence of the PD-1 inhibitor pembrolizumab.

SEQ ID NO:27 is the heavy chain variable region ($V_H$) amino acid sequence of the PD-1 inhibitor pembrolizumab.

SEQ ID NO:28 is the light chain variable region ($V_L$) amino acid sequence of the PD-1 inhibitor pembrolizumab.

SEQ ID NO:29 is the heavy chain CDR1 amino acid sequence of the PD-1 inhibitor pembrolizumab.

SEQ ID NO:30 is the heavy chain CDR2 amino acid sequence of the PD-1 inhibitor pembrolizumab.

SEQ ID NO:31 is the heavy chain CDR3 amino acid sequence of the PD-1 inhibitor pembrolizumab.

SEQ ID NO:32 is the light chain CDR1 amino acid sequence of the PD-1 inhibitor pembrolizumab.

SEQ ID NO:33 is the light chain CDR2 amino acid sequence of the PD-1 inhibitor pembrolizumab.

SEQ ID NO:34 is the light chain CDR3 amino acid sequence of the PD-1 inhibitor pembrolizumab.

SEQ ID NO:35 is the heavy chain amino acid sequence of the PD-1 inhibitor pidilizumab.

SEQ ID NO:36 is the light chain amino acid sequence of the PD-1 inhibitor pidilizumab.

SEQ ID NO:37 is the heavy chain variable region ($V_H$) amino acid sequence of the PD-1 inhibitor pidilizumab.

SEQ ID NO:38 is the light chain variable region ($V_L$) amino acid sequence of the PD-1 inhibitor pidilizumab.

SEQ ID NO:39 is the heavy chain amino acid sequence of the PD-L1 inhibitor durvalumab.

SEQ ID NO:40 is the light chain amino acid sequence of the PD-L1 inhibitor durvalumab.

SEQ ID NO:41 is the heavy chain variable region ($V_H$) amino acid sequence of the PD-L1 inhibitor durvalumab.

SEQ ID NO:42 is the light chain variable region ($V_L$) amino acid sequence of the PD-L1 inhibitor durvalumab.

SEQ ID NO:43 is the heavy chain CDR1 amino acid sequence of the PD-L1 inhibitor durvalumab.

SEQ ID NO:44 is the heavy chain CDR2 amino acid sequence of the PD-L1 inhibitor durvalumab.

SEQ ID NO:45 is the heavy chain CDR3 amino acid sequence of the PD-L1 inhibitor durvalumab.

SEQ ID NO:46 is the light chain CDR1 amino acid sequence of the PD-L1 inhibitor durvalumab.

SEQ ID NO:47 is the light chain CDR2 amino acid sequence of the PD-L1 inhibitor durvalumab.

SEQ ID NO:48 is the light chain CDR3 amino acid sequence of the PD-L1 inhibitor durvalumab.

SEQ ID NO:49 is the heavy chain amino acid sequence of the PD-L1 inhibitor atezolizumab.

SEQ ID NO:50 is the light chain amino acid sequence of the PD-L1 inhibitor atezolizumab.

SEQ ID NO:51 is the heavy chain variable region ($V_H$) amino acid sequence of the PD-L1 inhibitor atezolizumab.

SEQ ID NO:52 is the light chain variable region ($V_L$) amino acid sequence of the PD-L1 inhibitor atezolizumab.

SEQ ID NO:53 is the heavy chain CDR1 amino acid sequence of the PD-L1 inhibitor atezolizumab.

SEQ ID NO:54 is the heavy chain CDR2 amino acid sequence of the PD-L1 inhibitor atezolizumab.

SEQ ID NO: 55 is the heavy chain CDR3 amino acid sequence of the PD-L1 inhibitor atezolizumab.

SEQ ID NO:56 is the light chain CDR1 amino acid sequence of the PD-L1 inhibitor atezolizumab.

SEQ ID NO:57 is the light chain CDR2 amino acid sequence of the PD-L1 inhibitor atezolizumab.

SEQ ID NO:58 is the light chain CDR3 amino acid sequence of the PD-L1 inhibitor atezolizumab.

SEQ ID NO:59 is the heavy chain amino acid sequence of the PD-L1 inhibitor avelumab.

SEQ ID NO:60 is the light chain amino acid sequence of the PD-L1 inhibitor avelumab.

SEQ ID NO:61 is the heavy chain variable region ($V_H$) amino acid sequence of the PD-L1 inhibitor avelumab.

SEQ ID NO:62 is the heavy chain variable region ($V_H$) amino acid sequence of the PD-L1 inhibitor avelumab.

SEQ ID NO:63 is the heavy chain CDR1 amino acid sequence of the PD-L1 inhibitor avelumab.

SEQ ID NO:64 is the heavy chain CDR2 amino acid sequence of the PD-L1 inhibitor avelumab.

SEQ ID NO:65 is the heavy chain CDR3 amino acid sequence of the PD-L1 inhibitor avelumab.

SEQ ID NO:66 is the light chain CDR1 amino acid sequence of the PD-L1 inhibitor avelumab.

SEQ ID NO:67 is the light chain CDR2 amino acid sequence of the PD-L1 inhibitor avelumab.

SEQ ID NO:68 is the light chain CDR3 amino acid sequence of the PD-L1 inhibitor avelumab.

DETAILED DESCRIPTION OF THE INVENTION

Unless defined otherwise, all technical and scientific terms used herein have the same meaning as is commonly understood by one of skill in the art to which this invention belongs.

The terms "co-administration," "co-administering," "administered in combination with," "administering in combination with," "simultaneous," and "concurrent," as used herein, encompass administration of two or more active pharmaceutical ingredients (in a preferred embodiment of the present invention, for example, at least one antifolate compound and at least one BTK inhibitor) to a subject so that both active pharmaceutical ingredients and/or their metabolites are present in the subject at the same time. Co-administration includes simultaneous administration in separate compositions, administration at different times in separate compositions, or administration in a composition in which two or more active pharmaceutical ingredients are present. Simultaneous administration in separate compositions and administration in a composition in which both agents are present are preferred.

The term *"in vivo"* refers to an event that takes place in a subject's body.

The term *"in vitro"* refers to an event that takes places outside of a subject's body. *In vitro* assays encompass cell-based assays in which cells alive or dead are employed and may also encompass a cell-free assay in which no intact cells are employed.

The term "effective amount" or "therapeutically effective amount" refers to that amount of a compound or combination of compounds as described herein that is sufficient to effect the intended application including, but not limited to, disease treatment. A therapeutically effective amount may vary depending upon the intended application (*in vitro* or *in vivo*), or the subject and disease condition being treated (*e.g.,* the weight, age and gender of the subject), the severity of the disease condition, the manner of administration, *etc.* which can readily be determined by one of ordinary skill in the art. The term also applies to a dose that will induce a particular response in target cells (*e.g.,* the reduction of platelet adhesion and/or cell migration). The specific dose will vary depending on the particular compounds chosen, the dosing regimen to be followed, whether the compound is administered in combination with other compounds, timing of administration, the tissue to which it is administered, and the physical delivery system in which the compound is carried.

A "therapeutic effect" as that term is used herein, encompasses a therapeutic benefit and/or a prophylactic benefit. A prophylactic effect includes delaying or eliminating the appearance of a disease or condition, delaying or eliminating the onset of symptoms of a disease or condition, slowing, halting, or reversing the progression of a disease or condition, or any combination thereof.

The terms "QD," "qd," or "q.d." mean *quaque die,* once a day, or once daily. The terms "BID," "bid," or "b.i.d." mean *bis in die*, twice a day, or twice daily. The terms "TID," "tid," or "t.i.d." mean *ter in die*, three times a day, or three times daily. The terms "QID," "qid," or "q.i.d." mean *quater in die*, four times a day, or four times daily. The terms "PO", "po" or "p.o." *mean per os,* by mouth or orally.

The term "pharmaceutically acceptable salt" refers to salts derived from a variety of organic and inorganic counter ions known in the art. Pharmaceutically acceptable acid addition salts can be formed with inorganic acids and organic acids. Preferred inorganic acids from which salts can be derived include, for example, hydrochloric acid, hydrobromic acid, sulfuric acid, nitric acid and phosphoric acid. Preferred organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid and salicylic acid. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese and aluminum. Organic bases from which salts can be derived include, for example, primary, secondary, and tertiary amines, substituted amines including naturally occurring substituted amines, cyclic amines and basic ion exchange resins. Specific examples include isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts.

"Pharmaceutically acceptable carrier" or "pharmaceutically acceptable excipient" is intended to include any and all solvents, dispersion media, coatings, antibacterial and antifungal agents, isotonic and absorption delaying agents, and inert ingredients. The use of such pharmaceutically acceptable carriers or pharmaceutically acceptable excipients for active pharmaceutical ingredients is well known in the art. Except insofar as any conventional pharmaceutically

acceptable carrier or pharmaceutically acceptable excipient is incompatible with the active pharmaceutical ingredient, its use in the therapeutic compositions for use in the invention is contemplated. Additional active pharmaceutical ingredients, such as other drugs, can also be incorporated into the described compositions and methods.

As used herein, the term "warhead" or "warhead group" refers to a functional group present on a compound for use in the present invention wherein that functional group is capable of covalently binding to an amino acid residue present in the binding pocket of the target protein (such as cysteine, lysine, histidine, or other residues capable of being covalently modified), thereby irreversibly inhibiting the protein.

Unless otherwise stated, the chemical structures depicted herein are intended to include compounds which differ only in the presence of one or more isotopically enriched atoms. For example, compounds where one or more hydrogen atoms is replaced by deuterium or tritium, or wherein one or more carbon atoms is replaced by $^{13}C$- or $^{14}C$-enriched carbons, are within the scope of this invention.

When ranges are used herein to describe, for example, physical or chemical properties such as molecular weight or chemical formulae, all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, preferably from 0% to 10%, more preferably from 0% to 5% of the stated number or numerical range. The term "comprising" (and related terms such as "comprise" or "comprises" or "having" or "including") includes those embodiments such as, for example, an embodiment of any composition of matter, method or process that "consist of' or "consist essentially of' the described features.

"Isomers" are different compounds that have the same molecular formula. "Stereoisomers" are isomers that differ only in the way the atoms are arranged in space - *i.e.,* having a different stereochemical configuration. "Enantiomers" are a pair of stereoisomers that are non-superimposable mirror images of each other. A 1:1 mixture of a pair of enantiomers is a "racemic" mixture. The term "(±)" is used to designate a racemic mixture where appropriate. "Diastereoisomers" are stereoisomers that have at least two asymmetric atoms, but which are not mirror-images of each other. The absolute stereochemistry is specified according to the Cahn-Ingold-Prelog R-S system. When a compound is a pure enantiomer the stereochemistry at each chiral carbon can be specified by either (*R*) or (*S*). Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and can thus give rise to enantiomers, diastereomers, and other stereoisomeric forms that can be defined, in terms of absolute stereochemistry, as (*R*) or (*S*). The present chemical entities, pharmaceutical compositions and methods are meant to include all such possible isomers, including racemic mixtures, optically pure forms and intermediate mixtures. Optically active (*R*)- and (*S*)-isomers can be prepared using chiral synthons or chiral reagents, or resolved using conventional techniques. When the compounds described herein contain olefinic double bonds or other centers of geometric asymmetry, and unless specified otherwise, it is intended that the compounds include both *E* and *Z* geometric isomers.

"Enantiomeric purity" as used herein refers to the relative amounts, expressed as a percentage, of the presence of a specific enantiomer relative to the other enantiomer. For example, if a compound, which may potentially have an (*R*)- or an (*S*)-isomeric configuration, is present as a racemic mixture, the enantiomeric purity is about 50% with respect to either the (*R*)- or (*S*)-isomer. If that compound has one isomeric form predominant over the other, for example, 80% (*S*)-isomer and 20% (*R*)-isomer, the enantiomeric purity of the compound with respect to the (*S*)-isomeric form is 80%. The enantiomeric purity of a compound can be determined in a number of ways known in the art, including but not limited to chromatography using a chiral support, polarimetric measurement of the rotation of polarized light, nuclear magnetic resonance spectroscopy using chiral shift reagents which include but are not limited to lanthanide containing chiral complexes or Pirkle's reagents, or derivatization of a compounds using a chiral compound such as Mosher's acid followed by chromatography or nuclear magnetic resonance spectroscopy.

In preferred embodiments, the enantiomerically enriched composition has a higher potency with respect to therapeutic utility per unit mass than does the racemic mixture of that composition. Enantiomers can be isolated from mixtures by methods known to those skilled in the art, including chiral high pressure liquid chromatography (HPLC) and the formation and crystallization of chiral salts; or preferred enantiomers can be prepared by asymmetric syntheses. See, for example, Jacques, et al., Enantiomers, Racemates and Resolutions, Wiley Interscience, New York (1981); E. L. Eliel, Stereochemistry of Carbon Compounds, McGraw-Hill, New York (1962); and E. L. Eliel and S. H. Wilen, Stereochemistry of Organic Compounds, Wiley-Interscience, New York (1994).

The terms "enantiomerically enriched" and "non-racemic," as used herein, refer to compositions in which the percent by weight of one enantiomer is greater than the amount of that one enantiomer in a control mixture of the racemic composition (e.g., greater than 1:1 by weight). For example, an enantiomerically enriched preparation of the (S)-enantiomer, means a preparation of the compound having greater than 50% by weight of the (S)-enantiomer relative to the (*R*)-enantiomer, such as at least 75% by weight, or such as at least 80% by weight. In some embodiments, the enrichment

can be significantly greater than 80% by weight, providing a "substantially enantiomerically enriched" or a "substantially non-racemic" preparation, which refers to preparations of compositions which have at least 85% by weight of one enantiomer relative to other enantiomer, such as at least 90% by weight, or such as at least 95% by weight. The terms "enantiomerically pure" or "substantially enantiomerically pure" refers to a composition that comprises at least 98% of a single enantiomer and less than 2% of the opposite enantiomer.

"Moiety" refers to a specific segment or functional group of a molecule. Chemical moieties are often recognized chemical entities embedded in or appended to a molecule.

"Tautomers" are structurally distinct isomers that interconvert by tautomerization. "Tautomerization" is a form of isomerization and includes prototropic or proton-shift tautomerization, which is considered a subset of acid-base chemistry. "Prototropic tautomerization" or "proton-shift tautomerization" involves the migration of a proton accompanied by changes in bond order, often the interchange of a single bond with an adjacent double bond. Where tautomerization is possible (*e.g.*, in solution), a chemical equilibrium of tautomers can be reached. An example of tautomerization is keto-enol tautomerization. A specific example of keto-enol tautomerization is the interconversion of pentane-2,4-dione and 4-hydroxypent-3-en-2-one tautomers. Another example of tautomerization is phenol-keto tautomerization. A specific example of phenol-keto tautomerization is the interconversion of pyridin-4-ol and pyridin-4(1*H*)-one tautomers.

A "leaving group or atom" is any group or atom that will, under selected reaction conditions, cleave from the starting material, thus promoting reaction at a specified site. Examples of such groups, unless otherwise specified, include halogen atoms and mesyloxy, p-nitrobenzensulphonyloxy and tosyloxy groups.

"Protecting group" is intended to mean a group that selectively blocks one or more reactive sites in a multifunctional compound such that a chemical reaction can be carried out selectively on another unprotected reactive site and the group can then be readily removed or deprotected after the selective reaction is complete. A variety of protecting groups are disclosed, for example, in T. H. Greene and P. G. M. Wuts, Protective Groups in Organic Synthesis, Third Edition, John Wiley & Sons, New York (1999).

"Substituted" means that the referenced group may have attached one or more additional groups, radicals or moieties individually and independently selected from, for example, acyl, alkyl, alkylaryl, cycloalkyl, aralkyl, aryl, carbohydrate, carbonate, heteroaryl, heterocycloalkyl, hydroxy, alkoxy, aryloxy, mercapto, alkylthio, arylthio, cyano, halo, carbonyl, ester, thiocarbonyl, isocyanato, thiocyanato, isothiocyanato, nitro, oxo, perhaloalkyl, perfluoroalkyl, phosphate, silyl, sulfinyl, sulfonyl, sulfonamidyl, sulfoxyl, sulfonate, urea, and amino, including mono- and di-substituted amino groups, and protected derivatives thereof. The substituents themselves may be substituted, for example, a cycloalkyl substituent may itself have a halide substituent at one or more of its ring carbons. The term "optionally substituted" means optional substitution with the specified groups, radicals or moieties.

The compounds disclosed herein for use in the invention also include crystalline and amorphous forms of those compounds, including, for example, polymorphs, pseudopolymorphs, unsolvated polymorphs (including anhydrates), conformational polymorphs, and amorphous forms of the compounds, as well as mixtures thereof. "Crystalline form" and "polymorph" are intended to include all crystalline and amorphous forms of the compound, including, for example, polymorphs, pseudopolymorphs, unsolvated polymorphs (including anhydrates), conformational polymorphs, and amorphous forms, as well as mixtures thereof, unless a particular crystalline or amorphous form is referred to. The combination and compositions for use in the present invention may comprise antibodies. The terms "antibody" and its plural form "antibodies" refer to whole immunoglobulins and any antigen-binding fragment ("antigen-binding portion") or single chains thereof. An "antibody" further refers to a glycoprotein comprising at least two heavy (H) chains and two light (L) chains inter-connected by disulfide bonds, or an antigen-binding portion thereof. Each heavy chain is comprised of a heavy chain variable region (abbreviated herein as $V_H$) and a heavy chain constant region. The heavy chain constant region is comprised of three domains, CHI, CH2 and CH3. Each light chain is comprised of a light chain variable region (abbreviated herein as $V_L$) and a light chain constant region. The light chain constant region is comprised of one domain, $C_L$. The $V_H$ and $V_L$ regions of an antibody may be further subdivided into regions of hypervariability, which are referred to as complementarity determining regions (CDR) or hypervariable regions (HVR), and which can be interspersed with regions that are more conserved, termed framework regions (FR). Each $V_H$ and $V_L$ is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen epitope or epitopes. The constant regions of the antibodies may mediate the binding of the immunoglobulin to host tissues or factors, including various cells of the immune system (*e.g.*, effector cells) and the first component (Clq) of the classical complement system. The terms "monoclonal antibody," "mAb," "monoclonal antibody composition," or their plural forms refer to a preparation of antibody molecules of single molecular composition. A monoclonal antibody composition displays a single binding specificity and affinity for a particular epitope. Monoclonal antibodies specific to, *e.g.,* CD20, PD-1, PD-L1, or PD-L2 can be made using knowledge and skill in the art of injecting test subjects with CD20, PD-1, PD-L1, or PD-L2 antigen and then isolating hybridomas expressing antibodies having the desired sequence or functional characteristics. DNA encoding the monoclonal antibodies is readily isolated and sequenced using conventional procedures (*e.g.*, by using oligonucleotide probes that are capable of binding specifically to genes encoding the heavy and light chains of

the monoclonal antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies will be described in more detail below.

The terms "antigen-binding portion" or "antigen-binding fragment" of an antibody (or simply "antibody portion"), as used herein, refers to one or more fragments of an antibody that retain the ability to specifically bind to an antigen (e.g., PD-1, PD-L1, or PD-L2). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the $V_L$, $V_H$, $C_L$ and CH1 domains; (ii) a F(ab')2 fragment, a bivalent fragment comprising two Fab fragments linked by a disulfide bridge at the hinge region; (iii) a Fd fragment consisting of the $V_H$ and CH1 domains; (iv) a Fv fragment consisting of the $V_L$ and $V_H$ domains of a single arm of an antibody, (v) a domain antibody (dAb) fragment (Ward, et al., Nature, 1989, 341, 544-546), which may consist of a $V_H$ or a $V_L$ domain; and (vi) an isolated complementarity determining region (CDR). Furthermore, although the two domains of the Fv fragment, $V_L$ and $V_H$, are coded for by separate genes, they can be joined, using recombinant methods, by a synthetic linker that enables them to be made as a single protein chain in which the $V_L$ and $V_H$ regions pair to form monovalent molecules known as single chain Fv (scFv); see, *e.g.,* Bird, et al., Science 1988, 242, 423-426; and Huston, et al., Proc. Natl. Acad. Sci. USA 1988, 85, 5879-5883). Such scFv antibodies are also intended to be encompassed within the terms "antigen-binding portion" or "antigen-binding fragment" of an antibody. These antibody fragments are obtained using conventional techniques known to those with skill in the art, and the fragments are screened for utility in the same manner as are intact antibodies.

The term "human antibody," as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention may include amino acid residues not encoded by human germline immunoglobulin sequences (e.g., mutations introduced by random or site-specific mutagenesis *in vitro* or by somatic mutation *in vivo*). The term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human germline immunoglobulin sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as (a) antibodies isolated from an animal (e.g., a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the $V_H$ and $V_L$ regions of the recombinant antibodies are sequences that, while derived from and related to human germline $V_H$ and $V_L$ sequences, may not naturally exist within the human antibody germline repertoire *in vivo.*

As used herein, "isotype" refers to the antibody class (e.g., IgM or IgG1) that is encoded by the heavy chain constant region genes.

The phrases "an antibody recognizing an antigen" and "an antibody specific for an antigen" are used interchangeably herein with the term "an antibody which binds specifically to an antigen."

The term "human antibody derivatives" refers to any modified form of the human antibody, e.g., a conjugate of the antibody and another active pharmaceutical ingredient or antibody. The terms "conjugate," "antibody-drug conjugate", "ADC," or "immunoconjugate" refers to an antibody, or a fragment thereof, conjugated to a therapeutic moiety, such as a bacterial toxin, a cytotoxic drug or a radionuclide-containing toxin. Toxic moieties can be conjugated to antibodies of the invention using methods available in the art.

The terms "humanized antibody," "humanized antibodies," and "humanized" are intended to refer to antibodies in which CDR sequences derived from the germline of another mammalian species, such as a mouse, have been grafted onto human framework sequences. Additional framework region modifications may be made within the human framework sequences. Humanized forms of non-human (for example, murine) antibodies are chimeric antibodies that contain

minimal sequence derived from non-human immunoglobulin. For the most part, humanized antibodies are human immunoglobulins (recipient antibody) in which residues from a hypervariable region of the recipient are replaced by residues from a 15 hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones, et al., Nature 1986, 321, 522-525; Riechmann, et al., Nature 1988, 332, 323-329; and Presta, Curr. Op. Struct. Biol. 1992, 2, 593-596.

The term "chimeric antibody" is intended to refer to antibodies in which the variable region sequences are derived from one species and the constant region sequences are derived from another species, such as an antibody in which the variable region sequences are derived from a mouse antibody and the constant region sequences are derived from a human antibody.

A "diabody" is a small antibody fragment with two antigen-binding sites. The fragments comprises a heavy chain variable domain ($V_H$) connected to a light chain variable domain ($V_L$) in the same polypeptide chain ($V_H$-$V_L$ or $V_L$-$V_H$). By using a linker that is too short to allow pairing between the two domains on the same chain, the domains are forced to pair with the complementary domains of another chain and create two antigen-binding sites. Diabodies are described more fully in, e.g., European Patent No. EP 404,097, International Patent Publication No. WO 93/11161; and Bolliger, et al., Proc. Natl. Acad. Sci. USA 1993, 90, 6444-6448.

The term "glycosylation" refers to a modified derivative of an antibody. An aglycoslated antibody lacks glycosylation. Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Aglycosylation may increase the affinity of the antibody for antigen, as described in U.S. Patent Nos. 5,714,350 and 6,350,861. Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. For example, the cell lines Ms704, Ms705, and Ms709 lack the fucosyltransferase gene, FUT8 (alpha (1,6) fucosyltransferase), such that antibodies expressed in the Ms704, Ms705, and Ms709 cell lines lack fucose on their carbohydrates. The Ms704, Ms705, and Ms709 FUT8-/- cell lines were created by the targeted disruption of the FUT8 gene in CHO/DG44 cells using two replacement vectors (see e.g. U.S. Patent Publication No. 2004/0110704 or Yamane-Ohnuki, et al., Biotechnol. Bioeng., 2004, 87, 614-622). As another example, European Patent No. EP 1,176,195 describes a cell line with a functionally disrupted FUT8 gene, which encodes a fucosyl transferase, such that antibodies expressed in such a cell line exhibit hypofucosylation by reducing or eliminating the alpha 1,6 bond-related enzyme, and also describes cell lines which have a low enzyme activity for adding fucose to the N-acetylglucosamine that binds to the Fc region of the antibody or does not have the enzyme activity, for example the rat myeloma cell line YB2/0 (ATCC CRL 1662). International Patent Publication WO 03/035835 describes a variant CHO cell line, Lec 13 cells, with reduced ability to attach fucose to Asn(297)-linked carbohydrates, also resulting in hypofucosylation of antibodies expressed in that host cell (see also Shields, et al., J. Biol. Chem. 2002, 277, 26733-26740. International Patent Publication WO 99/54342 describes cell lines engineered to express glycoprotein-modifying glycosyl transferases (e.g., beta(1,4)-N-acetylglucosaminyltransferase III (GnTIII)) such that antibodies expressed in the engineered cell lines exhibit increased bisecting GlcNac structures which results in increased ADCC activity of the antibodies (see also Umana, et al., Nat. Biotech. 1999, 17, 176-180). Alternatively, the fucose residues of the antibody may be cleaved off using a fucosidase enzyme. For example, the fucosidase alpha-L-fucosidase removes fucosyl residues from antibodies as described in Tarentino, et al., Biochem. 1975, 14, 5516-5523.

"Pegylation" refers to a modified antibody, or a fragment thereof, that typically is reacted with polyethylene glycol (PEG), such as a reactive ester or aldehyde derivative of PEG, under conditions in which one or more PEG groups become attached to the antibody or antibody fragment. Pegylation may, for example, increase the biological (e.g., serum) half life of the antibody. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as

mono ($C_1$-$C_{10}$) alkoxy- or aryloxy-polyethylene glycol or polyethylene glycol-maleimide. The antibody to be pegylated may be an aglycosylated antibody. Methods for pegylation are known in the art and can be applied to the antibodies of the invention, as described for example in European Patent Nos. EP 0154316 and EP 0401384.

The term "conservative amino acid substitutions" in means amino acid sequence modifications which do not abrogate the binding of the antibody to the antigen. Conservative amino acid substitutions include the substitution of an amino acid in one class by an amino acid of the same class, where a class is defined by common physicochemical amino acid side chain properties and high substitution frequencies in homologous proteins found in nature, as determined, for example, by a standard Dayhoff frequency exchange matrix or BLOSUM matrix. Six general classes of amino acid side chains have been categorized and include: Class I (Cys); Class II (Ser, Thr, Pro, Ala, Gly); Class III (Asn, Asp, Gln, Glu); Class IV (His, Arg, Lys); Class V (Ile, Leu, Val, Met); and Class VI (Phe, Tyr, Trp). For example, substitution of an Asp for another class III residue such as Asn, Gln, or Glu, is a conservative substitution. Thus, a predicted nonessential amino acid residue in a PD-1 or PD-L1 antibody is preferably replaced with another amino acid residue from the same class. Methods of identifying amino acid conservative substitutions which do not eliminate antigen binding are well-known in the art (see, *e.g.,* Brummell, et al., Biochemistry 1993, 32, 1180-1187; Kobayashi, et al., Protein Eng. 1999, 12, 879-884 (1999); and Burks, et al., Proc. Natl. Acad. Sci. USA 1997, 94, 412-417.

The terms "sequence identity," "percent identity," and "sequence percent identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. Suitable programs to determine percent sequence identity include for example the BLAST suite of programs available from the U.S. Government's National Center for Biotechnology Information BLAST web site. Comparisons between two sequences can be carried using either the BLASTN or BLASTP algorithm. BLASTN is used to compare nucleic acid sequences, while BLASTP is used to compare amino acid sequences. ALIGN, ALIGN-2 (Genentech, South San Francisco, California) or MegAlign, available from DNASTAR, are additional publicly available software programs that can be used to align sequences. One skilled in the art can determine appropriate parameters for maximal alignment by particular alignment software. In certain embodiments, the default parameters of the alignment software are used.

Certain embodiments of the present invention comprise a variant of an antibody, *e.g.,* an antifolate compound that is an antibody, an anti-CD20 antibody, an anti-PD-1 antibody, an anti-PD-LI antibody, and/or an anti-PD-L2 antibody. As used herein, the term "variant" encompasses but is not limited to antibodies which comprise an amino acid sequence which differs from the amino acid sequence of a reference antibody by way of one or more substitutions, deletions and/or additions at certain positions within or adjacent to the amino acid sequence of the reference antibody. The variant may comprise one or more conservative substitutions in its amino acid sequence as compared to the amino acid sequence of a reference antibody. Conservative substitutions may involve, e.g., the substitution of similarly charged or uncharged amino acids. The variant retains the ability to specifically bind to the antigen of the reference antibody.

The term "radioisotope-labeled complex" refers to both non-covalent and covalent attachment of a radioactive isotope, such as $^{90}Y$, $^{111}In$, or $^{131}I$, to an antibody, including conjugates.

The term "biosimilar" means a biological product that is highly similar to a U.S. licensed reference biological product notwithstanding minor differences in clinically inactive components, and for which there are no clinically meaningful differences between the biological product and the reference product in terms of the safety, purity, and potency of the product. Furthermore, a similar biological or "biosimilar" medicine is a biological medicine that is similar to another biological medicine that has already been authorized for use by the European Medicines Agency. The term "biosimilar" is also used synonymously by other national and regional regulatory agencies. Biological products or biological medicines are medicines that are made by or derived from a biological source, such as a bacterium or yeast. They can consist of relatively small molecules such as human insulin or erythropoietin, or complex molecules such as monoclonal antibodies. For example, if the reference anti-CD20 monoclonal antibody is rituximab, an anti-CD20 biosimilar monoclonal antibody approved by drug regulatory authorities with reference to rituximab is a "biosimilar to" rituximab or is a "biosimilar thereof' of rituximab. In Europe, a similar biological or "biosimilar" medicine is a biological medicine that is similar to another biological medicine that has already been authorized for use by the European Medicines Agency (EMA). The relevant legal basis for similar biological applications in Europe is Article 6 of Regulation (EC) No 726/2004 and Article 10(4) of Directive 2001/83/EC, as amended and therefore in Europe, the biosimilar may be authorised, approved for authorisation or subject of an application for authorisation under Article 6 of Regulation (EC) No 726/2004 and Article 10(4) of Directive 2001/83/EC. The already authorized original biological medicinal product may be referred to as a "reference medicinal product" in Europe. Some of the requirements for a product to be considered a biosimilar are outlined in the CHMP Guideline on Similar Biological Medicinal Products. In addition, product specific guidelines, including guidelines relating to monoclonal antibody biosimilars, are provided on a product-by-product basis by the EMA and published on its website.

A biosimilar as described herein may be similar to the reference medicinal product by way of quality characteristics, biological activity, mechanism of action, safety profiles and/or efficacy. In addition, the biosimilar may be used or be intended for use to treat the same conditions as the reference medicinal product. Thus, a biosimilar as described herein may be deemed to have similar or highly similar quality characteristics to a reference medicinal product. Alternatively, or in addition, a biosimilar as described herein may be deemed to have similar or highly similar biological activity to a reference medicinal product. Alternatively, or in addition, a biosimilar as described herein may be deemed to have a similar or highly similar safety profile to a reference medicinal product. Alternatively, or in addition, a biosimilar as described herein may be deemed to have similar or highly similar efficacy to a reference medicinal product. As described herein, a biosimilar in Europe is compared to a reference medicinal product which has been authorised by the EMA However, in some instances, the biosimilar may be compared to a biological medicinal product which has been authorised outside the European Economic Area (a non-EEA authorised "comparator") in certain studies. Such studies include for example certain clinical and in vivo non-clinical studies. As used herein, the term "biosimilar" also relates to a biological medicinal product which has been or may be compared to a non-EEA authorised comparator. Certain biosimilars are proteins such as antibodies, antibody fragments (for example, antigen binding portions) and fusion proteins. A protein biosimilar may have an amino acid sequence that has minor modifications in the amino acid structure (including for example deletions, additions, and/or substitutions of amino acids) which do not significantly affect the function of the polypeptide. The biosimilar may comprise an amino acid sequence having a sequence identity of 97% or greater to the amino acid sequence of its reference medicinal product, e.g., 97%, 98%, 99% or 100%. The biosimilar may comprise one or more post-translational modifications, for example, although not limited to, glycosylation, oxidation, deamidation, and/or truncation which is/are different to the post-translational modifications of the reference medicinal product, provided that the differences do not result in a change in safety and/or efficacy of the medicinal product. The biosimilar may have an identical or different glycosylation pattern to the reference medicinal product. Particularly, although not exclusively, the biosimilar may have a different glycosylation pattern if the differences address or are intended to address safety concerns associated with the reference medicinal product. Additionally, the biosimilar may deviate from the reference medicinal product in for example its strength, pharmaceutical form, formulation, excipients and/or presentation, providing safety and efficacy of the medicinal product is not compromised. The biosimilar may comprise differences in for example pharmacokinetic (PK) and/or pharmacodynamic (PD) profiles as compared to the reference medicinal product but is still deemed sufficiently similar to the reference medicinal product as to be authorised or considered suitable for authorisation. In certain circumstances, the biosimilar exhibits different binding characteristics as compared to the reference medicinal product, wherein the different binding characteristics are considered by a Regulatory Authority such as the EMA not to be a barrier for authorisation as a similar biological product. The term "biosimilar" is also used synonymously by other national and regional regulatory agencies.

The term "antifolate compound" as used herein includes a compound inhibits one or more folate-requiring enzymes of the thymidine and purine biosynthetic pathways, including thymidylate synthase (TS), dihydrofolate reductase (DHFR), and glycinamide ribonucleotide formyltransferase.

For the avoidance of doubt, it is intended herein that particular features (for example integers, characteristics, values, uses, diseases, formulae, compounds or groups) described in conjunction with a particular aspect, embodiment or example of the invention are to be understood as applicable to any other aspect, embodiment or example described herein unless incompatible therewith. Thus such features may be used where appropriate in conjunction with any of the definition, claims or embodiments defined herein. All of the features disclosed in this specification (including any accompanying claims, abstract and drawings), and/or all of the steps of any method or process so disclosed, may be combined in any combination, except combinations where at least some of the features and/or steps are mutually exclusive. The invention is not restricted to any details of any disclosed embodiments.

## Co-administration of Compounds

In an embodiment, the invention includes a composition, such as a pharmaceutical composition, comprising a combination of a BTK inhibitor of formula (2) and methotrexate, for use in treating rheumatoid arthritis.

Another aspect of the invention is a kit containing a BTK inhibitor of formula (2) and methotraxate, for use in treating rheumatoid arthritis, wherein each of the inhibitor and methotrexate is formulated into a separate pharmaceutical composition, and wherein said separate pharmaceutical compositions are formulated for co-administration.

Another aspect of the invention is a combination of a BTK inhibitor of formula (2) and methotrexate for use in the treatment of rheumatoid arthritis In an embodiment, the foregoing use exhibits synergistic effects that may result in greater efficacy, less side effects, the use of less active pharmaceutical ingredient to achieve a given clinical result, or other synergistic effects. A combination of a BTK inhibitor of formula (2) and methotrexate is a preferred embodiment. The pharmaceutical composition comprising the combination, and the kit, are both for use in treating such disease or condition.

In an embodiment, the combination of the BTK inhibitor of formula (2) and methotrexate is administered by

oral, intravenous, intramuscular, intraperitoneal, subcutaneous, or transdermal means.

In an embodiment, the BTK inhibitor of formula (2) is in the form of a pharmaceutically acceptable salt.

In an embodiment, methotrexate is in the form of a pharmaceutically acceptable salt.

In an embodiment, methotrexate is administered to the subject before administration of the BTK inhibitor of formula (2).

In an embodiment, methotrexate is administered concurrently with the administration of the BTK inhibitor of formula (2).

In an embodiment, methotrexate is administered to the subject after administration of the BTK inhibitor of formula (2).

In a preferred embodiment, the subject is a mammal, such as a human. In an embodiment, the subject is a human. In an embodiment, the subject is a companion animal. In an embodiment, the subject is a canine, feline, or equine.

<u>BTK Inhibitors</u>

The BTK inhibitor is a compound of formula (2) as described in more detail in the following paragraphs. The BTK inhibitor is a compound of Formula (2):

Formula (2)

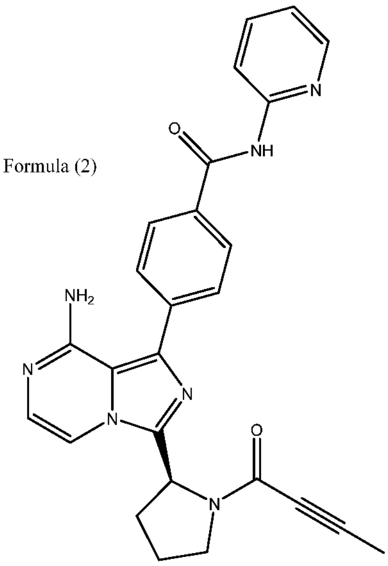

or a pharmaceutically acceptable salt thereof. The preparation of this compound is described in International Patent Application Publication No. WO 2013/010868 and U.S. Patent Application Publication No. US 2014/0155385 A1.

In a preferred embodiment, the BTK inhibitor is (*S*)-4-(8-amino-3-(1-(but-2-ynoyl)pyrrolidin-2-yl)imidazo[1,5-*a*]pyrazin-1-yl)-*N*-(pyridin-2-yl)benzamide or a pharmaceutically acceptable salt thereof.

<u>Antifolates</u>

The antifolate compound is the antifolate compound described in more detail in the following paragraph. In preferred embodiments, the compositions described herein provide a combination of methotrexate with a BTK inhibitor of formula (2), and a use of the combination in the treatment of rheumatoid arthritis. In an embodiment, an antifolate compound inhibits thymidylate synthase (TS), dihydrofolate reductase (DHFR), glycinamide ribonucleotide formyltransferase (GARFT), and combinations thereof.

The antifolate compound is (2*S*)-2-[(4-{[(2,4-Diaminopteridin-6-yl)methyl](methyl)amino}benzoyl)amino]pentanedioic acid ("methotrexate," also known as amethopterin) having the structure of Formula (26):

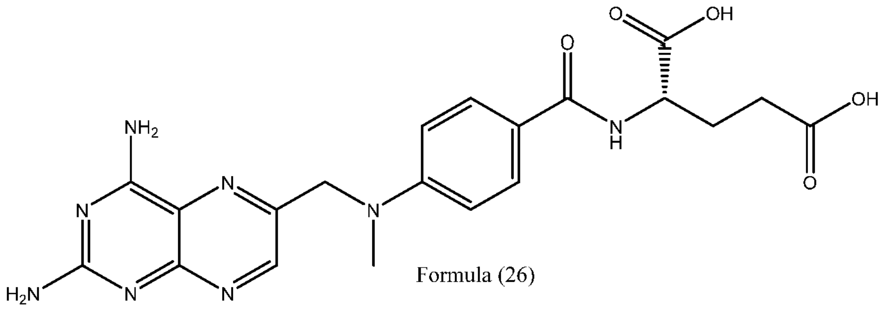

Formula (26)

or a pharmaceutically acceptable salt thereof. The synthesis and properties of methotrexate are described in U.S. Patent No. 2,512,572. Methotrexate is commercially available from multiple suppliers under brandnames such as TREXALL, RHEUMATREX, OTREXUP, and RASUVO.

Pharmaceutical Compositions

In one embodiment, the invention provides a pharmaceutical composition for use in the treatment of rheumatoid arthritis..

In some embodiments, the invention provides a composition comprising therapeutically effective amounts of (1) methotrexate or a pharmaceutically acceptable salt thereof; and (2) a BTK inhibitor having the structure:

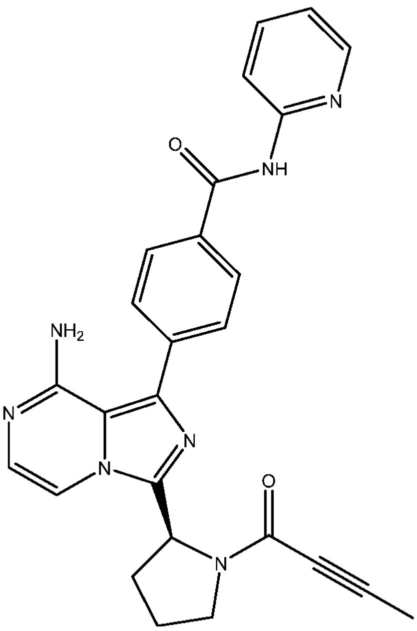

or a pharmaceutically acceptable salt thereof. This composition is typically a pharmaceutical composition.

In some embodiments, the invention provides a composition comprising therapeutically effective amounts of (1) methotrexate and pharmaceutically acceptable saltsthereof; and (2) a BTK inhibitor of formula (2) or a pharmaceutically acceptable salt thereof, for use in treating rheumatoid arthritis. This composition is typically a pharmaceutical composition.

The pharmaceutical compositions are typically formulated to provide a therapeutically effective amount of a combination as described herein, *i.e.,* a combination of methotrexate and a BTK inhibitor of formula (2) as the active ingredients, or pharmaceutically acceptable salts thereof. Where desired, the pharmaceutical compositions contain a pharmaceutically acceptable salt and/or coordination complex of one or more of the active ingredients. Typically, the pharmaceutical compositions also comprise one or more pharmaceutically acceptable excipients, carriers, including inert solid diluents and fillers, diluents, including sterile aqueous solution and various organic solvents, permeation enhancers, solubilizers and adjuvants.

The pharmaceutical compositions described above are for use in the treatment of rheumatoid arthritis.

The pharmaceutical compositions may be administered as a combination of methotrexate and a BTK inhibitor of formula (2). Where desired, other active pharmaceutical ingredient(s) may be mixed into a preparation or two or more components of the combination may be formulated into separate preparations for use in combination separately or at

the same time. A kit containing the components of the combination, formulated into separate preparations for said use, in also provided for use in the invention.

In an embodiment, the molar ratio of methotrexate to the BTK inhibitor of formula (2) in the pharmaceutical compositions is in the range from about 10:1 to about 1:20, preferably from about 2.5:1 to about 1:2.5, and more preferably about 1:1. In an embodiment, the weight ratio of methotrexate to the BTK inhibitor of formula (2) in the pharmaceutical compositions is selected from the group consisting of about 20:1, about 19:1, about 18:1, about 17:1, about 16:1, about 15:1, about 14:1, about 13:1, about 12:1, about 11:1, about 10:1, about 9:1, about 8:1, about 7:1, about 6:1, about 5:1, about 4:1, about 3:1, about 2:1, about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:16, about 1:17, about 1:18, about 1:19, and about 1:20.

In some embodiments, the concentration of any one or two of methotrexate and BTK inhibitor of formula (2) provided in the pharmaceutical compositions for use in the invention is independently less than, for example, 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v or v/v of the pharmaceutical composition.

In some embodiments, the concentration of any one or two of methotrexate and BTK inhibitor of formula (2) provided in the pharmaceutical compositions for use in the invention is independently greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002% or 0.0001% w/w, w/v, or v/v of the pharmaceutical composition.

In some embodiments, the concentration of any one or two of methotrexate and BTK inhibitor of formula (2) provided in the pharmaceutical compositions is independently in the range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12% or about 1% to about 10% w/w, w/v or v/v of the pharmaceutical composition.

In some embodiments, the concentration of any one or two of methotrexate and BTK inhibitor of formula (2) provided in the pharmaceutical compositions is independently in the range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, about 0.1% to about 0.9% w/w, w/v or v/v of the pharmaceutical composition.

In some embodiments, the amount of any one or two of methotrexate and BTK inhibitor of formula (2) provided in the pharmaceutical compositions is independently equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of any one or two of methotrexate and BTK inhibitor of formula (2) provided in the pharmaceutical compositions is independently more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5 g, 7 g, 7.5 g, 8 g, 8.5 g, 9 g, 9.5 g, or 10 g.

Each of methotrexate and BTK inhibitor of formula (2) is effective over a wide dosage range. For example, in the treatment of adult humans, dosages independently ranging from 0.01 to 1000 mg, from 0.5 to 100 mg, from 1 to 50 mg per day, and from 5 to 40 mg per day are examples of dosages that may be used. The exact dosage will depend upon the route of administration, the form in which the compound is administered, the gender and age of the subject to be treated, the body weight of the subject to be treated, and the preference and experience of the attending physician.

Described below are non-limiting pharmaceutical compositions and methods for preparing the same.

Pharmaceutical Compositions for Oral Administration

In preferred embodiments, the invention provides a pharmaceutical composition for oral administration containing the combination of methotrexate and a BTK inhibitor of formula (2), and a pharmaceutical excipient suitable for oral administration for use in treating rheumatoid arthritis.

In preferred embodiments, the invention provides a solid pharmaceutical composition for oral administration containing: (i) an effective amount of each of methotrexate and a BTK inhibitor of formula (2) in combination and (ii) a pharmaceutical excipient suitable for oral administration for use in treating rheumatoid arthritis. In some embodiments, the composition further contains (iii) an effective amount of a third or fourth active pharmaceutical ingredient.

In some embodiments, the pharmaceutical composition may be a liquid pharmaceutical composition suitable for oral consumption.

Pharmaceutical compositions for use in the invention suitable for oral administration can be presented as discrete dosage forms, such as capsules, sachets, tablets, liquids, or aerosol sprays each containing a predetermined amount of an active ingredient as a powder or in granules, a solution, or a suspension in an aqueous or non-aqueous liquid, an oil-in-water emulsion, a water-in-oil liquid emulsion, powders for reconstitution, powders for oral consumptions, bottles (including powders or liquids in a bottle), orally dissolving films, lozenges, pastes, tubes, gums, and packs. Such dosage forms can be prepared by any of the methods of pharmacy, but all methods include the step of bringing the active ingredient(s) into association with the carrier, which constitutes one or more necessary ingredients. In general, the compositions are prepared by uniformly and intimately admixing the active ingredient(s) with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free-flowing form such as powder or granules, optionally mixed with an excipient such as, but not limited to, a binder, a lubricant, an inert diluent, and/or a surface active or dispersing agent. Molded tablets can be made by molding in a suitable machine a mixture of the powdered compound moistened with an inert liquid diluent.

The invention further encompasses the use of anhydrous pharmaceutical compositions and dosage forms since water can facilitate the degradation of some compounds. For example, water may be added (*e.g.,* 5%) in the pharmaceutical arts as a means of simulating long-term storage in order to determine characteristics such as shelf-life or the stability of formulations over time. Anhydrous pharmaceutical compositions and dosage forms for use in the invention can be prepared using anhydrous or low moisture containing ingredients and low moisture or low humidity conditions. Pharmaceutical compositions and dosage forms for use in the invention which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include, but are not limited to, hermetically sealed foils, plastic or the like, unit dose containers, blister packs, and strip packs.

Each of methotrexate and BTK inhibitor of formula (2) as active ingredients can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, microcrystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques.

Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g.*, ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof.

Examples of suitable fillers for use in the pharmaceutical compositions and dosage forms disclosed herein include, but are not limited to, talc, calcium carbonate (*e.g.*, granules or powder), microcrystalline cellulose, powdered cellulose, dextrates, kaolin, mannitol, silicic acid, sorbitol, starch, pre-gelatinized starch, and mixtures thereof.

Disintegrants may be used in the compositions for use in the invention to provide tablets that disintegrate when exposed to an aqueous environment. Too much of a disintegrant may produce tablets which disintegrate in the bottle. Too little may be insufficient for disintegration to occur, thus altering the rate and extent of release of the active ingredients from the dosage form. Thus, a sufficient amount of disintegrant that is neither too little nor too much to detrimentally

alter the release of the active ingredient(s) may be used to form the dosage forms of the compounds disclosed herein. The amount of disintegrant used may vary based upon the type of formulation and mode of administration, and may be readily discernible to those of ordinary skill in the art. About 0.5 to about 15 weight percent of disintegrant, or about 1 to about 5 weight percent of disintegrant, may be used in the pharmaceutical composition. Disintegrants that can be used to form pharmaceutical compositions and dosage forms for use in the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which can be used to form pharmaceutical compositions and dosage forms for use in the invention include, but are not limited to, calcium stearate, magnesium stearate, sodium stearyl fumarate, mineral oil, light mineral oil, glycerin, sorbitol, mannitol, polyethylene glycol, other glycols, stearic acid, sodium lauryl sulfate, talc, hydrogenated vegetable oil (*e.g.,* peanut oil, cottonseed oil, sunflower oil, sesame oil, olive oil, corn oil, and soybean oil), zinc stearate, ethyl oleate, ethylaureate, agar, or mixtures thereof. Additional lubricants include, for example, a syloid silica gel, a coagulated aerosol of synthetic silica, silicified microcrystalline cellulose, or mixtures thereof. A lubricant can optionally be added in an amount of less than about 0.5% or less than about 1% (by weight) of the pharmaceutical composition.

When aqueous suspensions and/or elixirs are desired for oral administration, the active pharmaceutical ingredient(s) may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Surfactants which can be used to form pharmaceutical compositions and dosage forms for use in the invention include, but are not limited to, hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance ("HLB" value). Surfactants with lower HLB values are more lipophilic or hydrophobic, and have greater solubility in oils, while surfactants with higher HLB values are more hydrophilic, and have greater solubility in aqueous solutions. Hydrophilic surfactants are generally considered to be those compounds having an HLB value greater than about 10, as well as anionic, cationic, or zwitterionic compounds for which the HLB scale is not generally applicable. Similarly, lipophilic (*i.e.,* hydrophobic) surfactants are compounds having an HLB value equal to or less than about 10. However, HLB value of a surfactant is merely a rough guide generally used to enable formulation of industrial, pharmaceutical and cosmetic emulsions.

Hydrophilic surfactants may be either ionic or non-ionic. Suitable ionic surfactants include, but are not limited to, alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teracecyl sulfate, docusate, lauroyl carnitines, palmitoyl carnitines, myristoyl carnitines, and salts and mixtures thereof.

Hydrophilic non-ionic surfactants may include, but not limited to, alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include, without limitation, PEG-10 laurate, PEG-12 laurate, PEG-20 laurate, PEG-32 laurate, PEG-32 dilaurate, PEG-12 oleate, PEG-15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG-100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG-100 succinate, PEG-24 cholesterol, polyglyceryl-10 oleate, Tween 40, Tween 60, sucrose monostearate, sucrose monolaurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; and mixtures thereof. Within this group, preferred lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

In an embodiment, the composition may include a solubilizer to ensure good solubilization and/or dissolution of the compound for use in the present invention and to minimize precipitation of the compound for use in the present invention. This can be especially important for compositions for non-oral use - *e.g.*, compositions for injection. A solubilizer may also be added to increase the solubility of the hydrophilic drug and/or other components, such as surfactants, or to maintain the composition as a stable or homogeneous solution or dispersion.

Examples of suitable solubilizers include, but are not limited to, the following: alcohols and polyols, such as ethanol, isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG; amides and other nitrogen-containing compounds such as 2-pyrrolidone, 2-piperidone, ε-caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, .epsilon.-caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water.

Mixtures of solubilizers may also be used. Examples include, but not limited to, triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, polyethylene glycol 200-100, glycofurol, transcutol, propylene glycol, and dimethyl isosorbide. Particularly preferred solubilizers include sorbitol, glycerol, triacetin, ethyl alcohol, PEG-400, glycofurol and propylene glycol.

The amount of solubilizer that can be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the composition to a patient

using conventional techniques, such as distillation or evaporation. Thus, if present, the solubilizer can be in a weight ratio of 10%, 25%, 50%, 100%, or up to about 200% by weight, based on the combined weight of the drug, and other excipients. If desired, very small amounts of solubilizer may also be used, such as 5%, 2%, 1% or even less. Typically, the solubilizer may be present in an amount of about 1% to about 100%, more typically about 5% to about 25% by weight.

The composition can further include one or more pharmaceutically acceptable additives and excipients. Such additives and excipients include, without limitation, detackifiers, anti-foaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

In addition, an acid or a base may be incorporated into the composition to facilitate processing, to enhance stability, or for other reasons. Examples of pharmaceutically acceptable bases include amino acids, amino acid esters, ammonium hydroxide, potassium hydroxide, sodium hydroxide, sodium hydrogen carbonate, aluminum hydroxide, calcium carbonate, magnesium hydroxide, magnesium aluminum silicate, synthetic aluminum silicate, synthetic hydrocalcite, magnesium aluminum hydroxide, diisopropylethylamine, ethanolamine, ethylenediamine, triethanolamine, triethylamine, triisopropanolamine, trimethylamine, tris(hydroxymethyl)aminomethane (TRIS) and the like. Also suitable are bases that are salts of a pharmaceutically acceptable acid, such as acetic acid, acrylic acid, adipic acid, alginic acid, alkanesulfonic acid, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid, and the like. Salts of polyprotic acids, such as sodium phosphate, disodium hydrogen phosphate, and sodium dihydrogen phosphate can also be used. When the base is a salt, the cation can be any convenient and pharmaceutically acceptable cation, such as ammonium, alkali metals and alkaline earth metals. Example may include, but not limited to, sodium, potassium, lithium, magnesium, calcium and ammonium.

Suitable acids are pharmaceutically acceptable organic or inorganic acids. Examples of suitable inorganic acids include hydrochloric acid, hydrobromic acid, hydriodic acid, sulfuric acid, nitric acid, boric acid, phosphoric acid, and the like. Examples of suitable organic acids include acetic acid, acrylic acid, adipic acid, alginic acid, alkanesulfonic acids, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, methanesulfonic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid and uric acid.

Pharmaceutical Compositions for Injection

In preferred embodiments, the invention provides the use of a pharmaceutical composition for injection containing the combination of methotrexate and the BTK inhibitor of formula (2), and a pharmaceutical excipient suitable for injection. Components and amounts of agents in the compositions are as described herein.

The forms in which the compositions for use in the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline are also conventionally used for injection. Ethanol, glycerol, propylene glycol and liquid polyethylene glycol (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid and thimerosal.

Sterile injectable solutions are prepared by incorporating the combination of methotrexate and BTK inhibitor of formula (2) in the required amounts in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain desirable methods of preparation are vacuum-drying and freeze-drying techniques which yield a powder of the active ingredient plus any additional desired ingredient from a previously sterile-filtered solution thereof.

Pharmaceutical Compositions for Topical Delivery

In preferred embodiments, the invention provides the use of a pharmaceutical composition for transdermal delivery containing the combination of methotrexate and the BTK inhibitor of formula (2), and a pharmaceutical excipient suitable for transdermal delivery.

Compositions for use in the present invention can be formulated into preparations in solid, semi-solid, or liquid forms suitable for local or topical administration, such as gels, water soluble jellies, creams, lotions, suspensions, foams, powders, slurries, ointments, solutions, oils, pastes, suppositories, sprays, emulsions, saline solutions, dimethylsulfoxide (DMSO)-based solutions. In general, carriers with higher densities are capable of providing an area with a prolonged exposure to the active ingredients. In contrast, a solution formulation may provide more immediate exposure of the active ingredient to the chosen area.

The pharmaceutical compositions also may comprise suitable solid or gel phase carriers or excipients, which are compounds that allow increased penetration of, or assist in the delivery of, therapeutic molecules across the stratum corneum permeability barrier of the skin. There are many of these penetration-enhancing molecules known to those trained in the art of topical formulation. Examples of such carriers and excipients include, but are not limited to, humectants (*e.g.,* urea), glycols (*e.g.,* propylene glycol), alcohols (*e.g.,* ethanol), fatty acids (*e.g.,* oleic acid), surfactants (*e.g.*, isopropyl myristate and sodium lauryl sulfate), pyrrolidones, glycerol monolaurate, sulfoxides, terpenes (*e.g.*, menthol), amines, amides, alkanes, alkanols, water, calcium carbonate, calcium phosphate, various sugars, starches, cellulose derivatives, gelatin, and polymers such as polyethylene glycols.

Another exemplary formulation for use in the the present invention employs transdermal delivery devices ("patches"). Such transdermal patches may be used to provide continuous or discontinuous infusion of the combination of methotrexate and BTK inhibitors of formula (2) in controlled amounts, either with or without another active pharmaceutical ingredient.

The construction and use of transdermal patches for the delivery of pharmaceutical agents is well known in the art. See, *e.g.,* U.S. Patent Nos. 5,023,252; 4,992,445 and 5,001,139. Such patches may be constructed for continuous, pulsatile, or on demand delivery of pharmaceutical agents.

Pharmaceutical Compositions for Inhalation

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices that deliver the formulation in an appropriate manner. Dry powder inhalers may also be used to provide inhaled delivery of the compositions.

Other Pharmaceutical Compositions

Pharmaceutical compositions may also be prepared from compositions described herein and one or more pharmaceutically acceptable excipients suitable for sublingual, buccal, rectal, intraosseous, intraocular, intranasal, epidural, or intraspinal administration. Preparations for such pharmaceutical compositions are well-known in the art. See, *e.g.*, Anderson, Philip O.; Knoben, James E.; Troutman, William G, eds., Handbook of Clinical Drug Data, Tenth Edition, McGraw-Hill, 2002; and Pratt and Taylor, eds., Principles of Drug Action, Third Edition, Churchill Livingston, N.Y., 1990.

Administration of the combination of methotrexate and the BTK inhibitor of formula (2) or pharmaceutical composition of these compounds can be effected by any method that enables delivery of the compounds to the site of action. These methods include oral routes, intraduodenal routes, parenteral injection (including intravenous, intraarterial, subcutaneous, intramuscular, intravascular, intraperitoneal or infusion), topical (*e.g.*, transdermal application), rectal administration, via local delivery by catheter or stent or through inhalation. The combination of compounds can also be administered intraadiposally or intrathecally.

The compositions for use in the invention may also be delivered via an impregnated or coated device such as a stent, for example, or an artery-inserted cylindrical polymer. Such a method of administration may, for example, aid in the prevention or amelioration of restenosis following procedures such as balloon angioplasty. Without being bound by theory, compounds for use in the invention may slow or inhibit the migration and proliferation of smooth muscle cells in the arterial wall which contribute to restenosis. A compound for use in the invention may be administered, for example, by local delivery from the struts of a stent, from a stent graft, from grafts, or from the cover or sheath of a stent. In some embodiments, a compound for use in the invention is admixed with a matrix. Such a matrix may be a polymeric matrix, and may serve to bond the compound to the stent. Polymeric matrices suitable for such use, include, for example, lactone-based polyesters or copolyesters such as polylactide, polycaprolactonglycolide, polyorthoesters, polyanhydrides, polyaminoacids, polysaccharides, polyphosphazenes, poly(ether-ester) copolymers (*e.g.*, PEO-PLLA); polydimethylsiloxane, poly(ethylene-vinylacetate), acrylate-based polymers or copolymers (*e.g.*, polyhydroxyethyl methylmethacrylate, polyvinyl pyrrolidinone), fluorinated polymers such as polytetrafluoroethylene and cellulose esters. Suitable matrices may be nondegrading or may degrade with time, releasing the compound or compounds. The combination of methotrexate and BTK inhibitors of formula (2) may be applied to the surface of the stent by various methods such as dip/spin coating, spray coating, dip-coating, and/or brush-coating. The compounds may be applied in a solvent and the solvent may be allowed to evaporate, thus forming a layer of compound onto the stent. Alternatively, the compound may be located in the body of the stent or graft, for example in microchannels or micropores. When implanted, the compound diffuses out of the body of the stent to contact the arterial wall. Such stents may be prepared by dipping a stent manufactured to contain such micropores or microchannels into a solution of the compound for use in the invention in a suitable solvent, followed by evaporation of the solvent. Excess drug on the surface of the stent may be removed via an additional brief solvent wash. In yet other embodiments, compounds for use in the invention may be covalently linked to a stent or graft. A covalent linker may be used which degrades *in vivo,* leading to the release of the compound for use in the invention. Any bio-labile linkage may be used for such a purpose, such as ester, amide or anhydride linkages. The combination of methotrexate and the BTK inhibitor of formula (2) may additionally be administered intravascularly from a balloon used during angioplasty. Extravascular administration of the combination of methotrexate and the BTK inhibitor of formula (2) via the pericard or via advential application of formulations for use in the invention may also be performed to decrease restenosis.

Exemplary parenteral administration forms include solutions or suspensions of active compound in sterile aqueous solutions, for example, aqueous propylene glycol or dextrose solutions. Such dosage forms can be suitably buffered, if desired.

The invention also provides kits as defined above for use in treating rheumatoid arthritis. The kits include each of methotrexate and the BTK inhibitor of formula (2), either alone or in combination in suitable packaging, and written material that can include instructions for use, discussion of clinical studies and listing of side effects. Such kits may also include information, such as scientific literature references, package insert materials, clinical trial results, and/or summaries of these and the like, which indicate or establish the activities and/or advantages of the composition, and/or which describe dosing, administration, side effects, drug interactions, or other information useful to the health care provider. Such information may be based on the results of various studies, for example, studies using experimental animals involving in vivo models and studies based on human clinical trials. The kit may further contain another active pharmaceutical ingredient. In selected embodiments, methotrexate and the BTK inhibitor of formula (2) and another active pharmaceutical ingredient are provided as separate compositions in separate containers within the kit. In selected embodiments, methotrexate and The BTK inhibitor of formula (2) and the agent are provided as a single composition within a container in the kit. Suitable packaging and additional articles for use (*e.g.*, measuring cup for liquid preparations, foil wrapping to minimize exposure to air, and the like) are known in the art and may be included in the kit. Kits described herein can be provided, marketed and/or promoted to health providers, including physicians, nurses, pharmacists, formulary officials, and the like. Kits may also, in selected embodiments, be marketed directly to the consumer.

In some embodiments, the invention provides a kit for use in the treatment of rheumatoid arthritis comprising (1) a composition comprising a therapeutically effective amount of methotrexate or pharmaceutically acceptable salt thereof, and (2) a composition comprising a therapeutically effective amount of a BTK inhibitor of formula (2) or a pharmaceutically acceptable salt thereof. These compositions are typically pharmaceutical compositions. The kit is for co-administration of the methotrexate and the BTK inhibitor of formula (2), either simultaneously or separately.

The kits described above are for use in the treatment of rheumatoid arthritis.

Dosages and Dosing Regimens

The amounts of BTK inhibitor of formula (2) and methotrexate administered will be dependent on the human or mammal being treated, the severity of the disorder or condition, the rate of administration, the disposition of the compounds and the discretion of the prescribing physician. However, an effective dosage of each is in the range of about 0.001 to about 100 mg per kg body weight per day, such as about 1 to about 35 mg/kg/day, in single or divided doses. For a 70 kg human, this would amount to about 0.05 to 7 g/day, such as about 0.05 to about 2.5 g/day. In some instances, dosage levels below the lower limit of the aforesaid range may be more than adequate, while in other cases still larger doses may be employed without causing any harmful side effect - *e.g.,* by dividing such larger doses into several small doses for administration throughout the day. The dosage of BTK inhibitor of formula (2) and methotrexate may be provided in units of mg/kg of body mass or in mg/m$^2$ of body surface area. In an embodiment, the ratio of the dose of methotrexate to the dose of the BTK inhibitor of formula (2) in mg/kg or in mg/m$^2$ is in the range from 10:1 to 1:10, preferably from 2.5:1 to 1:2.5, and more preferably about 1:1. In an embodiment, the ratio of methotrexate to the BTK inhibitor of formula (2) in mg/kg or in mg/m$^2$ is selected from the group consisting of about 20:1, about 19:1, about 18:1, about 17:1, about 16:1, about 15:1, about 14:1, about 13:1, about 12:1, about 11:1, about 10:1, about 9:1, about 8:1, about 7:1, about 6:1, about 5:1, about 4:1, about 3:1, about 2:1, about 1:1, about 1:2, about 1:3, about 1:4, about 1:5, about 1:6, about 1:7, about 1:8, about 1:9, about 1:10, about 1:11, about 1:12, about 1:13, about 1:14, about 1:15,

about 1:16, about 1:17, about 1:18, about 1:19, and about 1:20.

In some embodiments, the combination of methotrexate and BTK inhibitor of formula (2) is administered in a single dose. Such administration may be by injection, *e.g.*, intravenous injection, in order to introduce methotrexate and BTK inhibitor of formula (2) quickly. However, other routes, including the preferred oral route, may be used as appropriate. A single dose of the combination of methotrexate and BTK inhibitor of formula (2) may also be used for treatment of an acute condition.

In some embodiments, the combination of methotrexate and BTK inhibitor of formula (2) is administered in multiple doses. In a preferred embodiment, the combination of methotrexate and BTK inhibitor of formula (2) is administered in multiple doses. Dosing may be once, twice, three times, four times, five times, six times, or more than six times per day. Dosing may be once a month, once every two weeks, once a week, or once every other day. In other embodiments, the combination of methotrexate and BTK inhibitor of formula (2) is administered about once per day to about 6 times per day. In some embodiments, the combination of methotrexate and BTK inhibitor of formula (2) is administered once daily, while in other embodiments, the combination methotrexate and BTK inhibitor of formula (2) is administered twice daily, and in other embodiments the combination of methotrexate and BTK inhibitor of formula (2) is administered three times daily.

Administration of the active pharmaceutical ingredients may continue as long as necessary. In selected embodiments, the combination of methotrexate and BTK inhibitor of formula (2) is administered for more than 1, 2, 3, 4, 5, 6, 7, 14, or 28 days. In some embodiments, the combination of methotrexate and BTK inhibitor of formula (2) is administered for less than 28, 14, 7, 6, 5, 4, 3, 2, or 1 day. In selected embodiments, the combination of methotrexate and BTK inhibitor of formula (2) is administered chronically on an ongoing basis - *e.g.,* for the treatment of chronic effects. In another embodiment the administration of the combination of methotrexate and BTK inhibitor of formula (2) continues for less than about 7 days. In yet another embodiment the administration continues for more than about 6, 10, 14, 28 days, two months, six months, or one year. In some cases, continuous dosing is achieved and maintained as long as necessary.

In some embodiments, an effective dosage of a BTK inhibitor of formula (2) disclosed herein is in the range of about 1 mg to about 500 mg, about 10 mg to about 300 mg, about 20 mg to about 250 mg, about 25 mg to about 200 mg, about 10 mg to about 200 mg, about 20 mg to about 150 mg, about 30 mg to about 120 mg, about 10 mg to about 90 mg, about 20 mg to about 80 mg, about 30 mg to about 70 mg, about 40 mg to about 60 mg, about 45 mg to about 55 mg, about 48 mg to about 52 mg, about 50 mg to about 150 mg, about 60 mg to about 140 mg, about 70 mg to about 130 mg, about 80 mg to about 120 mg, about 90 mg to about 110 mg, about 95 mg to about 105 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 202 mg. In some embodiments, an effective dosage of a BTK inhibitor of formula (2) disclosed herein is about 25 mg, about 50 mg, about 75 mg, about 100 mg, about 125 mg, about 150 mg, about 175 mg, about 200 mg, about 225 mg, or about 250 mg.

In some embodiments, an effective dosage of a BTK inhibitor of formula (2) disclosed herein is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.15 mg/kg to about 2.85 mg/kg, about 0.3 mg to about 2.15 mg/kg, about 0.45 mg/kg to about 1.7 mg/kg, about 0.15 mg/kg to about 1.3 mg/kg, about 0.3 mg/kg to about 1.15 mg/kg, about 0.45 mg/kg to about 1 mg/kg, about 0.55 mg/kg to about 0.85 mg/kg, about 0.65 mg/kg to about 0.8 mg/kg, about 0.7 mg/kg to about 0.75 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg mg to about 1.6 mg/kg, about 1.35 mg/kg to about 1.5 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg. In some embodiments, an effective dosage of a BTK inhibitor of formula (2) disclosed herein is about 0.35 mg/kg, about 0.7 mg/kg, about 1 mg/kg, about 1.4 mg/kg, about 1.8 mg/kg, about 2.1 mg/kg, about 2.5 mg/kg, about 2.85 mg/kg, about 3.2 mg/kg, or about 3.6 mg/kg.

In some embodiments, an effective dosage of methotrexate is in the range of about 1 mg to about 500 mg, about 10 mg to about 300 mg, about 20 mg to about 250 mg, about 25 mg to about 200 mg, about 1 mg to about 50 mg, about 5 mg to about 45 mg, about 10 mg to about 40 mg, about 15 mg to about 35 mg, about 20 mg to about 30 mg, about 23 mg to about 28 mg, about 50 mg to about 150 mg, about 60 mg to about 140 mg, about 70 mg to about 130 mg, about 80 mg to about 120 mg, about 90 mg to about 110 mg, or about 95 mg to about 105 mg, about 98 mg to about 102 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 207 mg. In some embodiments, an effective dosage of methotrexate is about 25 mg, about 50 mg, about 75 mg, about 100 mg, about 125 mg, about 150 mg, about 175 mg, about 200 mg, about 225 mg, or about 250 mg.

In some embodiments, an effective dosage of methotrexate is in the range of about 0.01 mg/kg to about 4.3 mg/kg, about 0.15 mg/kg to about 3.6 mg/kg, about 0.3 mg/kg to about 3.2 mg/kg, about 0.35 mg/kg to about 2.85 mg/kg, about 0.01 mg/kg to about 0.7 mg/kg, about 0.07 mg/kg to about 0.65 mg/kg, about 0.15 mg/kg to about 0.6 mg/kg,

about 0.2 mg/kg to about 0.5 mg/kg, about 0.3 mg/kg to about 0.45 mg/kg, about 0.3 mg/kg to about 0.4 mg/kg, about 0.7 mg/kg to about 2.15 mg/kg, about 0.85 mg/kg to about 2 mg/kg, about 1 mg/kg to about 1.85 mg/kg, about 1.15 mg/kg to about 1.7 mg/kg, about 1.3 mg/kg to about 1.6 mg/kg, about 1.35 mg/kg to about 1.5 mg/kg, about 1.4 mg/kg to about 1.45 mg/kg, about 2.15 mg/kg to about 3.6 mg/kg, about 2.3 mg/kg to about 3.4 mg/kg, about 2.4 mg/kg to about 3.3 mg/kg, about 2.6 mg/kg to about 3.15 mg/kg, about 2.7 mg/kg to about 3 mg/kg, about 2.8 mg/kg to about 3 mg/kg, or about 2.85 mg/kg to about 2.95 mg/kg. In some embodiments, an effective dosage of methotrexate is about 0.4 mg/kg, about 0.7 mg/kg, about 1 mg/kg, about 1.4 mg/kg, about 1.8 mg/kg, about 2.1 mg/kg, about 2.5 mg/kg, about 2.85 mg/kg, about 3.2 mg/kg, or about 3.6 mg/kg.

In some embodiments, a combination of a BTK inhibitor of formula (2) and methotrexate is administered at a dosage of 10 to 200 mg BID, including 10, 20, 30, 40, 50, 60, 70, 80, 90, 100, 150, or 200 mg BID, for the BTK inhibitor of formula (2), and 10 to 200 mg BID, including 25, 50, 75, 100, 150, or 200 mg BID for methotrexate.

In some embodiments, a combination of a BTK inhibitor of formula (2) and methotrexate is administered at a dosage of 10 to 200 mg BID, including 10, 20, 30, 40, 50, 60, 70, 80, 90, 100, or 150 mg BID, for the BTK inhibitor of formula (2), and 1 to 500 mg BID, including 1, 5, 10, 15, 25, 50, 75, 100, 150, 200, 300, 400, or 500 mg BID for methotrexate inhibitor.

In some instances, dosage levels below the lower limit of the aforesaid ranges may be more than adequate, while in other cases still larger doses may be employed without causing any harmful side effect - *e.g.,* by dividing such larger doses into several small doses for administration throughout the day.

An effective amount of the combination of methotrexate and BTK inhibitor of formula (2) may be administered in either single or multiple doses by any of the accepted modes of administration of agents having similar utilities, including rectal, buccal, intranasal and transdermal routes, by intra-arterial injection, intravenously, intraperitoneally, parenterally, intramuscularly, subcutaneously, orally, topically, or as an inhalant.

Treatment of Inflammation, Immune and Autoimmune Disorders, and Other Diseases

The compositions and combinations of inhibitors described above can be used in the treatment of BTK-mediated disorders and diseases. Specifically, the present invention provides compositions and combinations described herein for use in the treatment of rheumatoid arthritis.

Efficacy of the methods, compounds, and combinations of compounds described herein in treating, preventing and/or managing the indicated diseases or disorders can be tested using various animal models known in the art. Efficacy in treating, preventing and/or managing arthritis (*e.g.*, rheumatoid or psoriatic arthritis) can be assessed using the autoimmune animal models described in, for example, Williams, et al., Chem. Biol. 2010, 17, 123-34, WO 2009/088986, WO 2009/088880, and WO 2011/008302.

Use in Treating Patients Intolerant to Bleeding Events

In selected embodiments, the invention provides compositions and combinations for use in the treatment of rheumatoid arthritis in a human sensitive to or intolerant to bleeding events, comprising the step of administering a therapeutically effective amount of a BTK inhibitor of formula (2), or a pharmaceutically-acceptable salt thereof, and methotrexate or a pharmaceutically-acceptable salt thereof.

Combinations of BTK Inhibitors, Antifolate Compounds, and Anti-CD20 Antibodies

The BTK inhibitor with antifolate compound for use in the present invention may also be safely co-administered with immunotherapeutic antibodies such as the anti-CD20 antibodies rituximab, obinutuzumab, ofatumumab, veltuzumab, tositumomab, and ibritumomab, and or antigen-binding fragments, derivatives, conjugates, variants, and radioisotope-labeled complexes thereof. In an embodiment, the foregoing combinations exhibit synergistic effects that may result in greater efficacy, less side effects, the use of less active pharmaceutical ingredient to achieve a given clinical result, or other synergistic effects.

Anti-CD20 monoclonal antibodies are classified as Type I or Type II, as described in Klein, et al., mAbs 2013, 5, 22-33. Type I anti-CD20 monoclonal antibodies are characterized by binding to the Class I epitope, localization of CD20 to lipid rafts, high complement-dependent cytotoxicity, full binding capacity, weak homotypic aggregation, and moderate cell death induction. Type II anti-CD20 monoclonal antibodies are characterized by binding to the Class I epitope, a lack of localization of CD20 to lipid rafts, low complement-dependent cytotoxicity, half binding capacity, homotypic aggregation, and strong cell death induction. Both Type I and Type II anti-CD20 monoclonal antibodies exhibit antibody-dependent cytotoxiticy (ADCC) and are thus useful with the BTK inhibitor of formula (2) described herein. Type I anti-CD20 monoclonal antibodies include but are not limited to rituximab, ocrelizumab, and ofatumumab. Type II anti-CD20 monoclonal antibodies include but are not limited to obinutuzumab and tositumomab. In an embodiment, the

foregoing methods exhibit synergistic effects that may result in greater efficacy, less side effects, the use of less active pharmaceutical ingredient to achieve a given clinical result, or other synergistic effects.

In an embodiment, the invention provides combinations and compositions for use in treating rheumatoid arthritis in a human, the use comprising the step of administering to said human a BTK inhibitor of formula (2) and a pharmaceutically acceptable salt thereof, and methotrexate, and further comprising the step of administering an anti-CD20 antibody, wherein the anti-CD20 antibody is a monoclonal antibody or an antigen-binding fragment, derivative, conjugate, variant, or radioisotope-labeled complex thereof.

In selected embodiments, the combinations of the BTK inhibitor of formula (2) with methotrexate, and the anti-CD20 monoclonal antibody are administered sequentially. In selected embodiments, the combinations of the BTK inhibitor of formula (2) with methotrexate, and the anti-CD20 monoclonal antibody are administered concomitantly. In selected embodiments, the combinations of the BTK inhibitor of formula (2) with methotrexate are administered before the anti-CD20 monoclonal antibody. In selected embodiments, the combinations of the BTK inhibitor of formula (2) with methotrexate are administered after the anti-CD20 monoclonal antibody. In selected embodiments, the combinations of the BTK inhibitor of formula (2) with methotrexate and the anti-CD20 monoclonal antibody are administered over the same time period, and the BTK inhibitor of formula (2) administration continues after the anti-CD20 monoclonal antibody administration is completed.

In an embodiment, the anti-CD20 monoclonal antibody is rituximab, or an antigen-binding fragment, derivative, conjugate, variant, or radioisotope-labeled complex thereof. Rituximab is a chimeric murine-human monoclonal antibody directed against CD20, and its structure comprises an IgG1 kappa immunoglobulin containing murine light- and heavy-chain variable region sequences and human constant region sequences. Rituximab is composed of two heavy chains of 451 amino acids and two light chains of 213 amino acids. The amino acid sequence for the heavy chains of rituximab is set forth in SEQ ID NO:1. The amino acid sequence for the light chains of rituximab is set forth in SEQ ID NO:2. Rituximab is commercially available, and its properties and use in cancer and other diseases is described in more detail in Rastetter, et al., Ann. Rev. Med. 2004, 55, 477-503, and in Plosker and Figgett, Drugs, 2003, 63, 803-43. In an embodiment, the anti-CD20 monoclonal antibody is an anti-CD20 biosimilar monoclonal antibody approved by drug regulatory authorities with reference to rituximab. In an embodiment, the anti-CD20 monoclonal antibody has a heavy chain sequence identity of greater than 90% to SEQ ID NO:1. In an embodiment, the anti-CD20 monoclonal antibody has a light chain sequence identity of greater than 90% to SEQ ID NO:2. In an embodiment, the anti-CD20 monoclonal antibody has a heavy chain sequence identity of greater than 95% to SEQ ID NO:1. In an embodiment, the anti-CD20 monoclonal antibody has a light chain sequence identity of greater than 95% to SEQ ID NO:2. In an embodiment, the anti-CD20 monoclonal antibody has a heavy chain sequence identity of greater than 98% to SEQ ID NO:1. In an embodiment, the anti-CD20 monoclonal antibody has a light chain sequence identity of greater than 98% to SEQ ID NO:2. In an embodiment, the anti-CD20 monoclonal antibody has a heavy chain sequence identity of greater than 99% to SEQ ID NO:1. In an embodiment, the anti-CD20 monoclonal antibody has a light chain sequence identity of greater than 99% to SEQ ID NO:2.

In an embodiment, the anti-CD20 monoclonal antibody is obinutuzumab, or an antigen-binding fragment, derivative, conjugate, variant, or radioisotope-labeled complex thereof. Obinutuzumab is also known as afutuzumab or GA-101. Obinutuzumab is a humanized monoclonal antibody directed against CD20. The amino acid sequence for the heavy chains of obinutuzumab is set forth in SEQ ID NO:3. The amino acid sequence for the light chains of obinutuzumab is set forth in SEQ ID NO:4. Obinutuzumab is commercially available, and its properties and use in cancer and other diseases is described in more detail in Robak, Curr. Opin. Investig. Drugs 2009, 10, 588-96. In an embodiment, the anti-CD20 monoclonal antibody is an anti-CD20 biosimilar monoclonal antibody approved by drug regulatory authorities with reference to obinutuzumab. In an embodiment, the anti-CD20 monoclonal antibody has a heavy chain sequence identity of greater than 90% to SEQ ID NO:3. In an embodiment, the anti-CD20 monoclonal antibody has a light chain sequence identity of greater than 90% to SEQ ID NO:4. In an embodiment, the anti-CD20 monoclonal antibody has a heavy chain sequence identity of greater than 95% to SEQ ID NO:3. In an embodiment, the anti-CD20 monoclonal antibody has a light chain sequence identity of greater than 95% to SEQ ID NO:4. In an embodiment, the anti-CD20 monoclonal antibody has a heavy chain sequence identity of greater than 98% to SEQ ID NO:3. In an embodiment, the anti-CD20 monoclonal antibody has a light chain sequence identity of greater than 98% to SEQ ID NO:4. In an embodiment, the anti-CD20 monoclonal antibody has a heavy chain sequence identity of greater than 99% to SEQ ID NO:3. In an embodiment, the anti-CD20 monoclonal antibody has a light chain sequence identity of greater than 99% to SEQ ID NO:4. In an embodiment, the anti-CD20 monoclonal antibody obinutuzumab is an immunoglobulin G1, anti-(human B-lymphocyte antigen CD20 (membrane-spanning 4-domains subfamily A member 1, B-lymphocyte surface antigen B1, Leu-16 or Bp35)), humanized mouse monoclonal obinutuzumab des-CH3107-K-$\gamma$1 heavy chain (222-219')-disulfide with humanized mouse monoclonal obinutuzumab $\kappa$ light chain dimer (228-228":231-231")-bisdisulfide antibody.

In an embodiment, the anti-CD20 monoclonal antibody is ofatumumab, or an antigen-binding fragment, derivative, conjugate, variant, or radioisotope-labeled complex thereof. Ofatumumab is described in Cheson, J. Clin. Oncol. 2010, 28, 3525-30. The crystal structure of the Fab fragment of ofatumumab has been reported in Protein Data Bank

reference 3GIZ and in Du, et al., Mol. Immunol. 2009, 46, 2419-2423. Ofatumumab is commercially available, and its preparation, properties, and use in cancer and other diseases are described in more detail in U.S. Patent No. 8,529,202 B2. In an embodiment, the anti-CD20 monoclonal antibody is an anti-CD20 biosimilar monoclonal antibody approved by drug regulatory authorities with reference to ofatumumab. In an embodiment, the anti-CD20 monoclonal antibody has a variable heavy chain sequence identity of greater than 90% to SEQ ID NO:5. In an embodiment, the anti-CD20 monoclonal antibody has a variable light chain sequence identity of greater than 90% to SEQ ID NO:6. In an embodiment, the anti-CD20 monoclonal antibody has a variable heavy chain sequence identity of greater than 95% to SEQ ID NO:5. In an embodiment, the anti-CD20 monoclonal antibody has a variable light chain sequence identity of greater than 95% to SEQ ID NO:6. In an embodiment, the anti-CD20 monoclonal antibody has a variable heavy chain sequence identity of greater than 98% to SEQ ID NO:5. In an embodiment, the anti-CD20 monoclonal antibody has a variable light chain sequence identity of greater than 98% to SEQ ID NO:6. In an embodiment, the anti-CD20 monoclonal antibody has a variable heavy chain sequence identity of greater than 99% to SEQ ID NO:5. In an embodiment, the anti-CD20 monoclonal antibody has a variable light chain sequence identity of greater than 99% to SEQ ID NO:6. In an embodiment, the anti-CD20 monoclonal antibody has a Fab fragment heavy chain sequence identity of greater than 90% to SEQ ID NO:7. In an embodiment, the anti-CD20 monoclonal antibody has a Fab fragment light chain sequence identity of greater than 90% to SEQ ID NO:8. In an embodiment, the anti-CD20 monoclonal antibody has a Fab fragment heavy chain sequence identity of greater than 95% to SEQ ID NO:7. In an embodiment, the anti-CD20 monoclonal antibody has a Fab fragment light chain sequence identity of greater than 95% to SEQ ID NO:8. In an embodiment, the anti-CD20 monoclonal antibody has a Fab fragment heavy chain sequence identity of greater than 98% to SEQ ID NO:7. In an embodiment, the anti-CD20 monoclonal antibody has a Fab fragment light chain sequence identity of greater than 98% to SEQ ID NO:8. In an embodiment, the anti-CD20 monoclonal antibody has a Fab fragment heavy chain sequence identity of greater than 99% to SEQ ID NO:7. In an embodiment, the anti-CD20 monoclonal antibody has a Fab fragment light chain sequence identity of greater than 99% to SEQ ID NO:8. In an embodiment, the anti-CD20 monoclonal antibody ofatumumab is an immunoglobulin G1, anti-(human B-lymphocyte antigen CD20 (membrane-spanning 4-domains subfamily A member 1, B-lymphocyte surface antigen B1, Leu-16 or Bp35)); human monoclonal ofatumumab-CD20 $\gamma$1 heavy chain (225-214')-disulfide with human monoclonal ofatumumab-CD20 $\kappa$ light chain, dimer (231-231":234-234")-bisdisulfide antibody.

In an embodiment, the anti-CD20 monoclonal antibody is veltuzumab, or an antigen-binding fragment, derivative, conjugate, variant, or radioisotope-labeled complex thereof. Veltuzumab is also known as hA20. Veltuzumab is described in Goldenberg, et al., Leuk. Lymphoma 2010, 51, 747-55. In an embodiment, the anti-CD20 monoclonal antibody is an anti-CD20 biosimilar monoclonal antibody approved by drug regulatory authorities with reference to veltuzumab. In an embodiment, the anti-CD20 monoclonal antibody has a heavy chain sequence identity of greater than 90% to SEQ ID NO:9. In an embodiment, the anti-CD20 monoclonal antibody has a light chain sequence identity of greater than 90% to SEQ ID NO:10. In an embodiment, the anti-CD20 monoclonal antibody has a heavy chain sequence identity of greater than 95% to SEQ ID NO:9. In an embodiment, the anti-CD20 monoclonal antibody has a light chain sequence identity of greater than 95% to SEQ ID NO:10. In an embodiment, the anti-CD20 monoclonal antibody has a heavy chain sequence identity of greater than 98% to SEQ ID NO:9. In an embodiment, the anti-CD20 monoclonal antibody has a light chain sequence identity of greater than 98% to SEQ ID NO:10. In an embodiment, the anti-CD20 monoclonal antibody has a heavy chain sequence identity of greater than 99% to SEQ ID NO:9. In an embodiment, the anti-CD20 monoclonal antibody has a light chain sequence identity of greater than 99% to SEQ ID NO:10. In an embodiment, the anti-CD20 monoclonal antibody ofatumumab is an immunoglobulin G1, anti-(human B-lymphocyte antigen CD20 (membrane-spanning 4-domains subfamily A member 1, Leu-16, Bp35)); [218- arginine,360-glutamic acid,362-methionine]humanized mouse monoclonal hA20 $\gamma$1 heavy chain (224-213')-disulfide with humanized mouse monoclonal hA20 $\kappa$ light chain (230-230":233-233")-bisdisulfide dimer

In an embodiment, the anti-CD20 monoclonal antibody is tositumomab, or an antigen-binding fragment, derivative, conjugate, variant, or radioisotope-labeled complex thereof. In an embodiment, the anti-CD20 monoclonal antibody is $^{131}$I-labeled tositumomab. In an embodiment, the anti-CD20 monoclonal antibody is an anti-CD20 biosimilar monoclonal antibody approved by drug regulatory authorities with reference to tositumomab. In an embodiment, the anti-CD20 monoclonal antibody has a heavy chain sequence identity of greater than 90% to SEQ ID NO:11. In an embodiment, the anti-CD20 monoclonal antibody has a light chain sequence identity of greater than 90% to SEQ ID NO:12. In an embodiment, the anti-CD20 monoclonal antibody has a heavy chain sequence identity of greater than 95% to SEQ ID NO:11. In an embodiment, the anti-CD20 monoclonal antibody has a light chain sequence identity of greater than 95% to SEQ ID NO:12. In an embodiment, the anti-CD20 monoclonal antibody has a heavy chain sequence identity of greater than 98% to SEQ ID NO:11. In an embodiment, the anti-CD20 monoclonal antibody has a light chain sequence identity of greater than 98% to SEQ ID NO:12. In an embodiment, the anti-CD20 monoclonal antibody has a heavy chain sequence identity of greater than 99% to SEQ ID NO:11. In an embodiment, the anti-CD20 monoclonal antibody has a light chain sequence identity of greater than 99% to SEQ ID NO:12.

In an embodiment, the anti-CD20 monoclonal antibody is ibritumomab, or an antigen-binding fragment, derivative, conjugate, variant, or radioisotope-labeled complex thereof. The active form of ibritumomab used in therapy is

ibritumomab tiuxetan. When used with ibritumomab, the chelator tiuxetan (diethylene triamine pentaacetic acid) is complexed with a radioactive isotope such as $^{90}Y$ or $^{111}In$. In an embodiment, the anti-CD20 monoclonal antibody is ibritumomab tiuxetan, or radioisotope-labeled complex thereof. In an embodiment, the anti-CD20 monoclonal antibody is an anti-CD20 biosimilar monoclonal antibody approved by drug regulatory authorities with reference to tositumomab. In an embodiment, the anti-CD20 monoclonal antibody has a heavy chain sequence identity of greater than 90% to SEQ ID NO:13. In an embodiment, the anti-CD20 monoclonal antibody has a light chain sequence identity of greater than 90% to SEQ ID NO:14. In an embodiment, the anti-CD20 monoclonal antibody has a heavy chain sequence identity of greater than 95% to SEQ ID NO:13. In an embodiment, the anti-CD20 monoclonal antibody has a light chain sequence identity of greater than 95% to SEQ ID NO:14. In an embodiment, the anti-CD20 monoclonal antibody has a heavy chain sequence identity of greater than 98% to SEQ ID NO:13. In an embodiment, the anti-CD20 monoclonal antibody has a light chain sequence identity of greater than 98% to SEQ ID NO:14. In an embodiment, the anti-CD20 monoclonal antibody has a heavy chain sequence identity of greater than 99% to SEQ ID NO:13. In an embodiment, the anti-CD20 monoclonal antibody has a light chain sequence identity of greater than 99% to SEQ ID NO:14.

In an embodiment, an anti-CD20 antibody selected from the group consisting of obinutuzumab, ofatumumab, veltuzumab, tositumomab, and ibritumomab, and or antigen-binding fragments, derivatives, conjugates, variants, and radioisotope-labeled complexes thereof, is administered to a subject by infusing a dose selected from the group consisting of about 10 mg, about 20 mg, about 25 mg, about 50 mg, about 75 mg, 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, and about 2000 mg. In an embodiment, the anti-CD20 antibody is admininstered weekly. In an embodiment, the anti-CD20 antibody is admininstered every two weeks. In an embodiment, the anti-CD20 antibody is admininstered every three weeks. In an embodiment, the anti-CD20 antibody is admininstered monthly. In an embodiment, the anti-CD20 antibody is administered at a lower initial dose, which is escalated when administered at subsequent intervals admininstered monthly. For example, the first infusion can deliver 300 mg of anti-CD20 antibody, and subsequent weekly doses could deliver 2,000 mg of anti-CD20 antibody for eight weeks, followed by monthly doses of 2,000 mg of anti-CD20 antibody. During any of the foregoing embodiments, the the BTK inhibitors of formula (2) with methotrexate may be administered daily, twice daily, or at different intervals as described above, at the dosages described above.

In an embodiment, the anti-CD20 monoclonal antibody is an anti-CD20 biosimilar monoclonal antibody approved by drug regulatory authorities with reference to rituximab, obinutuzumab, ofatumumab, veltuzumab, tositumomab, or ibritumomab. In an embodiment, the biosimilar comprises an anti-CD20 antibody comprising an amino acid sequence which has at least 97% sequence identity, e.g., 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is rituximab, obinutuzumab, ofatumumab, veltuzumab, tositumomab, or ibritumomab. In some embodiments, the one or more post-translational modifications are selected from one or more of: glycosylation, oxidation, deamidation, and truncation. In some embodiments, the biosimilar is an anti-CD20 antibody authorized or submitted for authorization, wherein the anti-CD20 antibody is provided in a formulation which differs from the formulations of a reference medicinal product or reference biological product, wherein the reference medicinal product or reference biological product is rituximab, obinutuzumab, ofatumumab, veltuzumab, tositumomab, or ibritumomab. The anti-CD20 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is rituximab, obinutuzumab, ofatumumab, veltuzumab, tositumomab, or ibritumomab. In some embodiments, the biosimilar comprises one or more excipients selected from tris-hydrochloride, sodium chloride, mannitol, pentetic acid, polysorbate 80, sodium hydroxide, and hydrochloric acid.

The anti-CD20 antibody sequences referenced in the foregoing are summarized in Table 1.

TABLE 1. Anti-CD20 antibody sequences.

| Identifier | Sequence (One-Letter Amino Acid Symbols) |
|---|---|
| SEQ ID NO:1 rituximab heavy chain | QVQLQQPGAE LVKPGASVKM SCKASGYTFT SYNMHWVKQT PGRGLEWIGA IYPGNGDTSY 60<br>NQKFKGKATL TADKSSSTAY MQLSSLTSED SAVYYCARST YYGGDWYFNV WGAGTTVTVS 120<br>AASTKGPSVF PLAPSSKSTS GGTAALGCLV KDYFPEPVTV SWNSGALTSG VHTFPAVLQS 180<br>SGLYSLSSVV TVPSSSLGTQ TYICNVNHKP SNTKVDKKVE PKSCDKTHTC PPCPAPELLG 240<br>GPSVFLFPPK PKDTLMISRT PEVTCVVVDV SHEDPEVKFN WYVDGVEVHN AKTKPREEQY 300<br>NSTYRVVSVL TVLHQDWLNG KEYKCKVSNK ALPAPIEKTI SKAKGQPREP QVYTLPPSRD 360<br>ELTKNQVSLT CLVKGFYPSD IAVEWESNGQ PENNYKTTPP VLDSDGSFFL YSKLTVDKSR 420<br>WQQGNVFSCS VMHEALHNHY TQKSLSLSPG K 451 |

(continued)

| Identifier | Sequence (One-Letter Amino Acid Symbols) |
|---|---|
| SEQ ID NO:2 rituximab light chain | QIVLSQSPAI LSASPGEKVT MTCRASSSVS YIHWFQQKPG SSPKPWIYAT SNLASGVPVR 60<br>FSGSGSGTSY SLTISRVEAE DAATYYCQQW TSNPPTFGGG TKLEIKRTVA APSVFIFPPS 120<br>DEQLKSGTAS VVCLLNNFYP REAKVQWKVD NALQSGNSQE SVTEQDSKDS TYSLSSTLTL 180<br>SKADYEKHKV YACEVTHQGL SSPVTKSFNR GEC 213 |
| SEQ ID NO:3 obinutuzumab heavy chain | QVQLVQSGAE VKKPGSSVKV SCKASGYAFS YSWINWVRQA PGQGLEWMGR IFPGDGDTDY 60<br>NGKFKGRVTI TADKSTSTAY MELSSLRSED TAVYYCARNV FDGYWLVYWG QGTLVTVSSA 120<br>STKGPSVFPL APSSKSTSGG TAALGCLVKD YFPEPVTVSW NSGALTSGVH TFPAVLQSSG 180<br>LYSLSSVVTV PSSSLGTQTY ICNVNHKPSN TKVDKKVEPK SCDKTHTCPP CPAPELLGGP 240<br>SVFLFPPKPK DTLMISRTPE VTCVVVDVSH EDPEVKFNWY VDGVEVHNAK TKPREEQYNS 300<br>TYRVVSVLTV LHQDWLNGKE YKCKVSNKAL PAPIEKTISK AKGQPREPQV YTLPPSRDEL 360<br>TKNQVSLTCL VKGFYPSDIA VEWESNGQPE NNYKTTPPVL DSDGSFFLYS KLTVDKSRWQ 420<br>QGNVFSCSVM HEALHNHYTQ KSLSLSPGK 449 |
| SEQ ID NO:4 obinutuzumab light chain | DIVMTQTPLS LPVTPGEPAS ISCRSSKSLL HSNGITYLYW YLQKPGQSPQ LLIYQMSNLV 60<br>SGVPDRFSGS GSGTDFTLKI SRVEAEDVGV YYCAQNLELP YTFGGGTKVE IKRTVAAPSV 120<br>FIFPPSDEQL KSGTASVVCL LNNFYPREAK VQWKVDNALQ SGNSQESVTE QDSKDSTYSL 180<br>SSTLTLSKAD YEKHKVYACE VTHQGLSSPV TKSFNRGEC 219 |
| SEQ ID NO:5 ofatumumab variable heavy chain | EVQLVESGGG LVQPGRSLRL SCAASGFTFN DYAMHWVRQA PGKGLEWVST ISWNSGSIGY 60<br>ADSVKGRFTI SRDNAKKSLY LQMNSLRAED TALYYCAKDI QYGNYYYGMD VWGQGTTVTV 120<br>SS 122 |
| SEQ ID NO:6 ofatumumab variable light chain | EIVLTQSPAT LSLSPGERAT LSCRASQSVS SYLAWYQQKP GQAPRLLIYD ASNRATGIPA 60<br>RFSGSGSGTD FTLTISSLEP EDFAVYYCQQ RSNWPITFGQ GTRLEIK 107 |
| SEQ ID NO:7 ofatumumab Fab fragment heavy chain | EVQLVESGGG LVQPGRSLRL SCAASGFTFN DYAMHWVRQA PGKGLEWVST ISWNSGSIGY 60<br>ADSVKGRFTI SRDNAKKSLY LQMNSLRAED TALYYCAKDI QYGNYYYGMD VWGQGTTVTV 120<br>SSASTKGPSV FPLAPGSSKS TSGTAALGCL VKDYFPEPVT VSWNSGALTS GVHTFPAVLQ 180<br>SSGLYSLSSV VTVPSSSLGT QTYICNVNHK PSNTKVDKKV EP 222 |
| SEQ ID NO:8 ofatumumab Fab fragment light chain | EIVLTQSPAT LSLSPGERAT LSCRASQSVS SYLAWYQQKP GQAPRLLIYD ASNRATGIPA 60<br>RFSGSGSGTD FTLTISSLEP EDFAVYYCQQ RSNWPITFGQ GTRLEIKRTV AAPSVFIFPP 120<br>SDEQLKSGTA SVVCLLNNFY PREAKVQWKV DNALQSGNSQ ESVTEQDSKD STYSLSSTLT 180<br>LSKADYEKHK VYACEVTHQG LSSPVTKSFN R 211 |
| SEQ ID NO:9 veltuzumab heavy chain | QVQLQQSGAE VKKPGSSVKV SCKASGYTFT SYNMHWVKQA PGQGLEWIGA IYPGMGDTSY 60<br>NQKFKGKATL TADESTNTAY MELSSLRSED TAFYYCARST YYGGDWYFDV WGQGTTVTVS 120<br>SASTKGPSVF PLAPSSKSTS GGTAALGCLV KDYFPEPVTV SWNSGALTSG VHTFPAVLQS 180<br>SGLYSLSSVV TVPSSSLGTQ TYICNVNHKP SNTKVDKRVE PKSCDKTHTC PPCPAPELLG 240<br>GPSVFLFPPK PKDTLMISRT PEVTCVVVDV SHEDPEVKFN WYVDGVEVHN AKTKPREEQY 300<br>NSTYRVVSVL TVLHQDWLNG KEYKCKVSNK ALPAPIEKTI SKAKGQPREP QVYTLPPSRE 360<br>EMTKNQVSLT CLVKGFYPSD IAVEWESNGQ PENNYKTTPP VLDSDGSFFL YSKLTVDKSR 420<br>WQQGNVFSCS VMHEALHNHY TQKSLSLSPG K 451 |
| SEQ ID NO:10 veltuzumab light chain | DIQLTQSPSS LSASVGDRVT MTCRASSSVS YIHWFQQKPG KAPKPWIYAT SNLASGVPVR 60<br>FSGSGSGTDY TFTISSLQPE DIATYYCQQW TSNPPTFGGG TKLEIKRTVA APSVFIFPPS 120<br>DEQLKSGTAS VVCLLNNFYP REAKVQWKVD NALQSGNSQE SVTEQDSKDS TYSLSSTLTL 180<br>SKADYEKHKV YACEVTHQGL SSPVTKSFNR GEC 213 |
| SEQ ID NO:11 tositumomab heavy chain | QAYLQQSGAE LVRPGASVKM SCKASGYTFT SYNMHWVKQT PRQGLEWIGA IYPGNGDTSY 60<br>NQKFKGKATL TVDKSSSTAY MQLSSLTSED SAVYFCARVV YYSNSYWYFD VWGTGTTVTV 120<br>SGPSVFPLAP SSKSTSGGTA ALGCLVKDYF PEPVTVSWNS GALTSGVHTF PAVLQSSGLY 180<br>SLSSVVTVPS SSLGTQTYIC NVNHKPSNTK VDKKAEPKSC DKTHTCPPCP APELLGGPSV 240<br>FLFPPKPKDT LMISRTPEVT CVVVDVSHED PEVKFNWYVD GVEVHNAKTK PREEQYNSTY 300<br>RVVSVLTVLH QDWLNGKEYK CKVSNKALPA PIEKTISKAK GQPREPQVYT LPPSRDELTK 360<br>NQVSLTCLVK GFYPSDIAVE WESNGQPENN YKTTPPVLDS DGSFFLYSKL TVDKSRWQQG 420<br>NVFSCSVMHE ALHNHYTQKS LSLSPGK 447 |
| SEQ ID NO:12 tositumomab light chain | QIVLSQSPAI LSASPGEKVT MTCRASSSVS YMHWYQQKPG SSPKPWIYAP SNLASGVPAR 60<br>FSGSGSGTSY SLTISRVEAE DAATYYCQQW SFNPPTFGAG TKLELKRTVA APSVFIFPPS 120<br>DEQLKSGTAS VVCLLNNFYP REAKVQWKVD NALQSGNSQE SVTEQDSKDS TYSLSSTLTL 180<br>SKADYEKHKV YACEVTHQGL SSPVTKSFNR 210 |

(continued)

| Identifier | Sequence (One-Letter Amino Acid Symbols) |
|---|---|
| SEQ ID NO:13 ibritumomab heavy chain | QAYLQQSGAE LVRPGASVKM SCKASGYTFT SYNMHWVKQT PRQGLEWIGA IYPGNGDTSY 60<br>NQKFKGKATL TVDKSSSTAY MQLSSLTSED SAVYFCARVV YYSNSYWYFD VWGTGTTVTV 120<br>SAPSVYPLAP VCGDTTGSSV TLGCLVKGYF PEPVTLTWNS GSLSSGVHTF PAVLQSDLYT 180<br>LSSSVTVTSS TWPSQSITCN VAHPASSTKV DKKIEPRGPT IKPCPPCKCP APNLLGGPSV 240<br>FIFPPKIKDV LMISLSPIVT CVVVDVSEDD PDVQISWFVN NVEVHTAQTQ THREDYNSTL 300<br>RVVSALPIQH QDWMSGKEFK CKVNNKDLPA PIERTISKPK GSVRAPQVYV LPPPEEEMTK 360<br>KQVTLTCMVT DFMPEDIYVE WTNNGKTELN YKNTEPVLDS DGSYFMYSKL RVEKKNWVER 420<br>NSYSCSVVHE GLHNHHTTKS FSR 443 |
| SEQ ID NO:14 ibritumomab light chain | QIVLSQSPAI LSASPGEKVT MTCRASSSVS YMHWYQQKPG SSPKPWIYAP SNLASGVPAR 60<br>FSGSGSGTSY SLTISRVEAE DAATYYCQQW SFNPPTFGAG TKLELKRADA APTVFIFPPS 120<br>DEQLKSGTAS VVCLLNNFYP REAKVQWKVD NALQSGNSQE SVTEQDSKDS TYSLSSTLTL 180<br>SKADYEKHKV YACEVTHQGL SSPVTKSFN 209 |

Combinations of BTK Inhibitors, Antifolate Compounds, and PD-1 and PD-L1 Inhibitors

The combinations of the BTK inhibitor of formula (2) with methotrexate may also be further combined with programmed death-1 (PD-1), programmed death ligand 1 (PD-L1), and/or programmed death ligand 2 (PD-L2) binding antibodies or inhibitors (*i.e.*, blockers). In a preferred embodiment, the PD-1 or PD-L1 inhibitor for use in combination with methotrexate and a BTK inhibitor of formula (2) is selected from the group consisting of nivolumab, pembrolizumab, pidilizumab, durvalumab, atezolizumab, avelumab, and antigen-binding fragments, variants, conjugates, or biosimilars thereof. In a preferred embodiment, the invention provides a combination or compositions for use in the treatment of rheumatoid arthritis in a human comprising the step of administering to said human a BTK inhibitor of formula (2), or a pharmaceutically acceptable salt thereof, and methotrexate or a pharmaceutically acceptable salt thereof, and further comprising the step of administering an PD-1 or PD-L1 inhibitor, or an antigen-binding fragment, derivative, conjugate, variant, or biosimilar thereof. In an embodiment, the BTK inhibitor is a compound of formula (2), or pharmaceutically acceptable salts thereof.

Programmed death 1 (PD-1) is a 288-amino acid transmembrane immunocheckpoint receptor protein expressed by T cells, B cells, natural killer (NK) T cells, activated monocytes, and dendritic cells. PD-1, which is also known as CD279, is an immunoreceptor belonging to the CD28 family and in humans is encoded by the *Pdcd1* gene on chromosome 2. PD-1 consists of one immunoglobulin (Ig) superfamily domain, a transmembrane region, and an intracellular domain containing an immunoreceptor tyrosine-based inhibitory motif (ITIM) and an immunoreceptor tyrosine-based switch motif (ITSM). PD-1 and its ligands (PD-L1 and PD-L2) play a key role in immune tolerance, as described in Keir, et al., Annu. Rev. Immunol. 2008, 26, 677-704. PD-1 provides inhibitory signals that negatively regulate T cell immune responses. PD-L1 (also known as B7-H1 or CD274) and PD-L2 (also known as B7-DC or CD273) are expressed on tumor cells and stromal cells, which may be encountered by activated T cells expressing PD-1, leading to immunosuppression of the T cells. PD-L1 is a 290 amino acid transmembrane protein encoded by the *Cd274* gene on human chromosome 9. Blocking the interaction between PD-1 and its ligands PD-L1 and PD-L2 by use of a PD-1 inhibitor, a PD-L1 inhibitor, and/or a PD-L2 inhibitor can overcome immune resistance, as demonstrated in recent clinical studies, such as that described in Topalian, et al., N. Eng. J. Med. 2012, 366, 2443-54. PD-L1 is expressed on many tumor cell lines, while PD-L2 is expressed is expressed mostly on dendritic cells and a few tumor lines. In addition to T cells (which inducibly express PD-1 after activation), PD-1 is also expressed on B cells, natural killer cells, macrophages, activated monocytes, and dendritic cells.

In an embodiment, the PD-1 inhibitor may be any PD-1 inhibitor or PD-1 blocker known in the art. In particular, it is one of the PD-1 inhibitors or blockers described in more detail in the following paragraphs. The terms "inhibitor" and "blocker" are used interchangeably herein in reference to PD-1 inhibitors. For avoidance of doubt, references herein to a PD-1 inhibitor that is an antibody may refer to a compound or antigen-binding fragments, variants, conjugates, or biosimilars thereof. For avoidance of doubt, references herein to a PD-1 inhibitor may also refer to a compound or a pharmaceutically acceptable salt thereof.

In some embodiments, the compositions described herein include a PD-1 inhibitor. In some embodiments, the PD-1 inhibitor is a small molecule. In a preferred embodiment, the PD-1 inhibitor is an antibody (*i.e.*, an anti-PD-1 antibody), a fragment thereof, including Fab fragments, or a single-chain variable fragment (scFv) thereof. In some embodiments the PD-1 inhibitor is a polyclonal antibody. In a preferred embodiment, the PD-1 inhibitor is a monoclonal antibody. In some embodiments, the PD-1 inhibitor competes for binding with PD-1, and/or binds to an epitope on PD-1. In an embodiment, the antibody competes for binding with PD-1, and/or binds to an epitope on PD-1. In some embodiments, an anti-PD-1 monoclonal antibody is included in a composition and is further combined with a BTK inhibitor of formula (2) and methotrexateIn some embodiments, the PD-1 inhibitors provided herein are selective for PD-1, in that

the compounds bind or interact with PD-1 at substantially lower concentrations than they bind or interact with other receptors.

In some embodiments, the compositions described include a PD-1 inhibitor that binds human PD-1 with a $K_D$ of about 100 pM or lower, binds human PD-1 with a $K_D$ of about 90 pM or lower, binds human PD-1 with a $K_D$ of about 80 pM or lower, binds human PD-1 with a $K_D$ of about 70 pM or lower, binds human PD-1 with a $K_D$ of about 60 pM or lower, binds human PD-1 with a $K_D$ of about 50 pM or lower, binds human PD-1 with a $K_D$ of about 40 pM or lower, binds human PD-1 with a $K_D$ of about 30 pM or lower, binds human PD-1 with a $K_D$ of about 20 pM or lower, binds human PD-1 with a $K_D$ of about 10 pM or lower, or binds human PD-1 with a $K_D$ of about 1 pM or lower.

In some embodiments, the compositions described include a PD-1 inhibitor that binds to human PD-1 with a $k_{assoc}$ of about $7.5 \times 10^5$ l/M·s or faster, binds to human PD-1 with a $k_{assoc}$ of about $7.5 \times 10^5$ l/M·s or faster, binds to human PD-1 with a $k_{assoc}$ of about $8 \times 10^5$ l/M·s or faster, binds to human PD-1 with a $k_{assoc}$ of about $8.5 \times 10^5$ l/M·s or faster, binds to human PD-1 with a $k_{assoc}$ of about $9 \times 10^5$ l/M·s or faster, binds to human PD-1 with a $k_{assoc}$ of about $9.5 \times 10^5$ l/M·s or faster, or binds to human PD-1 with a $k_{assoc}$ of about $1 \times 10^6$ l/M·s or faster.

In some embodiments, the compositions described include a PD-1 inhibitor that binds to human PD-1 with a $k_{dissoc}$ of about $2 \times 10^{-5}$ 1/s or slower, binds to human PD-1 with a $k_{dissoc}$ of about $2.1 \times 10^{-5}$ 1/s or slower , binds to human PD-1 with a $k_{dissoc}$ of about $2.2 \times 10^{-5}$ 1/s or slower, binds to human PD-1 with a $k_{dissoc}$ of about $2.3 \times 10^{-5}$ 1/s or slower, binds to human PD-1 with a $k_{dissoc}$ of about $2.4 \times 10^{-5}$ 1/s or slower, binds to human PD-1 with a $k_{dissoc}$ of about $2.5 \times 10^{-5}$ 1/s or slower, binds to human PD-1 with a $k_{dissoc}$ of about $2.6 \times 10^{-5}$ 1/s or slower or binds to human PD-1 with a $k_{dissoc}$ of about $2.7 \times 10^{-5}$ 1/s or slower, binds to human PD-1 with a $k_{dissoc}$ of about $2.8 \times 10^{-5}$ 1/s or slower, binds to human PD-1 with a $k_{dissoc}$ of about $2.9 \times 10^{-5}$ 1/s or slower, or binds to human PD-1 with a $k_{dissoc}$ of about $3 \times 10^{-5}$ 1/s or slower.

In some embodiments, the compositions described include a PD-1 inhibitor that blocks or inhibits binding of human PD-L1 or human PD-L2 to human PD-1 with an $IC_{50}$ of about 10 nM or lower, blocks or inhibits binding of human PD-L1 or human PD-L2 to human PD-1 with an $IC_{50}$ of about 9 nM or lower, blocks or inhibits binding of human PD-L1 or human PD-L2 to human PD-1 with an $IC_{50}$ of about 8 nM or lower, blocks or inhibits binding of human PD-L1 or human PD-L2 to human PD-1 with an $IC_{50}$ of about 7 nM or lower, blocks or inhibits binding of human PD-L1 or human PD-L2 to human PD-1 with an $IC_{50}$ of about 6 nM or lower, blocks or inhibits binding of human PD-L1 or human PD-L2 to human PD-1 with an $IC_{50}$ of about 5 nM or lower, blocks or inhibits binding of human PD-L1 or human PD-L2 to human PD-1 with an $IC_{50}$ of about 4 nM or lower, blocks or inhibits binding of human PD-L1 or human PD-L2 to human PD-1 with an $IC_{50}$ of about 3 nM or lower, blocks or inhibits binding of human PD-L1 or human PD-L2 to human PD-1 with an $IC_{50}$ of about 2 nM or lower, or blocks or inhibits binding of human PD-L1 or human PD-L2 to human PD-1 with an $IC_{50}$ of about 1 nM or lower.

In an embodiment, an anti-PD-1 antibody comprises nivolumab (also known as OPDIVO and commercially available from Bristol-Myers Squibb Co.), or biosimilars, antigen-binding fragments, conjugates, or variants thereof. Nivolumab is referred to as 5C4 in International Patent Publication No. WO 2006/121168. Nivolumab is assigned Chemical Abstracts Service (CAS) registry number 946414-94-4 and is also known as BMS-936558, MDX-1106 or ONO-4538. Nivolumab is a fully human IgG4 antibody blocking the PD-1 receptor. The clinical safety and efficacy of nivolumab in various forms of cancer has been described in Wang et al., Cancer Immunol Res. 2014, 2, 846-56; Page et al., Ann. Rev. Med., 2014, 65, 185-202; and Weber, et al., J. Clin. Oncology, 2013, 31, 4311-4318. The nivolumab monoclonal antibody includes a heavy chain given by SEQ ID NO:15 and a light chain given by SEQ ID NO:16. Nivolumab has intra-heavy chain disulfide linkages at 22-96,140-196, 254-314, 360-418, 22"-96", 140"-196", 254"-314", and 360"-418"; intra-light chain disulfide linkages at 23'-88', 134'-194', 23"'-88"', and 134'"-194"'; inter-heavy-light chain disulfide linkages at 127-214', 127"-214"', inter-heavy-heavy chain disulfide linkages at 219-219" and 222-222"; and N-glycosylation sites (H $CH_2$ 84.4) at 290, 290". In an embodiment, the anti-PD-1 antibody is an immunoglobulin G4 kappa, anti-(human CD274) antibody. In an embodiment, an anti-PD-1 antibody comprises heavy and light chains having the sequences shown in SEQ ID NO:15 and SEQ ID NO:16, respectively, or antigen binding fragments, Fab fragments, single-chain variable fragments (scFv), variants, or conjugates thereof. In an embodiment, an anti-PD-1 antibody comprises heavy and light chains that are each at least 99% identical to the sequences shown in SEQ ID NO:15 and SEQ ID NO:16, respectively. In an embodiment, an anti-PD-1 antibody comprises heavy and light chains that are each at least 98% identical to the sequences shown in SEQ ID NO:15 and SEQ ID NO:16, respectively. In an embodiment, an anti-PD-1 antibody comprises heavy and light chains that are each at least 97% identical to the sequences shown in SEQ ID NO:15 and SEQ ID NO:16, respectively. In an embodiment, an anti-PD-1 antibody comprises heavy and light chains that are each at least 96% identical to the sequences shown in SEQ ID NO:15 and SEQ ID NO:16, respectively. In an embodiment, an anti-PD-1 antibody comprises heavy and light chains that are each at least 95% identical to the sequences shown in SEQ ID NO:15 and SEQ ID NO:16, respectively.

In an embodiment, the anti-PD-1 antibody is an anti-PD-1 biosimilar monoclonal antibody approved by drug regulatory authorities with reference to nivolumab. In an embodiment, the biosimilar comprises an anti-PD-1 antibody comprising an amino acid sequence which has at least 97% sequence identity, *e.g.*, 97%, 98%, 99% or 100% sequence

identity, to the amino acid sequence of a reference medicinal product or reference biological product and which comprises one or more post-translational modifications as compared to the reference medicinal product or reference biological product, wherein the reference medicinal product or reference biological product is nivolumab. In some embodiments, the one or more post-translational modifications are selected from one or more of: glycosylation, oxidation, deamidation, and truncation. In some embodiments, the biosimilar is an anti-PD-1 antibody authorized or submitted for authorization, wherein the anti-PD-1 antibody is provided in a formulation which differs from the formulations of a reference medicinal product or reference biological product, wherein the reference medicinal product or reference biological product is nivolumab. The anti-PD-1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is nivolumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is nivolumab.

In an embodiment, the anti-PD-1 antibody comprises the heavy and light chain CDRs or variable regions (VRs) of nivolumab. In one embodiment, the anti-PD-1 antibody heavy chain variable region ($V_H$) comprises the sequence shown in SEQ ID NO: 17, and the anti-PD-1 antibody light chain variable region ($V_L$) comprises the sequence shown in SEQ ID NO: 18. In an embodiment, an anti-PD-1 antibody comprises $V_H$ and $V_L$ regions that are each at least 99% identical to the sequences shown in SEQ ID NO:17 and SEQ ID NO: 18, respectively. In an embodiment, an anti-PD-1 antibody comprises $V_H$ and $V_L$ regions that are each at least 98% identical to the sequences shown in SEQ ID NO:17 and SEQ ID NO: 18, respectively. In an embodiment, an anti-PD-1 antibody comprises $V_H$ and $V_L$ regions that are each at least 97% identical to the sequences shown in SEQ ID NO:17 and SEQ ID NO: 18, respectively. In an embodiment, an anti-PD-1 antibody comprises $V_H$ and $V_L$ regions that are each at least 96% identical to the sequences shown in SEQ ID NO:17 and SEQ ID NO: 18, respectively. In an embodiment, an anti-PD-1 antibody comprises $V_H$ and $V_L$ regions that are each at least 95% identical to the sequences shown in SEQ ID NO:17 and SEQ ID NO: 18, respectively. In an alternative embodiment, the antibody comprises $V_H$ and/or $V_L$ regions having the amino acid sequences set forth in SEQ ID NO: 17 and/or SEQ ID NO: 18, respectively.

In an embodiment, the anti-PD-1 antibody comprises heavy chain CDR1, CDR2 and CDR3 domains having the sequences set forth in SEQ ID NO: 19, SEQ ID NO:20, and SEQ ID NO:21, respectively, and light chain CDR1, CDR2 and CDR3 domains having the sequences set forth in SEQ ID NO:22, SEQ ID NO:23, and SEQ ID NO:24, respectively.

In an embodiment, an anti-PD-1 antibody comprises a heavy chain that comprises CDR1, CDR2 and CDR3 domains that are each at least 95% identical to the sequences shown in SEQ ID NO: 19, SEQ ID NO:20, and SEQ ID NO:21, respectively. In an embodiment, an anti-PD-1 antibody comprises a heavy chain that comprises CDR1, CDR2 and CDR3 domains that are each at least 90% identical to the sequences shown in SEQ ID NO:19, SEQ ID NO:20, and SEQ ID NO:21, respectively. In an embodiment, an anti-PD-1 antibody comprises a heavy chain that comprises CDR1, CDR2 and CDR3 domains that are each at least 85% identical to the sequences shown in SEQ ID NO:19, SEQ ID NO:20, and SEQ ID NO:21, respectively. In an embodiment, an anti-PD-1 antibody comprises a heavy chain that comprises CDR1, CDR2 and CDR3 domains that are each at least 80% identical to the sequences shown in SEQ ID NO:19, SEQ ID NO:20, and SEQ ID NO:21, respectively. In another embodiment, the antibody competes for binding with, and/or binds to the same epitope on PD-1 as the aforementioned antibodies.

In an embodiment, an anti-PD-1 antibody comprises a light chain that comprises CDR1, CDR2 and CDR3 domains that are each at least 95% identical to the sequences shown in SEQ ID NO:22, SEQ ID NO:23, and SEQ ID NO:24, respectively. In an embodiment, an anti-PD-1 antibody comprises a light chain that comprises CDR1, CDR2 and CDR3 domains that are each at least 90% identical to the sequences shown in SEQ ID NO:22, SEQ ID NO:23, and SEQ ID NO:24, respectively. In an embodiment, an anti-PD-1 antibody comprises a light chain that comprises CDR1, CDR2 and CDR3 domains that are each at least 85% identical to the sequences shown in SEQ ID NO:22, SEQ ID NO:23, and SEQ ID NO:24, respectively. In an embodiment, an anti-PD-1 antibody comprises a light chain that comprises CDR1, CDR2 and CDR3 domains that are each at least 80% identical to the sequences shown in SEQ ID NO:22, SEQ ID NO:23, and SEQ ID NO:24, respectively. In another embodiment, the antibody competes for binding with, and/or binds to the same epitope on PD-1 as the aforementioned antibodies.

In an embodiment, the anti-PD-1 antibody is an antibody disclosed and/or prepared according to U.S. Patent No. 8,008,449 or U.S. Patent Application Publication Nos. 2009/0217401 A1 or 2013/0133091 A1. For example, in an embodiment, the monoclonal antibody includes 5C4 (referred to herein as nivolumab), 17D8, 2D3, 4H1, 4A11, 7D3, and 5F4, described in U.S. Patent No. 8,008,449. The PD-1 antibodies 17D8, 2D3, 4H1, 5C4, and 4A11, are all directed against human PD-1, bind specifically to PD-1 and do not bind to other members of the CD28 family. The sequences and CDR regions for these antibodies are provided in U.S. Patent No. 8,008,449, in particular in Figure 1 through Figure

12 therein.

The anti-PD-1 antibody nivolumab may be prepared by the following procedure, as described in U.S. Patent No. 8,008,449. Immunization protocols utilized as antigen both (i) a recombinant fusion protein comprising the extracellular portion of PD-1 and (ii) membrane bound full-length PD-1. Both antigens were generated by recombinant transfection methods in a CHO cell line. Fully human monoclonal antibodies to PD-1 were prepared using the HCo7 strain of HuMab transgenic mice and the KM strain of transgenic transchromosomic mice, each of which express human antibody genes. In each of these mouse strains, the endogenous mouse kappa light chain gene has been homozygously disrupted as described in Chen, et al. EMBO J. 1993, 12, 811-820 and the endogenous mouse heavy chain gene has been homozygously disrupted as described in Example 1 of International Patent Publication No. WO 01/09187. Each of these mouse strains carries a human kappa light chain transgene, KCo5, as described in Fishwild, et al. Nat. Biotechnology 1996, 14, 845-851. The HCo7 strain carries the HCo7 human heavy chain transgene as described in U.S. Patent Nos. 5,545,806; 5,625,825; and 5,545,807. The KM strain contains the SC20 transchromosome as described in International Patent Publication No. WO 02/43478. To generate fully human monoclonal antibodies to PD-1, HuMab mice and KM Mice™ were immunized with purified recombinant PD-1 fusion protein and PD-1-transfected CHO cells as antigen. General immunization schemes for HuMab mice are described in Lonberg, et al., Nature 1994, 368, 856-859; Fishwild, et al., Nat. Biotechnology 1996, 14, 845-851, and International Patent Publication No. WO 98/24884. The mice were 6-16 weeks of age upon the first infusion of antigen. A purified recombinant preparation (5-50 $\mu$g) of PD-1 fusion protein antigen and 5-10$\times 10^6$ cells were used to immunize the HuMab mice and KM Mice™ intraperitonealy, subcutaneously (Sc) or via footpad injection. Transgenic mice were immunized twice with antigen in complete Freund's adjuvant or Ribi adjuvant IP, followed by 3-21 days IP (up to a total of 11 immunizations) with the antigen in incomplete Freund's or Ribi adjuvant. The immune response was monitored by retroorbital bleeds. The plasma was screened by ELISA (as described below), and mice with sufficient titers of anti-PD-1 human immunoglobulin were used for fusions. Mice were boosted intravenously with antigen 3 days before sacrifice and removal of the spleen. Typically, 10-35 fusions for each antigen were performed. Several dozen mice were immunized for each antigen. To select HuMab or KM Mice™ producing antibodies that bound PD-1, sera from immunized mice were tested by ELISA as described by Fishwild, et al., Nat. Biotechnology 1996, 14, 845-851. Briefly, microtiter plates were coated with purified recombinant PD-1 fusion protein from transfected CHO cells at 1-2 $\mu$g/ml in PBS, 100 $\mu$L/wells incubated at 4° C overnight then blocked with 200 $\mu$L/well of 5% fetal bovine serum in PBS/Tween (0.05%). Dilutions of sera from PD-1-immunized mice were added to each well and incubated for 1-2 hours at ambient temperature. The plates were washed with PBS/Tween and then incubated with a goat-anti-human IgG polyclonal antibody conjugated with horseradish peroxidase (HRP) for 1 hour at room temperature. After washing, the plates were developed with ABTS substrate (Sigma, A-1888, 0.22 mg/ml) and analyzed by spectrophotometer at OD 415-495. Mice that developed the highest titers of anti-PD-1 antibodies were used for fusions. Fusions were performed as described below and hybridoma supernatants were tested for anti-PD-1 activity by ELISA. The mouse splenocytes, isolated from the HuMab or KM mice, were fused to a mouse myeloma cell line either using PEG based upon standard protocols or electric field based electrofusion using a Cyto Pulse large chamber cell fusion electroporator (Cyto Pulse Sciences, Inc., Glen Burnie, MD). The resulting hybridomas were then screened for the production of antigen-specific antibodies. Single cell suspensions of splenocytes from immunized mice were fused to one-fourth the number of SP2/0 nonsecreting mouse myeloma cells (ATCC, CRL 1581) with 50% PEG (Sigma). Cells were plated at approximately 1 $\times$ 105/well in flat bottom microtiter plate, followed by about two week incubation in selective medium containing 10% fetal bovine serum, 10% P388D1 (ATCC, CRL TIB-63) conditioned medium, 3-5% origen (IGEN) in DMEM (Mediatech, CRL 10013, with high glucose, L-glutamine and sodium pyruvate) plus 5 mM HEPES, 0.055 mM 2-mercaptoethanol, 50 mg/ml gentamycin and 1 $\times$HAT (Sigma, CRL P-7185). After 1-2 weeks, cells were cultured in medium in which the HAT was replaced with HT. Individual wells were then screened by ELISA (described above) for human anti-PD-1 monoclonal IgG antibodies. Once extensive hybridoma growth occurred, medium was monitored usually after 10-14 days. The antibody-secreting hybridomas were replated, screened again and, if still positive for human IgG, anti-PD-1 monoclonal antibodies were subcloned at least twice by limiting dilution. The stable subclones were then cultured in vitro to generate small amounts of antibody in tissue culture medium for further characterization. The antibody nivolumab may be produced in this manner, or by other known means given the disclosure of the amino acid sequences herein.

In another embodiment, the anti-PD-1 antibody comprises pembrolizumab (also known as KEYTRUDA), which is commercially available from Merck, or antigen-binding fragments, conjugates, or variants thereof. Pembrolizumab is assigned CAS registry number 1374853-91-4 and is also known as lambrolizumab, MK-3475, and SCH-900475. The structure, properties, uses, and preparation of pembrolizumab are described in International Patent Publication No. WO 2008/156712 A1, U.S. Patent No. 8,354,509 and U.S. Patent Application Publication Nos. US 2010/0266617 A1, US 2013/0108651 A1, and US 2013/0109843 A2. Pembrolizumab has an immunoglobulin G4, anti-(human protein PDCD1 (programmed cell death 1)) (human-Mus musculus monoclonal heavy chain), disulfide with human-Mus musculus monoclonal light chain, dimer structure. The structure of pembrolizumab may also be described as immunoglobulin G4, anti-(human programmed cell death 1); humanized mouse monoclonal [228-L-proline(H10-S>P)]$\gamma$4 heavy chain (134-218')-disulfide with humanized mouse monoclonal $\kappa$ light chain dimer (226-226":229-229")-bisdisulfide. The clinical

safety and efficacy of pembrolizumab in various forms of cancer is described in Fuerst, Oncology Times, 2014, 36, 35-36; Robert, et al., Lancet, 2014, 384, 1109-17; and Thomas, et al., Exp. Opin. Biol. Ther., 2014, 14, 1061-1064. In an embodiment, the pembrolizumab monoclonal antibody includes a heavy chain given by SEQ ID NO:25 and a light chain given by SEQ ID NO:26, and includes the following disulfide bridges: 22-96, 22"-96", 23'-92', 23"'-92"', 134-218', 134"-218"', 138'-198', 138"'-198"', 147-203, 147"-203", 226-226", 229-229", 261-321, 261"-321", 367-425, and 367"-425", and the following glycosylation sites (N): Asn-297 and Asn-297". Pembrolizumab is an IgG4/kappa isotype with a stabilizing S228P mutation in the Fc region; insertion of this mutation in the IgG4 hinge region prevents the formation of half molecules typically observed for IgG4 antibodies. Pembrolizumab is heterogeneously glycosylated at Asn297 within the Fc domain of each heavy chain, yielding a molecular weight of approximately 149 kDa for the intact antibody. The dominant glycoform of pembrolizumab is the fucosylated agalacto diantennary glycan form (G0F).

In an embodiment, an anti-PD-1 antibody comprises heavy and light chains having the sequences shown in SEQ ID NO:25 and SEQ ID NO:26, respectively, or antigen binding fragments and variants thereof. In an embodiment, an anti-PD-1 antibody comprises heavy and light chains that are each at least 99% identical to the sequences shown in SEQ ID NO:25 and SEQ ID NO:26, respectively, or antigen binding fragments and variants thereof. In an embodiment, an anti-PD-1 antibody comprises heavy and light chains that are each at least 98% identical to the sequences shown in SEQ ID NO:25 and SEQ ID NO:26, respectively, or antigen binding fragments and variants thereof. In an embodiment, an anti-PD-1 antibody comprises heavy and light chains that are each at least 97% identical to the sequences shown in SEQ ID NO:25 and SEQ ID NO:26, respectively, or antigen binding fragments and variants thereof. In an embodiment, an anti-PD-1 antibody comprises heavy and light chains that are each at least 96% identical to the sequences shown in SEQ ID NO:25 and SEQ ID NO:26, respectively, or antigen binding fragments and variants thereof. In an embodiment, an anti-PD-1 antibody comprises heavy and light chains that are each at least 95% identical to the sequences shown in SEQ ID NO:25 and SEQ ID NO:26, respectively, or antigen binding fragments and variants thereof.

In an embodiment, the anti-PD-1 antibody is an anti-PD-1 biosimilar monoclonal antibody approved by drug regulatory authorities with reference to pembrolizumab. In an embodiment, the biosimilar comprises an anti-PD-1 antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is pembrolizumab. In some embodiments, the one or more post-translational modifications are selected from one or more of: glycosylation, oxidation, deamidation, and truncation. In some embodiments, the biosimilar is an anti-PD-1 antibody authorized or submitted for authorization, wherein the anti-PD-1 antibody is provided in a formulation which differs from the formulations of a reference medicinal product or reference biological product, wherein the reference medicinal product or reference biological product is pembrolizumab. The anti-PD-1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is pembrolizumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is pembrolizumab.

In an embodiment, the anti-PD-1 antibody comprises the heavy and light chain CDRs or VRs of pembrolizumab. In one embodiment, the anti-PD-1 antibody $V_H$ region comprises the sequence shown in SEQ ID NO:27, and the anti-PD-1 antibody $V_L$ region comprises the sequence shown in SEQ ID NO:28. In an embodiment, an anti-PD-1 antibody comprises $V_H$ and $V_L$ regions that are each at least 99% identical to the sequences shown in SEQ ID NO:27 and SEQ ID NO:28, respectively. In an embodiment, an anti-PD-1 antibody comprises $V_H$ and $V_L$ regions that are each at least 98% identical to the sequences shown in SEQ ID NO:27 and SEQ ID NO:28, respectively. In an embodiment, an anti-PD-1 antibody comprises $V_H$ and $V_L$ regions that are each at least 97% identical to the sequences shown in SEQ ID NO:27 and SEQ ID NO:28, respectively. In an embodiment, an anti-PD-1 antibody comprises $V_H$ and $V_L$ regions that are each at least 96% identical to the sequences shown in SEQ ID NO:27 and SEQ ID NO:28, respectively. In an embodiment, an anti-PD-1 antibody comprises $V_H$ and $V_L$ regions that are each at least 95% identical to the sequences shown in SEQ ID NO:27 and SEQ ID NO:28, respectively. In an alternative embodiment, the antibody comprises $V_H$ and/or $V_L$ regions having the amino acid sequences set forth in SEQ ID NO:27 and/or SEQ ID NO:28, respectively.

In an embodiment, the anti-PD-1 antibody comprises heavy chain CDR1, CDR2 and CDR3 domains having the sequences set forth in SEQ ID NO:29, SEQ ID NO:30, and SEQ ID NO:31, respectively, and light chain CDR1, CDR2 and CDR3 domains having the sequences set forth in SEQ ID NO:32, SEQ ID NO:33, and SEQ ID NO:34, respectively.

In an embodiment, an anti-PD-1 antibody comprises a heavy chain that comprises CDR1, CDR2 and CDR3 domains that are each at least 95% identical to the sequences shown in SEQ ID NO:29, SEQ ID NO:30, and SEQ ID

NO:31, respectively. In an embodiment, an anti-PD-1 antibody comprises a heavy chain that comprises CDR1, CDR2 and CDR3 domains that are each at least 90% identical to the sequences shown in SEQ ID NO:29, SEQ ID NO:30, and SEQ ID NO:31, respectively. In an embodiment, an anti-PD-1 antibody comprises a heavy chain that comprises CDR1, CDR2 and CDR3 domains that are each at least 85% identical to the sequences shown in SEQ ID NO:29, SEQ ID NO:30, and SEQ ID NO:31, respectively. In an embodiment, an anti-PD-1 antibody comprises a heavy chain that comprises CDR1, CDR2 and CDR3 domains that are each at least 80% identical to the sequences shown in SEQ ID NO:29, SEQ ID NO:30, and SEQ ID NO:31, respectively. In another embodiment, the antibody competes for binding with, and/or binds to the same epitope on PD-1 as the aforementioned antibodies.

In an embodiment, an anti-PD-1 antibody comprises a light chain that comprises CDR1, CDR2 and CDR3 domains that are each at least 95% identical to the sequences shown in SEQ ID NO:32, SEQ ID NO:33, and SEQ ID NO:34, respectively. In an embodiment, an anti-PD-1 antibody comprises a light chain that comprises CDR1, CDR2 and CDR3 domains that are each at least 90% identical to the sequences shown in SEQ ID NO:32, SEQ ID NO:33, and SEQ ID NO:34, respectively. In an embodiment, an anti-PD-1 antibody comprises a light chain that comprises CDR1, CDR2 and CDR3 domains that are each at least 85% identical to the sequences shown in SEQ ID NO:32, SEQ ID NO:33, and SEQ ID NO:34, respectively. In an embodiment, an anti-PD-1 antibody comprises a light chain that comprises CDR1, CDR2 and CDR3 domains that are each at least 80% identical to the sequences shown in SEQ ID NO:32, SEQ ID NO:33, and SEQ ID NO:34, respectively. In another embodiment, the antibody competes for binding with, and/or binds to the same epitope on PD-1 as the aforementioned antibodies.

In an embodiment, the anti-PD-1 antibody is an antibody disclosed in U.S. Patent No. 8,354,509 or U.S. Patent Application Publication Nos. 2010/0266617 A1, 2013/0108651 A1, 2013/0109843 A2.

In an embodiment, the anti-PD-1 antibody is pidilizumab, which is also known as CT-011 (CureTech Ltd.), and which is disclosed in U.S. Patent No. 8,686,119 B2. The efficacy of pidilizumab in the treatment of cancers, such as hematological malignancies, is described in Berger, et al., Clin. Cancer Res. 2008, 14, 3044-51. The pidilizumab monoclonal antibody includes a heavy chain given by SEQ ID NO:35 and a light chain given by SEQ ID NO:36. Pidilizumab has intra-heavy chain disulfide linkages at 22-96, 144-200, 261-321, 367-425, 22''-96'', 144''-200'', 261''-321'', and 367''-425''; intra-light chain disulfide linkages at 23'-87', 133'-193', 23'''-87''', and 133'''-193'''; inter-heavy-light chain disulfide linkages at 220-213' and 220''-213''', inter-heavy-heavy chain disulfide linkages at 226-226'' 229-229''; and N-glycosylation sites (H $CH_2$ 84.4) at 297, 297''.

In an embodiment, the anti-PD-1 antibody is an immunoglobulin G1 kappa, anti-(human CD274) humanized monoclonal antibody. In an embodiment, an anti-PD-1 antibody comprises heavy and light chains having the sequences shown in SEQ ID NO:35 and SEQ ID NO:36, respectively, or antigen binding fragments, variants, or conjugates thereof. In an embodiment, an anti-PD-1 antibody comprises heavy and light chains that are each at least 99% identical to the sequences shown in SEQ ID NO:35 and SEQ ID NO:36, respectively. In an embodiment, an anti-PD-1 antibody comprises heavy and light chains that are each at least 98% identical to the sequences shown in SEQ ID NO:35 and SEQ ID NO:36, respectively. In an embodiment, an anti-PD-1 antibody comprises heavy and light chains that are each at least 97% identical to the sequences shown in SEQ ID NO:35 and SEQ ID NO:36, respectively. In an embodiment, an anti-PD-1 antibody comprises heavy and light chains that are each at least 96% identical to the sequences shown in SEQ ID NO:35 and SEQ ID NO:36, respectively. In an embodiment, an anti-PD-1 antibody comprises heavy and light chains that are each at least 95% identical to the sequences shown in SEQ ID NO:35 and SEQ ID NO:36, respectively.

In an embodiment, the anti-PD-1 antibody is an anti-PD-1 biosimilar monoclonal antibody approved by drug regulatory authorities with reference to pidilizumab. In an embodiment, the biosimilar comprises an anti-PD-1 antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is pidilizumab. In some embodiments, the one or more post-translational modifications are selected from one or more of: glycosylation, oxidation, deamidation, and truncation. In some embodiments, the biosimilar is an anti-PD-1 antibody authorized or submitted for authorization, wherein the anti-PD-1 antibody is provided in a formulation which differs from the formulations of a reference medicinal product or reference biological product, wherein the reference medicinal product or reference biological product is pidilizumab. The anti-PD-1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is pidilizumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is pidilizumab.

In an embodiment, an anti-PD-L1 antibody comprises $V_H$ and $V_L$ regions that are each at least 99% identical

to the sequences shown in SEQ ID NO:37 and SEQ ID NO:38, respectively. In an embodiment, an anti-PD-L1 antibody comprises $V_H$ and $V_L$ regions that are each at least 98% identical to the sequences shown in SEQ ID NO:37 and SEQ ID NO:38, respectively. In an embodiment, an anti-PD-L1 antibody comprises $V_H$ and $V_L$ regions that are each at least 97% identical to the sequences shown in SEQ ID NO:37 and SEQ ID NO:38, respectively. In an embodiment, an anti-PD-L1 antibody comprises $V_H$ and $V_L$ regions that are each at least 96% identical to the sequences shown in SEQ ID NO:37 and SEQ ID NO:38, respectively. In an embodiment, an anti-PD-L1 antibody comprises $V_H$ and $V_L$ regions that are each at least 95% identical to the sequences shown in SEQ ID NO:37 and SEQ ID NO:38, respectively.

In an embodiment, anti-PD-1 antibodies and other PD-1 inhibitors include those described in U.S. Patent Nos. 8,287,856, 8,580,247, and 8,168,757 and U.S. Patent Application Publication Nos. 2009/0028857 A1, 2010/0285013 A1, 2013/0022600 A1, and 2011/0008369 A1. In another embodiment, antibodies that compete with any of these antibodies for binding to PD-1 are also included. In another embodiment, the anti-PD-1 antibody is an antibody disclosed in U.S. Patent No. 8,735,553 B1.

In an embodiment, the anti-PD-1 antibody is a commercially-available monoclonal antibody, such as anti-m-PD-1 clones J43 (Cat # BE0033-2) and RMP1-14 (Cat # BE0146) (Bio X Cell, Inc., West Lebanon, NH, USA). A number of commercially-available anti-PD-1 antibodies are known to one of ordinary skill in the art.

Monoclonal antibodies that inhibit or block PD-1 can be prepared by procedures known to those of ordinary knowledge and skill in the art, *e.g.*, by injecting test subjects with PD-1 antigen and then isolating hybridomas expressing antibodies having the desired sequence or functional characteristics. DNA encoding the monoclonal antibodies is readily isolated and sequenced using conventional procedures (*e.g.*, by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, myeloma cells, or other suitable cells that do not otherwise produce immunoglobulin protein, to obtain the synthesis of monoclonal antibodies in the recombinant host cells. The details of recombinant production of specific antibodies may be found in the references cited in the foregoing. Monoclonal antibodies that inhibit PD-1 can be prepared by standard molecular biology methods using the sequences provided herein by reverse translation and insertion into appropriate DNA or RNA vectors.

In an embodiment, the PD-1 inhibitor may be a small molecule or a peptide, or a peptide derivative, such as those described in U.S. Patent Nos. 8,907,053; 9,096,642; and 9,044,442 and U.S. Patent Application Publication No. US 2015/0087581; 1,2,4-oxadiazole compounds and derivatives such as those described in U.S. Patent Application Publication No. 2015/0073024; cyclic peptidomimetic compounds and derivatives such as those described in U.S. Patent Application Publication No. US 2015/0073042; cyclic compounds and derivatives such as those described in U.S. Patent Application Publication No. US 2015/0125491; 1,3,4-oxadiazole and 1,3,4-thiadiazole compounds and derivatives such as those described in International Patent Application Publication No. WO 2015/033301; peptide-based compounds and derivatives such as those described in International Patent Application Publication Nos. WO 2015/036927 and WO 2015/04490, or a macrocyclic peptide-based compounds and derivatives such as those described in U.S. Patent Application Publication No. US 2014/0294898.

The anti-PD-1 antibody sequences discussed and referenced in some of the foregoing embodiments are summarized in Table 2.

TABLE 2. Anti-PD-1 antibody amino acid sequences.

| Identifier | Sequence (One-Letter Amino Acid Symbols) |
|---|---|
| SEQ ID NO:15 nivolumab heavy chain | QVQLVESGGG VVQPGRSLRL DCKASGITFS NSGMHWVRQA PGKGLEWVAV IWYDGSKRYY 60<br>ADSVKGRFTI SRDNSKNTLF LQMNSLRAED TAVYYCATND DYWGQGTLVT VSSASTKGPS 120<br>VFPLAPCSRS TSESTAALGC LVKDYFPEPV TVSWNSGALT SGVHTFPAVL QSSGLYSLSS 180<br>VVTVPSSSLG TKTYTCNVDH KPSNTKVDKR VESKYGPPCP PCPAPEFLGG PSVFLFPPKP 240<br>KDTLMISRTP EVTCVVVDVS QEDPEVQFNW YVDGVEVHNA KTKPREEQFN STYRVVSVLT 300<br>VLHQDWLNGK EYKCKVSNKG LPSSIEKTIS KAKGQPREPQ VYTLPPSQEE MTKNQVSLTC 360<br>LVKGFYPSDI AVEWESNGQP ENNYKTTPPV LDSDGSFFLY SRLTVDKSRW QEGNVFSCSV 420<br>MHEALHNHYT QKSLSLSLGK 440 |
| SEQ ID NO:16 nivolumab light chain | EIVLTQSPAT LSLSPGERAT LSCRASQSVS SYLAWYQQKP GQAPRLLIYD ASNRATGIPA 60<br>RFSGSGSGTD FTLTISSLEP EDFAVYYCQQ SSNWPRTFGQ GTKVEIKRTV AAPSVFIFPP 120<br>SDEQLKSGTA SVVCLLNNFY PREAKVQWKV DNALQSGNSQ ESVTEQDSKD STYSLSSTLT 180<br>LSKADYEKHK VYACEVTHQG LSSPVTKSFN RGEC 214 |
| SEQ ID NO:17 nivolumab variable heavy chain | QVQLVESGGG VVQPGRSLRL DCKASGITFS NSGMHWVRQA PGKGLEWVAV IWYDGSKRYY 60<br>ADSVKGRFTI SRDNSKNTLF LQMNSLRAED TAVYYCATND DYWGQGTLVT VSS 113 |

(continued)

| Identifier | Sequence (One-Letter Amino Acid Symbols) |
|---|---|
| SEQ ID NO:18 nivolumab variable light chain | EIVLTQSPAT LSLSPGERAT LSCRASQSVS SYLAWYQQKP GQAPRLLIYD ASNRATGIPA 60<br>RFSGSGSGTD FTLTISSLEP EDFAVYYCQQ SSNWPRTFGQ GTKVEIK 107 |
| SEQ ID NO:19 nivolumab heavy chain CDR1 | NSGMH 5 |
| SEQ ID NO:20 nivolumab heavy chain CDR2 | VIWYDGSKRY YADSVKG 17 |
| SEQ ID NO:21 nivolumab heavy chain CDR3 | NDDY 4 |
| SEQ ID NO:22 nivolumab light chain CDR1 | RASQSVSSYL A 11 |
| SEQ ID NO:23 nivolumab light chain CDR2 | DASNRAT 7 |
| SEQ ID NO:24 nivolumab light chain CDR3 | QQSSNWPRT 9 |
| SEQ ID NO:25 pembrolizumab heavy chain | QVQLVQSGVE VKKPGASVKV SCKASGYTFT NYYMYWVRQA PGQGLEWMGG INPSNGGTNF 60<br>NEKFKNRVTL TTDSSTTTAY MELKSLQFDD TAVYYCARRD YRFDMGFDYW GQGTTVTVSS 120<br>ASTKGPSVFP LAPCSRSTSE STAALGCLVK DYFPEPVTVS WNSGALTSGV HTFPAVLQSS 180<br>GLYSLSSVVT VPSSSLGTKT YTCNVDHKPS NTKVDKRVES KYGPPCPPCP APEFLGGPSV 240<br>FLFPPKPKDT LMISRTPEVT CVVVDVSQED PEVQFNWYVD GVEVHNAKTK PREEQFNSTY 300<br>RVVSVLTVLH QDWLNGKEYK CKVSNKGLPS SIEKTISKAK GQPREPQVYT LPPSQEEMTK 360<br>NQVSLTCLVK GFYPSDIAVE WESNGQPENN YKTTPPVLDS DGSFFLYSRL TVDKSRWQEG 420<br>NVFSCSVMHE ALHNHYTQKS LSLSLGK 447 |
| SEQ ID NO:26 pembrolizumab light chain | EIVLTQSPAT LSLSPGERAT LSCRASKGVS TSGYSYLHWY QQKPGQAPRL LIYLASYLES 60<br>GVPARFSGSG SGTDFTLTIS SLEPEDFAVY YCQHSRDLPL TFGGGTKVEI KRTVAAPSVF 120<br>IFPPSDEQLK SGTASVVCLL NNFYPREAKV QWKVDNALQS GNSQESVTEQ DSKDSTYSLS 180<br>STLTLSKADY EKHKVYACEV THQGLSSPVT KSFNRGEC 218 |
| SEQ ID NO:27 pembrolizumab variable heavy chain | QVQLVQSGVE VKKPGASVKV SCKASGYTFT NYYMYWVRQA PGQGLEWMGG INPSNGGTNF 60<br>NEKFKNRVTL TTDSSTTTAY MELKSLQFDD TAVYYCARRD YRFDMGFDYW GQGTTVTVSS 120 |
| SEQ ID NO:28 pembrolizumab variable light chain | EIVLTQSPAT LSLSPGERAT LSCRASKGVS TSGYSYLHWY QQKPGQAPRL LIYLASYLES 60<br>GVPARFSGSG SGTDFTLTIS SLEPEDFAVY YCQHSRDLPL TFGGGTKVEI K 111 |
| SEQ ID NO:29 pembrolizumab heavy chain CDR1 | NYYMY 5 |
| SEQ ID NO:30 pembrolizumab heavy chain CDR2 | GINPSNGGTN FNEKFK 16 |
| SEQ ID NO:31 pembrolizumab heavy chain CDR3 | RDYRFDMGFD Y 11 |

(continued)

| Identifier | Sequence (One-Letter Amino Acid Symbols) |
|---|---|
| SEQ ID NO:32 pembrolizumab light chain CDR1 | RASKGVSTSG YSYLH 15 |
| SEQ ID NO:33 pembrolizumab light chain CDR2 | LASYLES 7 |
| SEQ ID NO:34 pembrolizumab light chain CDR3 | QHSRDLPLT 9 |
| SEQ ID NO:35 pidilizumab heavy chain | QVQLVQSGSE LKKPGASVKI SCKASGYTFT NYGMNWVRQA PGQGLQWMGW INTDSGESTY 60<br>AEEFKGRFVF SLDTSVNTAY LQITSLTAED TGMYFCVRVG YDALDYWGQG TLVTVSSAST 120<br>KGPSVFPLAP SSKSTSGGTA ALGCLVKDYF PEPVTVSWNS GALTSGVHTF PAVLQSSGLY 180<br>SLSSVVTVPS SSLGTQTYIC NVNHKPSNTK VDKRVEPKSC DKTHTCPPCP APELLGGPSV 240<br>FLFPPKPKDT LMISRTPEVT CVVVDVSHED PEVKFNWYVD GVEVHNAKTK PREEQYNSTY 300<br>RVVSVLTVLH QDWLNGKEYK CKVSNKALPA PIEKTISKAK GQPREPQVYT LPPSREEMTK 360<br>NQVSLTCLVK GFYPSDIAVE WESNGQPENN YKTTPPVLDS DGSFFLYSKL TVDKSRWQQG 420<br>NVFSCSVMHE ALHNHYTQKS LSLSPGK 447 |
| SEQ ID NO:36 pidilizumab light chain | EIVLTQSPSS LSASVGDRVT ITCSARSSVS YMHWFQQKPG KAPKLWIYRT SNLASGVPSR 60<br>FSGSGSGTSY CLTINSLQPE DFATYYCQQR SSFPLTFGGG TKLEIKRTVA APSVFIFPPS 120<br>DEQLKSGTAS VVCLLNNFYP REAKVQWKVD NALQSGNSQE SVTEQDSKDS TYSLSSTLTL 180<br>SKADYEKHKV YACEVTHQGL SSPVTKSFNR GEC 213 |
| SEQ ID NO:37 pidilizumab variable heavy chain | QVQLVQSGSE LKKPGASVKI SCKASGYTFT NYGMNWVRQA PGQGLQWMGW INTDSGESTY 60<br>AEEFKGRFVF SLDTSVNTAY LQITSLTAED TGMYFCVRVG YDALDYWGQG TLVTVSS 117 |
| SEQ ID NO:38 pidilizumab variable light chain | EIVLTQSPSS LSASVGDRVT ITCSARSSVS YMHWFQQKPG KAPKLWIYRT SNLASGVPSR 60<br>FSGSGSGTSY CLTINSLQPE DFATYYCQQR SSFPLTFGGG TKLEIK 106 |

The PD-L1 or PD-L2 inhibitor may be any PD-L1 or PD-L2 inhibitor or blocker known in the art. In particular, it is one of the PD-L1 or PD-L2 inhibitors or blockers described in more detail in the following paragraphs. The terms "inhibitor" and "blocker" are used interchangeably herein in reference to PD-L1 and PD-L2 inhibitors. For avoidance of doubt, references herein to a PD-L1 or PD-L2 inhibitor that is an antibody may refer to a compound or antigen-binding fragments, variants, conjugates, or biosimilars thereof. For avoidance of doubt, references herein to a PD-L1 or PD-L2 inhibitor may refer to a compound or a pharmaceutically acceptable salt thereof.

In some embodiments, the compositions and methods include a PD-L1 or PD-L2 inhibitor. In some embodiments, the PD-L1 and PD-L2 inhibitor is a small molecule. In some embodiments, the PD-L1 or PD-L2 inhibitor is an anti-PD-L1 or anti-PD-L2 antibody, a fragment thereof, including Fab fragments or single-chain variable fragments (scFv). In an aspect of the invention, the anti-PD-1 antibody or fragment thereof in any of the aforementioned embodiments is replaced by, or combined with, an anti-PD-L1 or anti-PD-L2 antibody or fragment thereof. In an embodiment, the antibody competes for binding with, and/or binds to an epitope on PD-L1 and/or PD-L2. In some embodiments, the PD-L1 or PD-L2 inhibitor is a monoclonal antibody. In some embodiments the PD-L1 or PD-L2 inhibitor is a polyclonal antibody. In some embodiments, a PD-L1 inhibitor is included in a composition and is further combined with a BTK inhibitor of formula (2) and methotrexate. In some embodiments, an anti-PD-L1 monoclonal antibody is included in a composition and is further combined with a BTK inhibitor of formula (2) and methotrexate. In some embodiments, a PD-L2 inhibitor is included in a composition and is further combined with a BTK inhibitor of formula (2) and methotrexate. In some embodiments, an anti-PD-L2 monoclonal antibody is included in a composition and is further combined with a BTK inhibitor of formula (2) and methotrexate.

In preferred embodiments, the compositions described herein provide a combination of a PD-L1 and/or PD-L2 inhibitor with a BTK inhibitor of formula (2) and methotrexate. In some embodiments, the PD-L1 inhibitors provided herein are selective for PD-L1, in that the compounds bind or interact with PD-L1 at substantially lower concentrations than they bind or interact with other receptors, including the PD-L2 receptor. In certain embodiments, the compounds bind to the PD-L2 receptor at a binding constant that is at least about a 2-fold higher concentration, about a 3-fold higher concentration, about a 5-fold higher concentration, about a 10-fold higher concentration, about a 20-fold higher concentration, about a 30-fold higher concentration, about a 50-fold higher concentration, about a 100-fold higher concentration,

about a 200-fold higher concentration, about a 300-fold higher concentration, or about a 500-fold higher concentration than to the PD-L1 receptor.

In some embodiments, the compositions described include a PD-L1 and/or PD-L2 inhibitor that binds human PD-L1 and/or PD-L2 with a $K_D$ of about 100 pM or lower, binds human PD-L1 and/or PD-L2 with a $K_D$ of about 90 pM or lower, binds human PD-L1 and/or PD-L2 with a $K_D$ of about 80 pM or lower, binds human PD-L1 and/or PD-L2 with a $K_D$ of about 70 pM or lower, binds human PD-L1 and/or PD-L2 with a $K_D$ of about 60 pM or lower, a $K_D$ of about 50 pM or lower, binds human PD-L1 and/or PD-L2 with a $K_D$ of about 40 pM or lower, or binds human PD-L1 and/or PD-L2 with a KD of about 30 pM or lower,

In some embodiments, the compositions described include a PD-L1 and/or PD-L2 inhibitor that binds to human PD-L1 and/or PD-L2 with a $k_{assoc}$ of about $7.5 \times 10^5$ 1/M·s or faster, binds to human PD-L1 and/or PD-L2 with a $k_{assoc}$ of about $8 \times 10^5$ 1/M·s or faster, binds to human PD-L1 and/ or PD-L2 with a $k_{assoc}$ of about $8.5 \times 10^5$ 1/M·s or faster, binds to human PD-L1 and/or PD-L2 with a $k_{assoc}$ of about $9 \times 10^5$ 1/M·s or faster, binds to human PD-L1 and/or PD-L2 with a $k_{assoc}$ of about $9.5 \times 10^5$ 1/M·s and/or faster, or binds to human PD-L1 and/or PD-L2 with a $k_{assoc}$ of about $1 \times 10^6$ 1/M·s or faster.

In some embodiments, the compositions described include a PD-L1 and/or PD-L2 inhibitor that binds to human PD-L1 or PD-L2 with a $k_{dissoc}$ of about $2 \times 10^{-5}$ 1/s or slower, binds to human PD-1 with a $k_{dissoc}$ of about $2.1 \times 10^{-5}$ 1/s or slower , binds to human PD-1 with a $k_{dissoc}$ of about $2.2 \times 10^{-5}$ 1/s or slower, binds to human PD-1 with a $k_{dissoc}$ of about $2.3 \times 10^{-5}$ 1/s or slower, binds to human PD-1 with a $k_{dissoc}$ of about $2.4 \times 10^{-5}$ 1/s or slower, binds to human PD-1 with a $k_{dissoc}$ of about $2.5 \times 10^{-5}$ 1/s or slower, binds to human PD-1 with a $k_{dissoc}$ of about $2.6 \times 10^{-5}$ 1/s or slower, binds to human PD-L1 or PD-L2 with a $k_{dissoc}$ of about $2.7 \times 10^{-5}$ 1/s or slower, or binds to human PD-L1 or PD-L2 with a $k_{dissoc}$ of about $3 \times 10^{-5}$ 1/s or slower.

In some embodiments, the compositions described include a PD-L1 and/or PD-L2 inhibitor that blocks or inhibits binding of human PD-L1 or human PD-L2 to human PD-1 with an $IC_{50}$ of about 10 nM or lower; blocks or inhibits binding of human PD-L1 or human PD-L2 to human PD-1 with an $IC_{50}$ of about 9 nM or lower; blocks or inhibits binding of human PD-L1 or human PD-L2 to human PD-1 with an $IC_{50}$ of about 8 nM or lower; blocks or inhibits binding of human PD-L1 or human PD-L2 to human PD-1 with an $IC_{50}$ of about 7 nM or lower; blocks or inhibits binding of human PD-L1 or human PD-L2 to human PD-1 with an $IC_{50}$ of about 6 nM or lower; blocks or inhibits binding of human PD-L1 or human PD-L2 to human PD-1 with an $IC_{50}$ of about 5 nM or lower; blocks or inhibits binding of human PD-L1 or human PD-L2 to human PD-1 with an $IC_{50}$ of about 4 nM or lower; blocks or inhibits binding of human PD-L1 or human PD-L2 to human PD-1 with an $IC_{50}$ of about 3 nM or lower; blocks or inhibits binding of human PD-L1 or human PD-L2 to human PD-1 with an $IC_{50}$ of about 2 nM or lower; or blocks human PD-1, or blocks binding of human PD-L1 or human PD-L2 to human PD-1 with an $IC_{50}$ of about 1 nM or lower.

In an embodiment, the anti-PD-L1 antibody is durvalumab, also known as MEDI4736 (which is commercially available from Medimmune, LLC, Gaithersburg, Maryland, a subsidiary of AstraZeneca plc.), or antigen-binding fragments, conjugates, or variants thereof. In an embodiment, the anti-PD-L1 antibody is an antibody disclosed in U.S. Patent No. 8,779,108 or U.S. Patent Application Publication No. 2013/0034559. The clinical efficacy of durvalumab (SEQ ID NO:403 and SEQ ID NO:404) has been described in: Page, et al., Ann. Rev. Med., 2014, 65, 185-202; Brahmer, et al., J. Clin. Oncol. 2014, 32, 5s (supplement, abstract 8021); and McDermott, et al., Cancer Treatment Rev., 2014, 40, 1056-64. The durvalumab monoclonal antibody includes a $V_H$ region given by SEQ ID NO:41 (corresponding to SEQ ID NO:72 in U.S. Patent No. 8,779,108) and a $V_L$ region given by SEQ ID NO:42 (corresponding to SEQ ID NO:77 in U.S. Patent No. 8,779,108). The durvalumab monoclonal antibody includes disulfide linkages at 22-96, 22"-96", 23'-89', 23'''-89''', 135'-195', 135'''-195''', 148-204, 148"-204", 215'-224, 215'''-224", 230-230", 233-233", 265-325, 265"-325", 371-429, and 371"-429'; and N-glycosylation sites at Asn-301 and Asn-301".

In an embodiment, the anti-PD-L1 antibody is an immunoglobulin G1, anti-(human CD antigen CD274) (human monoclonal heavy chain), disulfide with human monoclonal κ-chain, dimer. In an embodiment, the anti-PD-L1 antibody comprises the heavy and light chains of durvalumab (MEDI4736). In an embodiment, an anti-PD-L1 antibody comprises heavy and light chains having the sequences shown in SEQ ID NO:39 and SEQ ID NO:40, respectively, or antigen binding fragments, variants, or conjugates thereof. In an embodiment, an anti-PD-L1 antibody comprises heavy and light chains that are each at least 99% identical to the sequences shown in SEQ ID NO:39 and SEQ ID NO:40, respectively. In an embodiment, an anti-PD-L1 antibody comprises heavy and light chains that are each at least 98% identical to the sequences shown in SEQ ID NO:39 and SEQ ID NO:40, respectively. In an embodiment, an anti-PD-L1 antibody comprises heavy and light chains that are each at least 97% identical to the sequences shown in SEQ ID NO:39 and SEQ ID NO:40, respectively. In an embodiment, an anti-PD-L1 antibody comprises heavy and light chains that are each at least 96% identical to the sequences shown in SEQ ID NO:39 and SEQ ID NO:40, respectively. In an embodiment, an anti-PD-L1 antibody comprises heavy and light chains that are each at least 95% identical to the sequences shown in SEQ ID NO:39 and SEQ ID NO:40, respectively.

In an embodiment, the anti-PD-L1 antibody is an anti-PD-L1 biosimilar monoclonal antibody approved by drug regulatory authorities with reference to durvalumab. In an embodiment, the biosimilar comprises an anti-PD-L1 antibody

comprising an amino acid sequence which has at least 97% sequence identity, e.g., 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is durvalumab. In some embodiments, the one or more post-translational modifications are selected from one or more of: glycosylation, oxidation, deamidation, and truncation. In some embodiments, the biosimilar is an anti-PD-L1 antibody authorized or submitted for authorization, wherein the anti-PD-L1 antibody is provided in a formulation which differs from the formulations of a reference medicinal product or reference biological product, wherein the reference medicinal product or reference biological product is durvalumab. The anti-PD-L1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is durvalumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is durvalumab.

In an embodiment, the anti-PD-L1 antibody comprises $V_H$ and $V_L$ regions having the sequences shown in SEQ ID NO:41 (corresponding to SEQ ID NO:72 in U.S. Patent No. 8,779,108) and SEQ ID NO:42 (corresponding to SEQ ID NO:77 in U.S. Patent No. 8,779,108), respectively, as described in U.S. Patent No. 8,779,108 or U.S. Patent Application Publication No. US 2013/0034559, the disclosures of which are specifically incorporated by reference herein, including antigen binding fragments, conjugates, and variants thereof. In an embodiment, an anti-PD-L1 antibody comprises $V_H$ and $V_L$ regions that are each at least 99% identical to the sequences shown in SEQ ID NO:41 and SEQ ID NO:42, respectively. In an embodiment, an anti-PD-L1 antibody comprises $V_H$ and $V_L$ regions that are each at least 98% identical to the sequences shown in SEQ ID NO:41 and SEQ ID NO:42, respectively. In an embodiment, an anti-PD-L1 antibody comprises $V_H$ and $V_L$ regions that are each at least 97% identical to the sequences shown in SEQ ID NO:41 and SEQ ID NO:42, respectively. In an embodiment, an anti-PD-L1 antibody comprises $V_H$ and $V_L$ regions that are each at least 96% identical to the sequences shown in SEQ ID NO:41 and SEQ ID NO:42, respectively. In an embodiment, an anti-PD-L1 antibody comprises $V_H$ and $V_L$ regions that are each at least 95% identical to the sequences shown in SEQ ID NO:41 and SEQ ID NO:42, respectively. In an embodiment, an anti-PD-L1 antibody comprises $V_H$ and $V_L$ regions that are each at least 90% identical to the sequences shown in SEQ ID NO:41 and SEQ ID NO:42, respectively.

In an embodiment, the anti-PD-L1 antibody comprises heavy chain CDR1, CDR2 and CDR3 domains having the sequences set forth in SEQ ID NO:43, SEQ ID NO:44, and SEQ ID NO:45, respectively, and light chain CDR1, CDR2 and CDR3 domains having the sequences set forth in SEQ ID NO:43, SEQ ID NO:44, and SEQ ID NO:45, respectively.

In an embodiment, an anti-PD-L1 antibody comprises a heavy chain that comprises CDR1, CDR2 and CDR3 domains that are each at least 95% identical to the sequences shown in SEQ ID NO:43, SEQ ID NO:44, and SEQ ID NO:45, respectively. In an embodiment, an anti-PD-L1 antibody comprises a heavy chain that comprises CDR1, CDR2 and CDR3 domains that are each at least 90% identical to the sequences shown in SEQ ID NO:43, SEQ ID NO:44, and SEQ ID NO:45, respectively. In an embodiment, an anti-PD-L1 antibody comprises a heavy chain that comprises CDR1, CDR2 and CDR3 domains that are each at least 85% identical to the sequences shown in SEQ ID NO:43, SEQ ID NO:44, and SEQ ID NO:45, respectively. In an embodiment, an anti-PD-L1 antibody comprises a heavy chain that comprises CDR1, CDR2 and CDR3 domains that are each at least 80% identical to the sequences shown in SEQ ID NO:43, SEQ ID NO:44, and SEQ ID NO:45, respectively. In another embodiment, the antibody competes for binding with, and/or binds to the same epitope on PD-L1 as the aforementioned antibodies.

In an embodiment, an anti-PD-L1 antibody comprises a light chain that comprises CDR1, CDR2 and CDR3 domains that are each at least 95% identical to the sequences shown in SEQ ID NO:46, SEQ ID NO:47, and SEQ ID NO:48, respectively. In an embodiment, an anti-PD-L1 antibody comprises a light chain that comprises CDR1, CDR2 and CDR3 domains that are each at least 90% identical to the sequences shown in SEQ ID NO:46, SEQ ID NO:47, and SEQ ID NO:48, respectively. In an embodiment, an anti-PD-L1 antibody comprises a light chain that comprises CDR1, CDR2 and CDR3 domains that are each at least 85% identical to the sequences shown in SEQ ID NO:46, SEQ ID NO:47, and SEQ ID NO:48, respectively. In an embodiment, an anti-PD-L1 antibody comprises a light chain that comprises CDR1, CDR2 and CDR3 domains that are each at least 80% identical to the sequences shown in SEQ ID NO:46, SEQ ID NO:47, and SEQ ID NO:48, respectively. In another embodiment, the antibody competes for binding with, and/or binds to the same epitope on PD-L1 as the aforementioned antibodies.

In an embodiment, anti-PD-L1 antibodies and other PD-L1 inhibitors include those described in U.S. Patent No. 8,779,108 and U.S. Patent Application Publication No. US 2013/0034559A1. In another embodiment, antibodies that compete with any of these antibodies for binding to PD-L1 are also included.

In an embodiment, the anti-PD-L1 antibody is atezolizumab, also known as MPDL3280A or RG7446 (commercially available from Genentech, Inc., a subsidiary of Roche), or antigen-binding fragments, conjugates, or variants thereof. In an embodiment, the anti-PD-L1 antibody is an antibody disclosed in U.S. Patent No. 8,217,149. In an embodiment, the anti-PD-L1 antibody is an antibody disclosed in U.S. Patent Application Publication Nos. 2010/0203056 A1, 2013/0045200 A1, 2013/0045201 A1, 2013/0045202 A1, or 2014/0065135 A1. The atezolizumab monoclonal antibody includes a heavy chain given by SEQ ID NO:49 and a light chain given by SEQ ID NO:50. Atezolizumab has intra-heavy chain disulfide linkages (C23-C104) at 22-96, 145-201, 262-322, 368-426, 22''-96'', 145''-201'', 262''-322'', and 368''-426''; intra-light chain disulfide linkages (C23-C104) at 23'-88', 134'-194', 23'''-88''', and 134'''-194'''; intra-heavy-light chain disulfide linkages (h 5-CL 126) at 221-214' and 221''-214'''; intra-heavy-heavy chain disulfide linkages (h 11, h 14) at 227-227'' and 230-230''; and N-glycosylation sites (H $CH_2$ N84.4>A) at 298 and 298'.

In an embodiment, the anti-PD-L1 antibody is an immunoglobulin G1 kappa, anti-(human PD-L1) humanized monoclonal antibody. In an embodiment, the anti-PD-L1 antibody comprises the heavy and light chains of atezolizumab (MPDL3280A). In an embodiment, an anti-PD-L1 antibody comprises heavy and light chains having the sequences shown in SEQ ID NO:49 and SEQ ID NO:50, respectively, or antigen binding fragments, variants, or conjugates thereof. In an embodiment, an anti-PD-L1 antibody comprises heavy and light chains that are each at least 99% identical to the sequences shown in SEQ ID NO:49 and SEQ ID NO:50, respectively. In an embodiment, an anti-PD-L1 antibody comprises heavy and light chains that are each at least 98% identical to the sequences shown in SEQ ID NO:49 and SEQ ID NO:50, respectively. In an embodiment, an anti-PD-L1 antibody comprises heavy and light chains that are each at least 97% identical to the sequences shown in SEQ ID NO:49 and SEQ ID NO:50, respectively. In an embodiment, an anti-PD-L1 antibody comprises heavy and light chains that are each at least 96% identical to the sequences shown in SEQ ID NO:49 and SEQ ID NO:50, respectively. In an embodiment, an anti-PD-L1 antibody comprises heavy and light chains that are each at least 95% identical to the sequences shown in SEQ ID NO:49 and SEQ ID NO:50, respectively.

In an embodiment, the anti-PD-L1 antibody is an anti-PD-L1 biosimilar monoclonal antibody approved by drug regulatory authorities with reference to atezolizumab. In an embodiment, the biosimilar comprises an anti-PD-L1 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is atezolizumab. In some embodiments, the one or more post-translational modifications are selected from one or more of: glycosylation, oxidation, deamidation, and truncation. In some embodiments, the biosimilar is an anti-PD-L1 antibody authorized or submitted for authorization, wherein the anti-PD-L1 antibody is provided in a formulation which differs from the formulations of a reference medicinal product or reference biological product, wherein the reference medicinal product or reference biological product is atezolizumab. The anti-PD-L1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is atezolizumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is atezolizumab.

In an embodiment, the anti-PD-L1 antibody comprises the heavy and light chain CDRs or VRs of atezolizumab (MPDL3280A). In an embodiment, the anti-PD-L1 antibody $V_H$ region comprises the sequence shown in SEQ ID NO:51 (corresponding to SEQ ID NO:20 in U.S. Patent No. 8,217,149), and the anti-PD-L1 antibody $V_L$ region comprises the sequence shown in SEQ ID NO:52 (corresponding to SEQ ID NO:21 in U.S. Patent No. 8,217,149). In an embodiment, an anti-PD-L1 antibody comprises $V_H$ and $V_L$ regions that are each at least 99% identical to the sequences shown in SEQ ID NO:51 and SEQ ID NO:52, respectively. In an embodiment, an anti-PD-L1 antibody comprises $V_H$ and $V_L$ regions that are each at least 98% identical to the sequences shown in SEQ ID NO:51 and SEQ ID NO:52, respectively. In an embodiment, an anti-PD-L1 antibody comprises $V_H$ and $V_L$ regions that are each at least 97% identical to the sequences shown in SEQ ID NO:51 and SEQ ID NO:52, respectively. In an embodiment, an anti-PD-L1 antibody comprises $V_H$ and $V_L$ regions that are each at least 96% identical to the sequences shown in SEQ ID NO:51 and SEQ ID NO:52, respectively. In an embodiment, an anti-PD-L1 antibody comprises $V_H$ and $V_L$ regions that are each at least 95% identical to the sequences shown in SEQ ID NO:51 and SEQ ID NO:52, respectively.

In an embodiment, the anti-PD-L1 antibody comprises a heavy chain variable region ($V_H$) polypeptide that comprises a CDR1, CDR2, and CDR3 sequence, wherein the CDR1 sequence is given by SEQ ID NO:53 (GFTFSX$_1$SWIH) (corresponding to SEQ ID NO:1 in U.S. Patent No. 8,217,149), the CDR2 sequence is SEQ ID NO:54 (AWIX$_2$PYGGSX$_3$YYADSVKG) (corresponding to SEQ ID NO:2 in U.S. Patent No. 8,217,149), and the CDR3 sequence is SEQ ID NO:55 (RHWPGGFDY) (corresponding to SEQ ID NO:3 in U.S. Patent No. 8,217,149), further wherein $X_1$ is D or G, $X_2$ is S or L, and $X_3$ is T or S, and the anti-PD-L1 antibody also comprises a light chain variable region ($V_L$) polypeptide that comprises a CDR1, CDR2, and CDR3 sequence wherein the CDR1 sequence is given by SEQ ID

NO:56 (RASQ$X_4X_5X_6$T$X_7X_8$A) (corresponding to SEQ ID NO:8 in U.S. Patent No. 8,217,149), the CDR2 sequence is given by SEQ ID NO:57 (SAS$X_9$L$X_{10}$S) (corresponding to SEQ ID NO:9 in U.S. Patent No. 8,217,149), and the CDR3 sequence is SEQ ID NO:58 (QQ$X_{11}X_{12}X_{13}X_{14}$P$X_{15}$T) (corresponding to SEQ ID NO: 10 in U.S. Patent No. 8,217,149), further wherein further wherein: $X_4$ is D or V; $X_5$ is V or I; $X_6$ is S or N; $X_7$ is A or F; Xg is V or L; $X_9$ is F or T; $X_{10}$ is Y or A; $X_{11}$ is Y, G, F, or S; $X_{12}$ is L, Y, F or W; $X_{13}$ is Y, N, A, T, G, F or I; $X_{14}$ is H, V, P, T or I; and $X_{15}$ is A, W, R, P or T.

In an embodiment, the anti-PD-L1 antibody is avelumab, also known as MSB0010718C (commercially available from Merck KGaA/EMD Serono), or antigen-binding fragments, conjugates, or variants thereof. In an embodiment, the anti-PD-L1 antibody is an antibody disclosed in U.S. Patent Application Publication No. US 2014/0341917 A1. The avelumab monoclonal antibody includes a heavy chain given by SEQ ID NO:59 and a light chain given by SEQ ID NO:60. Avelumab has intra-heavy chain disulfide linkages (C23-C104) at 22-96, 147-203, 264-324, 370-428, 22"-96", 147"-203", 264"-324", and 370"-428"; intra-light chain disulfide linkages (C23-C104) at 22'-90', 138'-197', 22"'-90"', and 138"'-197"'; intra-heavy-light chain disulfide linkages (h 5-CL 126) at 223-215' and 223"-215"'; intra-heavy-heavy chain disulfide linkages (h 11, h 14) at 229-229" and 232-232"; N-glycosylation sites (H $CH_2$ N84.4) at 300, 300"; fucosylated complex bi-antennary CHO-type glycans; and H CHS K2 C-terminal lysine clipping at 450 and 450'.

In an embodiment, the anti-PD-L1 antibody is an immunoglobulin G1 lambda-1, anti-(human PD-L1) human monoclonal antibody. In an embodiment, the anti-PD-L1 antibody comprises the heavy and light chains of avelumab (MSB0010718C). In an embodiment, an anti-PD-L1 antibody comprises heavy and light chains having the sequences shown in SEQ ID NO:59 and SEQ ID NO:60, respectively, or antigen binding fragments, variants, or conjugates thereof. In an embodiment, an anti-PD-L1 antibody comprises heavy and light chains that are each at least 99% identical to the sequences shown in SEQ ID NO:59 and SEQ ID NO:60, respectively. In an embodiment, an anti-PD-L1 antibody comprises heavy and light chains that are each at least 98% identical to the sequences shown in SEQ ID NO:59 and SEQ ID NO:60, respectively. In an embodiment, an anti-PD-L1 antibody comprises heavy and light chains that are each at least 97% identical to the sequences shown in SEQ ID NO:59 and SEQ ID NO:60, respectively. In an embodiment, an anti-PD-L1 antibody comprises heavy and light chains that are each at least 96% identical to the sequences shown in SEQ ID NO:59 and SEQ ID NO:60, respectively. In an embodiment, an anti-PD-L1 antibody comprises heavy and light chains that are each at least 95% identical to the sequences shown in SEQ ID NO:59 and SEQ ID NO:60, respectively.

In an embodiment, the anti-PD-L1 antibody is an anti-PD-L1 biosimilar monoclonal antibody approved by drug regulatory authorities with reference to avelumab. In an embodiment, the biosimilar comprises an anti-PD-L1 antibody comprising an amino acid sequence which has at least 97% sequence identity, *e.g.,* 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is avelumab. In some embodiments, the one or more post-translational modifications are selected from one or more of: glycosylation, oxidation, deamidation, and truncation. In some embodiments, the biosimilar is an anti-PD-L1 antibody authorized or submitted for authorization, wherein the anti-PD-L1 antibody is provided in a formulation which differs from the formulations of a reference medicinal product or reference biological product, wherein the reference medicinal product or reference biological product is avelumab. The anti-PD-L1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is avelumab. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is avelumab.

In an embodiment, the anti-PD-L1 antibody $V_H$ region comprises the sequence given in SEQ ID NO:61 (corresponding to SEQ ID NO:24 in U.S. Patent Application Publication No. US 2014/0341917 A1), and the anti-PD-L1 antibody $V_L$ region comprises the sequence given in SEQ ID NO:62 (corresponding to SEQ ID NO:25 in U.S. Patent Application Publication No. US 2014/0341917 A1). In an embodiment, an anti-PD-L1 antibody comprises $V_H$ and $V_L$ regions that are each at least 99% identical to the sequences shown in SEQ ID NO:61 and SEQ ID NO:62, respectively. In an embodiment, an anti-PD-L1 antibody comprises $V_H$ and $V_L$ regions that are each at least 98% identical to the sequences shown in SEQ ID NO:61 and SEQ ID NO:62, respectively. In an embodiment, an anti-PD-L1 antibody comprises $V_H$ and $V_L$ regions that are each at least 97% identical to the sequences shown in SEQ ID NO:61 and SEQ ID NO:62, respectively. In an embodiment, an anti-PD-L1 antibody comprises $V_H$ and $V_L$ regions that are each at least 96% identical to the sequences shown in SEQ ID NO:61 and SEQ ID NO:62, respectively. In an embodiment, an anti-PD-L1 antibody comprises $V_H$ and $V_L$ regions that are each at least 95% identical to the sequences shown in SEQ ID NO:61 and SEQ ID NO:62, respectively.

In an embodiment, the anti-PD-L1 antibody comprises a heavy chain variable region ($V_H$) polypeptide that comprises a CDR1, CDR2, and CDR3 sequence, wherein the CDR1 sequence is given by SEQ ID NO:63 (corresponding

to SEQ ID NO:15 in U.S. Patent Application Publication No. US 2014/0341917 A1), the CDR2 sequence is given by SEQ ID NO:64 (corresponding to SEQ ID NO:16 in U.S. Patent Application Publication No. US 2014/0341917 A1), and the CDR3 sequence is given by SEQ ID NO:65 (corresponding to SEQ ID NO:17 in U.S. Patent Application Publication No. US 2014/0341917 A1), and the anti-PD-L1 antibody also comprises a light chain variable region ($V_L$) polypeptide that comprises a CDR1, CDR2, and CDR3 sequence wherein the CDR1 sequence is given by SEQ ID NO:66 (corresponding to SEQ ID NO:18 in U.S. Patent Application Publication No. US 2014/0341917 A1), the CDR2 sequence is given by SEQ ID NO:67 (corresponding to SEQ ID NO:19 in U.S. Patent Application Publication No. US 2014/0341917 A1), and the CDR3 sequence is given by SEQ ID NO:68 (corresponding to SEQ ID NO:20 in U.S. Patent Application Publication No. US 2014/0341917 A1).

In an embodiment, an anti-PD-L1 antibody comprises a heavy chain that comprises CDR1, CDR2 and CDR3 domains that are each at least 95% identical to the sequences shown in SEQ ID NO:63, SEQ ID NO:64, and SEQ ID NO:65, respectively. In an embodiment, an anti-PD-L1 antibody comprises a heavy chain that comprises CDR1, CDR2 and CDR3 domains that are each at least 90% identical to the sequences shown in SEQ ID NO:63, SEQ ID NO:64, and SEQ ID NO:65, respectively. In an embodiment, an anti-PD-L1 antibody comprises a heavy chain that comprises CDR1, CDR2 and CDR3 domains that are each at least 85% identical to the sequences shown in SEQ ID NO:63, SEQ ID NO:64, and SEQ ID NO:65, respectively. In an embodiment, an anti-PD-L1 antibody comprises a heavy chain that comprises CDR1, CDR2 and CDR3 domains that are each at least 80% identical to the sequences shown in SEQ ID NO:63, SEQ ID NO:64, and SEQ ID NO:65, respectively. In another embodiment, the antibody competes for binding with, and/or binds to the same epitope on PD-L1 as the aforementioned antibodies.

In an embodiment, an anti-PD-L1 antibody comprises a light chain that comprises CDR1, CDR2 and CDR3 domains that are each at least 95% identical to the sequences shown in SEQ ID NO:66, SEQ ID NO:67, and SEQ ID NO:68, respectively. In an embodiment, an anti-PD-L1 antibody comprises a light chain that comprises CDR1, CDR2 and CDR3 domains that are each at least 90% identical to the sequences shown in SEQ ID NO:66, SEQ ID NO:67, and SEQ ID NO:68, respectively. In an embodiment, an anti-PD-L1 antibody comprises a light chain that comprises CDR1, CDR2 and CDR3 domains that are each at least 85% identical to the sequences shown in SEQ ID NO:66, SEQ ID NO:67, and SEQ ID NO:68, respectively. In an embodiment, an anti-PD-L1 antibody comprises a light chain that comprises CDR1, CDR2 and CDR3 domains that are each at least 80% identical to the sequences shown in SEQ ID NO:66, SEQ ID NO:67, and SEQ ID NO:68, respectively. In another embodiment, the antibody competes for binding with, and/or binds to the same epitope on PD-L1 as the aforementioned antibodies.

In an embodiment, anti-PD-L1 antibodies and other PD-L1 inhibitors include those described in U.S. Patent Application Publication No. US 2014/0341917 A1. In another embodiment, antibodies that compete with any of these antibodies for binding to PD-L1 are also included.

In an embodiment, the anti-PD-L1 antibody is MDX-1105, also known as BMS-935559, which is disclosed in U.S. Patent No. US 7,943,743 B2. In an embodiment, the anti-PD-L1 antibody is selected from the anti-PD-L1 antibodies disclosed in U.S. Patent No. US 7,943,743 B2.

In an embodiment, the anti-PD-L1 antibody is a commercially-available monoclonal antibody, such as INVIVOMAB anti-m-PD-L1 clone 10F.9G2 (Catalog # BE0101, Bio X Cell, Inc., West Lebanon, NH, USA). In an embodiment, the anti-PD-L1 antibody is a commercially-available monoclonal antibody, such as AFFYMETRIX EBIOSCIENCE (MIH1). A number of commercially-available anti-PD-L1 antibodies are known to one of ordinary skill in the art.

In an embodiment, the anti-PD-L2 antibody is a commercially-available monoclonal antibody, such as BIOLEGEND 24F.10C12 Mouse IgG2a, κ isotype (catalog # 329602 Biolegend, Inc., San Diego, CA), SIGMA anti-PD-L2 antibody (catalog # SAB3500395, Sigma-Aldrich Co., St. Louis, MO), or other commercially-available anti-PD-L2 antibodies known to one of ordinary skill in the art.

Monoclonal antibodies that inhibit PD-L1 and/or PD-L2 can be prepared by procedures known to those of ordinary knowledge and skill in the art, *e.g.*, by injecting test subjects with PD-L1 or PD-L2 antigen and then isolating hybridomas expressing antibodies having the desired sequence or functional characteristics. DNA encoding the monoclonal antibodies is readily isolated and sequenced using conventional procedures (*e.g.*, by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, myeloma cells, or other suitable cells that do not otherwise produce immunoglobulin protein, to obtain the synthesis of monoclonal antibodies in the recombinant host cells. The details of recombinant production of specific antibodies may be found in the references cited in the foregoing. Monoclonal antibodies that inhibit PD-1 can be prepared by standard molecular biology methods using the sequences provided herein by reverse translation and insertion into appropriate DNA or RNA vectors.

The anti-PD-L1 antibody sequences referenced in some of the foregoing embodiments are summarized in Table 3.

TABLE 3. Anti-PD-L1 antibody amino acid sequences.

| Identifier | Sequence (One-Letter Amino Acid Symbols) |
|---|---|
| SEQ ID NO:39 durvalumab (MEDI4736) heavy chain | EVQLVESGGG LVQPGGSLRL SCAASGFTFS RYWMSWVRQA PGKGLEWVAN IKQDGSEKYY 60<br>VDSVKGRFTI SRDNAKNSLY LQMNSLRAED TAVYYCAREG GWFGELAFDY WGQGTLVTVS 120<br>SASTKGPSVF PLAPSSKSTS GGTAALGCLV KDYFPEPVTV SWNSGALTSG VHTFPAVLQS 180<br>SGLYSLSSVV TVPSSSLGTQ TYICNVNHKP SNTKVDKRVE PKSCDKTHTC PPCPAPEFEG 240<br>GPSVFLFPPK PKDTLMISRT PEVTCVVVDV SHEDPEVKFN WYVDGVEVHN AKTKPREEQY 300<br>NSTYRVVSVL TVLHQDWLNG KEYKCKVSNK ALPASIEKTI SKAKGQPREP QVYTLPPSRE 360<br>EMTKNQVSLT CLVKGFYPSD IAVEWESNGQ PENNYKTTPP VLDSDGSFFL YSKLTVDKSR 420<br>WQQGNVFSCS VMHEALHNHY TQKSLSLSPG K 451 |
| SEQ ID NO:40 durvalumab (MEDI47 36) light chain | EVQLVESGGG LVQPGGSLRL SCAASGFTFS RYWMSWVRQA PGKGLEWVAN EIVLTQSPGT 60<br>LSLSPGERAT LSCRASQRVS SSYLAWYQQK PGQAPRLLIY DASSRATGIP DRFSGSGSGT 120<br>DFTLTISRLE PEDFAVYYCQ QYGSLPWTFG QGTKVEIKRT VAAPSVFIFP PSDEQLKSGT 180<br>ASVVCLLNNF YPREAKVQWK VDNALQSGNS QESVTEQDSK DSTYSLSSTL TLSKADYEKH 240<br>KVYACEVTHQ GLSSPVTKSF NRGEC 265 |
| SEQ ID NO:41 durvalumab variable heavy chain | EVQLVESGGG LVQPGGSLRL SCAASGFTFS RYWMSWVRQA PGKGLEWVAN IKQDGSEKYY 60<br>VDSVKGRFTI SRDNAKNSLY LQMNSLRAED TAVYYCAREG GWFGELAFDY WGQGTLVTVS 120<br>S 121 |
| SEQ ID NO:42 durvalumab variable light chain | EIVLTQSPGT LSLSPGERAT LSCRASQRVS SSYLAWYQQK PGQAPRLLIY DASSRATGIP 60<br>DRFSGSGSGT DFTLTISRLE PEDFAVYYCQ QYGSLPWTFG QGTKVEIK 108 |
| SEQ ID NO:43 durvalumab heavy chain CDR1 | RYWMS 5 |
| SEQ ID NO:44 durvalumab heavy chain CDR2 | NIKQDGSEKY YVDSVKG 17 |
| SEQ ID NO:45 durvalumab heavy chain CDR3 | EGGWFGELAF DY 12 |
| SEQ ID NO:46 durvalumab light chain CDR1 | RASQRVSSSY LA 12 |
| SEQ ID NO:47 durvalumab light chain CDR2 | DASSRAT 7 |
| SEQ ID NO:48 durvalumab light chain CDR3 | QQYGSLPWT 9 |
| SEQ ID NO:49 atezolizumab (MPDL3280A)heavy chain | EVQLVESGGG LVQPGGSLRL SCAASGFTFS DSWIHWVRQA PGKGLEWVAW ISPYGGSTYY 60<br>ADSVKGRFTI SADTSKNTAY LQMNSLRAED TAVYYCARRH WPGGFDYWGQ GTLVTVSSAS 120<br>TKGPSVFPLA PSSKSTSGGT AALGCLVKDY FPEPVTVSWN SGALTSGVHT FPAVLQSSGL 180<br>YSLSSVVTVP SSSLGTQTYI CNVNHKPSNT KVDKKVEPKS CDKTHTCPPC PAPELLGGPS 240<br>VFLFPPKPKD TLMISRTPEV TCVVVDVSHE DPEVKFNWYV DGVEVHNAKT KPREEQYAST 300<br>YRVVSVLTVL HQDWLNGKEY KCKVSNKALP APIEKTISKA KGQPREPQVY TLPPSREEMT 360<br>KNQVSLTCLV KGFYPSDIAV EWESNGQPEN NYKTTPPVLD SDGSFFLYSK LTVDKSRWQQ 420<br>GNVFSCSVMH EALHNHYTQK SLSLSPGK 448 |
| SEQ ID NO:50 atezolizumab (MPDL3280A) light chain | DIQMTQSPSS LSASVGDRVT ITCRASQDVS TAVAWYQQKP GKAPKLLIYS ASFLYSGVPS 60<br>RFSGSGSGTD FTLTISSLQP EDFATYYCQQ YLYHPATFGQ GTKVEIKRTV AAPSVFIFPP 120<br>SDEQLKSGTA SVVCLLNNFY PREAKVQWKV DNALQSGNSQ ESVTEQDSKD STYSLSSTLT 180<br>LSKADYEKHK VYACEVTHQG LSSPVTKSFN RGEC 214 |
| SEQ ID NO:51 atezolizumab variable heavy chain | EVQLVESGGG LVQPGGSLRL SCAASGFTFS DSWIHWVRQA PGKGLEWVAW ISPYGGSTYY 60<br>ADSVKGRFTI SADTSKNTAY LQMNSLRAED TAVYYCARRH WPGGFDYWGQ GTLVTVSA 118 |

(continued)

| Identifier | Sequence (One-Letter Amino Acid Symbols) |
|---|---|
| SEQ ID NO:52 atezolizumab variable light chain | DIQMTQSPSS LSASVGDRVT ITCRASQDVS TAVAWYQQKP GKAPKLLIYS ASFLYSGVPS 60<br>RFSGSGSGTD FTLTISSLQP EDFATYYCQQ YLYHPATFGQ GTKVEIKR 108 |
| SEQ ID NO:53 atezolizumab heavy chain CDR1 | GFTFSXSWIH 10 |
| SEQ ID NO:54 atezolizumab heavy chain CDR2 | AWIXPYGGSX YYADSVKG 18 |
| SEQ ID NO:55 atezolizumab heavy chain CDR3 | RHWPGGFDY 9 |
| SEQ ID NO:56 atezolizumab light chain CDR1 | RASQXXXTXX A 11 |
| SEQ ID NO:57 atezolizumab light chain CDR2 | SASXLXS 7 |
| SEQ ID NO:58 atezolizumab light chain CDR3 | QQXXXXPXT 9 |
| SEQ ID NO:59 avelumab (MSB0010718C) heavy chain | EVQLLESGGG LVQPGGSLRL SCAASGFTFS SYIMMWVRQA PGKGLEWVSS IYPSGGITFY 60<br>ADTVKGRFTI SRDNSKNTLY LQMNSLRAED TAVYYCARIK LGTVTTVDYW GQGTLVTVSS 120<br>ASTKGPSVFP LAPSSKSTSG GTAALGCLVK DYFPEPVTVS WNSGALTSGV HTFPAVLQSS 180<br>GLYSLSSVVT VPSSSLGTQT YICNVNHKPS NTKVDKKVEP KSCDKTHTCP PCPAPELLGG 240<br>PSVFLFPPKP KDTLMISRTP EVTCVVVDVS HEDPEVKFNW YVDGVEVHNA KTKPREEQYN 300<br>STYRVVSVLT VLHQDWLNGK EYKCKVSNKA LPAPIEKTIS KAKGQPREPQ VYTLPPSRDE 360<br>LTKNQVSLTC LVKGFYPSDI AVEWESNGQP ENNYKTTPPV LDSDGSFFLY SKLTVDKSRW 420<br>QQGNVFSCSV MHEALHNHYT QKSLSLSPGK 450 |
| SEQ ID NO:60 avelumab (MSB0010718C) light chain | QSALTQPASV SGSPGQSITI SCTGTSSDVG GYNYVSWYQQ HPGKAPKLMI YDVSNRPSGV 60<br>SNRFSGSKSG NTASLTISGL QAEDEADYYC SSYTSSSTRV FGTGTKVTVL GQPKANPTVT 120<br>LFPPSSEELQ ANKATLVCLI SDFYPGAVTV AWKADGSPVK AGVETTKPSK QSNNKYAASS 180<br>YLSLTPEQWK SHRSYSCQVT HEGSTVEKTV APTECS 216 |
| SEQ ID NO:61 avelumab variable heavy chain | EVQLLESGGG LVQPGGSLRL SCAASGFTFS SYIMMWVRQA PGKGLEWVSS IYPSGGITFY 60<br>ADTVKGRFTI SRDNSKNTLY LQMNSLRAED TAVYYCARIK LGTVTTVDYW GQGTLVTVSS 120 |
| SEQ ID NO:62 avelumab variable light chain | QSALTQPASV SGSPGQSITI SCTGTSSDVG GYNYVSWYQQ HPGKAPKLMI YDVSNRPSGV 60<br>SNRFSGSKSG NTASLTISGL QAEDEADYYC SSYTSSSTRV FGTGTKVTVL 110 |
| SEQ ID NO:63 avelumab heavy chain CDR1 | SYIMM 5 |
| SEQ ID NO:64 avelumab heavy chain CDR2 | SIYPSGGITF YADTVKG 17 |
| SEQ ID NO:65 avelumab heavy chain CDR3 | IKLGTVTTVD Y 11 |

(continued)

| Identifier | Sequence (One-Letter Amino Acid Symbols) |
|---|---|
| SEQ ID NO:66 avelumab light chain CDR1 | TGTSSDVGGY NYVS 14 |
| SEQ ID NO:67 avelumab light chain CDR2 | DVSNRPS 7 |
| SEQ ID NO:68 avelumab light chain CDR3 | SSYTSSSTRV 10 |

The preparation, properties, and uses of suitable PD-1 and PD-L1 inhibitors are described in, *e.g.*, U.S. Patent No. 8,008,449 or U.S. Patent Application Publication Nos. 2009/0217401 A1 or 2013/0133091 A1; U.S. Patent No. 8,354,509 and U.S. Patent Application Publication Nos. US 2010/0266617 A1, US 2013/0108651 A1, and US 2013/0109843 A2; U.S. Patent Nos. 8,287,856, 8,580,247, and 8,168,757 and U.S. Patent Application Publication Nos. US 2009/0028857 A1, US 2010/0285013 A1, US 2013/0022600 A1, and US 2011/0008369 A1; U.S. Patent No. 8,779,108 or U.S. Patent Application Publication No. US 2013/0034559 A1; U.S. Patent No. 8,217,149 and U.S. Patent Application Publication Nos. US 2010/0203056 A1, US 2013/0045200 A1, US 2013/0045201 A1, US 2013/0045202 A1, or US 2014/0065135 A1; and U.S. Patent Application Publication No. US 2014/0341917 A1.

In any of the foregoing embodiments, the PD-1 and/or PD-L1 inhibitors or combinations thereof may be administered before, concurrently, or after administration of the methotrexate and BTK inhibitor of formula (2).

In an embodiment, the PD-1 or PD-L1 inhibitor is an anti-PD-1 or anti-PD-L1 biosimilar monoclonal antibody approved by drug regulatory authorities with reference to inhibitor selected from the group consisting of nivolumab, pembrolizumab, pidilizumab, durvalumab, atezolizumab, or avelumab. In an embodiment, the biosimilar comprises an anti-PD-1 or anti-PD-L1 antibody comprising an amino acid sequence which has at least 97% sequence identity, *e.g.*, 97%, 98%, 99% or 100% sequence identity, to the amino acid sequence of a reference medicinal product or reference biological product and which comprises one or more post-translational modifications as compared to the reference medicinal product or reference biological product, wherein the reference medicinal product or reference biological product is nivolumab, pembrolizumab, pidilizumab, durvalumab, atezolizumab, or avelumab. In some embodiments, the one or more post-translational modifications are selected from one or more of: glycosylation, oxidation, deamidation, and truncation. In some embodiments, the biosimilar is an anti-PD1 or anti-PD-L1 antibody authorized or submitted for authorization, wherein the anti-PD1 or anti-PD-L1 antibody is provided in a formulation which differs from the formulations of a reference medicinal product or reference biological product, wherein the reference medicinal product or reference biological product is nivolumab, pembrolizumab, pidilizumab, durvalumab, atezolizumab, or avelumab. The anti-PD1 or anti-PD-L1 antibody may be authorized by a drug regulatory authority such as the U.S. FDA and/or the European Union's EMA. In some embodiments, the biosimilar is provided as a composition which further comprises one or more excipients, wherein the one or more excipients are the same or different to the excipients comprised in a reference medicinal product or reference biological product, wherein the reference medicinal product or reference biological product is nivolumab, pembrolizumab, pidilizumab, durvalumab, atezolizumab, or avelumab. In some embodiments, the biosimilar comprises one or more excipients selected from tris-hydrochloride, sodium chloride, mannitol, pentetic acid, polysorbate 80, sodium hydroxide, and hydrochloric acid.

In an embodiment, the invention provides a combination or composition for use in rheumatoid arthritis in a human comprising the step of administering to said human a BTK inhibitor of formula (2), or a pharmaceutically acceptable salt thereof, and methotrexate and further comprising the step of administering an PD-1 or PD-L1 inhibitor, or an antigen-binding fragment, derivative, conjugate, variant, radioisotope-labeled complex, or biosimilar thereof. In an embodiment, the PD-1 or PD-L1 inhibitor is selected from the group consisting of nivolumab, pembrolizumab, pidilizumab, durvalumab, atezolizumab, avelumab, and antigen-binding fragments, variants, conjugates, or biosimilars thereof.

While preferred embodiments of the invention are shown and described herein, such embodiments are provided by way of example only and are not intended to otherwise limit the scope of the invention. Various alternatives to the described embodiments of the invention may be employed in practicing the invention.

EXAMPLES

The embodiments encompassed herein are now described with reference to the following examples. These

examples are provided for the purpose of illustration only and the disclosure encompassed herein should in no way be construed as being limited to these examples, but rather should be construed to encompass any and all variations which become evident as a result of the teachings provided herein.

Example 1 - Synergistic Combinations of BTK Inhibitors and Antifolates

The *in vivo* use of the BTK inhibitor of Formula (2) and methotrexate ("MTX") was tested in a mouse model to determine the combination's effectiveness to inhibit inflammation, cartilage destruction, pannus formation and bone resorption associated with type II collagen arthritis. DBA/1lacJ Mice mice were anesthetized with Isoflurane and given intradermal injections of a total of 100 μL of 2 mg/mL of Type II collagen in 2.5 mg/mL Freund's complete adjuvant at the base of the tail on study days zero and 21. MTX treatments were initiated on day 18 and the BTK inhibitor of Formula (2) treatments were initiated on day 28 of the vehicle treated animals (arthritis score between 0.5 and 1) and continued for the remainder of the study. The BTK inhibitor of Formula (2) vehicle treatments were dosed 6 hours post MTX dosing.

Formula (2) and MTX were formulated with 0.5% w/v Methylcellulose; 4000 cps (DOW, Methocel A4M or Equivalent); 0.1% v/v Polysorbate 80 (Tween 80) and DI or RO water. Formula (2) was formulated at concentrations of 0.1 mg/mL and 0.5 mg/mL and MTX was formulated at 0.03 mg/mL, 0.05 mg/mL, 0.1 mg/ml and 0.5 mg/mL. MTX was administered PO gavage at doses of QD 0.3 mg/kg and QD 0.5 mg/kg in the mornings. Six hours later, Formula (2) was administered PO gavage at doses of QD 1 mg/kg and QD 5 mg/kg. All mice survived through study termination.

Daily clinical scores were based on daily paw scores, an area under the curve (AUC) calculation of the paw scoring over time, and the hind paw, ankle, and optionally knees histopathology, body weight measurements and body weight change from the start of dosing will be evaluated for signs of toxic effects of the test article(s) and/or vehicle(s). The daily clinical scores were determined for each paw on study days 18-43. The scoring was based on the following criteria: 0 = normal; 1 = one hind or fore paw joint affected or minimal diffuse erythema and swelling; 2 = two hind or fore paw joints affected or mild diffuse erythema and swelling; 3 = three hind or fore paw joints affected or moderate diffuse erythema and swelling; 4 = marked diffuse erythema and swelling, or four digit joints affected; and 5 = severe diffuse erythema and severe swelling entire paw, unable to flex digits.

Clinical scores were given for each of the paws (right front, left front, right rear, left rear) on Study Days 18-43. Clinical data for paw scores (means for animal) were then analyzed by determining the area under the dosing curve (AUC). AUC was calculated from MTX dosing initiation (Day 18) through study termination (Day 43) and from the BTK inhibitor of Formula (2) dosing initiation (Day 28) through study termination. For calculation of AUC, the daily mean scores for each mouse were entered into Microsoft Excel and the area between the treatment days and the final day was computed. Means for each group were determined. A one-way analysis of variance (1-way ANOVA) along with a Dunnett's or Sidak's post-hoc analysis or a Kruskal-Wallis test (non-parametric) along with a Dunn's posthoc analysis was used to the evaluate data collected in this study. A Student's two-tailed t-test was used to compare normal versus disease controls for model validation. Unless indicated, Bolder BioPATH, Inc. performs statistical analysis on raw (untransformed) data only. Statistical tests make certain assumptions regarding normality and homogeneity of variance, and further analysis may be required if testing resulted in violations of these assumptions. P values were rounded to three decimal places. Significance for all tests was set at $p < 0.050$. Statistical analysis was performed using Prism 6.0d softwareGraphPad. Percent inhibition is calculated using the following formula:

$$\% \text{ Change} = B/A \times 100 A = \text{Mean Normal} - \text{Mean Disease Control where } B = \text{Mean Treated} - \text{Mean Disease Control}$$

On study day 43, mice were euthanized and necropsy specimens were obtained. After terminal bleeds, animals were euthanized by cervical dislocation. Fore paws, hind paws, and knees were harvested and placed in 10% neutral buffered formalin (NBF) for microscopy. Spleens were harvested from a subset of mice and processed for splenocytes.

After 1-2 days in fixative and 4-5 days in 5% formic acid for decalcification, tissues were trimmed, and processed for paraffin embedding. Paws were embedded in paraffin in the frontal plane and knees were embedded with the patella facing down. Ankles, if left attached to the hind paw, were also embedded in the frontal plane but in some instances were detached and sectioned in the sagittal plane for special purposes. Left/right pairs were typically embedded in the same block. Sections were then cut and stained with toluidine blue.

When scoring paws or ankles from mice with lesions of type II collagen arthritis, severity of changes as well as number of individual joints affected was considered. When only one to three joints of the paws or ankles out of a possibility of numerous metacarpal/ metatarsal/ digit or tarsal/tibiotarsal joints was affected, an arbitrary assignment of a maximum score of 0.5, 1, 2 or 3 for parameters below was given depending on severity of changes. If more than three joints were involved, the criteria below were applied to the most severely affected/majority of joints. In the case of knees, severity of changes in medial and lateral, as well as femoropatellar spaces, were considered.

The following parameters were scored according to the indicated criteria: Inflammation Score, Pannus Score, Cartilage Damage Score, Bone Resorption Score, and Periosteal New Bone Formation Score and Measurements. Unless otherwise indicated, criteria apply to both paws and knees. Mean values for each parameter are determined separately for the paws, knees, and the entire animal (if applicable).

The Inflammation Score was determined based on the following criteria for Paw Score and Knee Score.

The Paw Score was classified based on the following criteria: 0 = Normal; 0.5 = Very minimal, affects only 1 joint or minimal multifocal periarticular infiltration of inflammatory cells; 1 = Minimal infiltration of inflammatory cells in synovium and periarticular tissue of affected joints; 2 = Mild infiltration of inflammatory cells. If referring to paws, generally restricted to affected joints (1-3 affected); 3 = Moderate infiltration with moderate edema. If referring to paws, restricted to affected joints, generally 3-4 joints and the wrist or ankle; 4 = Marked infiltration affecting most areas with marked edema, 1 or 2 unaffected joints may be present; 5 = Severe diffuse infiltration with severe edema affecting all joints (to some extent) and periarticular tissues.

The Knee Score was classified based on the following criteria: 0 = Normal; 0.5 = Very minimal, affects only one area of the synovium or minimal multifocal periarticular infiltration of inflammatory cells; 1 = Minimal infiltration of inflammatory cells in synovium and periarticular tissue of affected synovial areas; 2 = Mild diffuse infiltration of inflammatory cells; 3 = Moderate diffuse infiltration of inflammatory cells; 4 = Marked diffuse infiltration of inflammatory cells; 5 = Severe diffuse infiltration of inflammatory cells.

The inflammatory infiltrate in mice and rats with type II collagen arthritis consists of neutrophils and macrophages with smaller numbers of lymphocytes when the lesions are in the acute to subacute phase. Tissue edema and neutrophil exudates within the joint space are common in the acute to subacute phase. As the inflammation progresses to chronic, mononuclear inflammatory cells (monocytes, lymphocytes) predominate and fibroblast proliferation, often with deposition of metachromatic matrix, occurs in synovium and periarticular tissue. Exudate is less common in the joint space. Unless indicated in the comments area, the inflammation type is acute to subacute.

DBA mice have an increased incidence of dactylitis and onchyoperiostitis affecting the nail bed and distal phalynx as reported in Lories, et al., Ann. Rheum. Dis. 2004, 63, 595-598. These lesions are recorded but were not included in the inflammation score.

The Pannus Score was based on the following criteria: 0 = Normal; 0.5 = Very minimal, If paws, affects only one joint at marginal zone; 1 = Minimal infiltration of pannus in cartilage and subchondral bone, marginal zones. If paws, affects two or more joints; 2 = Mild infiltration with marginal zone destruction of hard tissue in affected joints; 3 = Moderate infiltration with moderate hard tissue destruction in affected joints; 4 = Marked infiltration with marked destruction of joint architecture, affecting most joints; 5 = Severe infiltration associated with total or near total destruction of joint architecture, affects all joints.

The Cartilage Damage Score was based on the following criteria: 0 = Normal; 0.5 = Very minimal = Affects marginal zones only of one to several areas (knees) or joints (paws); 1 = Minimal = Generally minimal to mild loss of toluidine blue staining (proteoglycan) with no obvious chondrocyte loss or collagen disruption in affected joints/areas; 2 = Mild = Generally mild loss of toluidine blue staining (proteoglycan) with focal areas of chondrocyte loss and/or collagen disruption in some affected joints/areas. Paws may have one or two digit joints with near total to total loss of cartilage; 3 = Moderate = Generally moderate loss of toluidine blue staining (proteoglycan) with multifocal chondrocyte loss and/or collagen disruption in affected joints/areas. Paws may have three or four joints with near total or total loss. In the knee, some matrix remains on any affected surface with areas of severe matrix loss; 4 = Marked = Marked loss of toluidine blue staining (proteoglycan) with multifocal marked (depth to deep zone or tidemark) chondrocyte loss and/or collagen disruption in most joints with a few unaffected or mildly affected. In the knee, one surface with total to near total cartilage loss; 5 = Severe = Severe diffuse loss of toluidine blue staining (proteoglycan) with severe (depth to tide mark) chondrocyte loss and/or collagen disruption in most or all joints. In the knee, two or more surfaces with total to near total cartilage loss.

The Bone Resorption Score was determined based on the following criteria for Paw Score and Knee score.

The Paw Score was based on the following criteria: 0 = Normal; 0.5 = Very Minimal = Affects only 1 joint or is restricted to cortical/subperiosteal areas; 1 = Minimal = Small/few areas of definite resorption, not readily apparent on low magnification, rare osteoclasts in affected joints, restricted to marginal zones; 2 = Mild = More numerous/larger areas of resorption, osteoclasts more numerous in affected joints, mainly in marginal zones but some extension to load bearing areas, may have endosteal proliferation in areas of resorption; 3 = Moderate = Obvious resorption of medullary trabecular and cortical bone without widespread full thickness defects in cortex, loss of medullary trabeculae, lesion apparent on low magnification, osteoclasts more numerous in affected joints, may have endosteal proliferation in areas of resorption; 4 = Marked = Full thickness defects in cortical bone, often with distortion of profile of remaining cortical surface, marked loss of medullary bone, numerous osteoclasts, affects most joints, may have endosteal proliferation in areas of resorption; 5 = Severe = Full thickness defects in cortical bone and destruction of joint architecture of all joints, may have endosteal proliferation in areas of resorption.

The Knee Score was based on the following criteria: 0 = Normal; 0.5 = Very Minimal = Minimal resorption affects only marginal zones; 1 = Minimal = Small areas of resorption, not readily apparent on low magnification, approximately 1-10% of total joint width of subchondral bone affected; 2 = Mild = More numerous areas of resorption, definite loss of subchondral bone, approximately 11-25% of total joint width of subchondral bone affected; 3 = Moderate = Obvious resorption of subchondral bone, approximately 26-50% of total joint width of subchondral bone affected; 4 = Marked = Obvious resorption of subchondral bone, approximately 51-75% of total joint width of subchondral bone affected; 5 = Severe = Distortion of entire joint due to destruction, approximately 76-100% of total joint width of subchondral bone affected.

Periosteal new bone formation score and measurements were also assessed. Studies that go beyond the acute inflammatory stage often show varying degrees of periosteal new bone formation. The width of the largest area of new bone formation in a non-tangential section ("Periosteal New Bone Formation Score") is measured and used to determine a score based on the following criteria for Paw Score and Knee Score.

The Paw Score was based on the following criteria: 0 = Normal, no periosteal proliferation; 0.5 = Minimal focal or multifocal early proliferation, measures less than 40 μm width (<1 unit on 25x); 1 = Minimal multifocal early proliferation, measures 40-80 μm width (1-2 units on 25x); 2 = Mild multifocal to diffuse with widths that measure approximately 120-200 μm (3-5 units on 25x); 3 = Moderate diffuse with widths that measure 240-280 μm (6-7 units on 25x); 4 = Marked diffuse with widths that measure 320-400 μm (8-10 units on 25x); 5 = Severe, diffuse with widths that measure greater than 400μm (>10 units on 25x).

The Knee Score was based on the following criteria: 0 = Normal, no periosteal proliferation; 0.5 = Minimal focal or multifocal early proliferation, measures 40 μm width or less (1-2 units on 50x); 1 = Minimal multifocal early proliferation, measures approximately 40-80μm width (3-4 units on 50x); 2 = Mild multifocal to diffuse with widths that measures approximately 100-140 μm (5-7 units on 50x); 3 = Moderate diffuse with widths that measure approximately 160-220 μm (8-11 units on 50x); 4 = Marked diffuse with widths that measure approximately 240-300 μm (12-15 units on 50x); 5 = Severe, diffuse with widths that measure greater than 300 μm (>15 units on 50x).

A sum of the five histopathology scores was also calculated for each joint.

Live phase and necropsy parameters were determined for various clinical and histopathology data including (i) change in body weight during days 18-43; (ii) clinical arthritis score AUC during days 18-43; (iii) clinical arthritis score AUC during days 28-43; (iv) percent incidence; and (v) histopathology summed scores for all joints. The data is summarized in Table 4. A detailed discussion of each data set is given below.

TABLE 4. Summary of Clinical and Histopathology Data

| Group | Treatment | Change in Body Weight (g) Day 18-43 | Clinical Arthritis Score AUC Day 18-43 | Clinical Arthritis Score AUC Day 28-43 | Percent Incidence | Histopathology Summed Scores (All Joints) |
|---|---|---|---|---|---|---|
| 1 | Normal + Vehicle, PO, QD | †0.67 (0.20) | †0.00 (0.00) | †0.00 (0.00) | 0% | †0.04 (0.04) |
| 2 | MTX Vehicle PO, QD (d18-43) + BTK inhibitor of formula (2) Vehicle PO, QD (d28-43) | -0.94 (0.26) | 71.73 (4.38) | 65.70 (3.73) | 100% | 15.02 (1.32) |
| 3 | MTX Vehicle PO, QD (d18-43) + BTK inhibitor of formula (2) (1 mg/kg) PO, QD (d28-43) | -0.19 (0.18) | 61.41 (3.58) | 54.81 (3.58) | 100% | 11.03 (1.01) |
| 4 | MTX Vehicle PO, QD (d18-43) + BTK inhibitor of formula (2) (5 mg/kg) PO, QD (d28-43) | *0.12 (0.28) | *27.30 (6.04) | *21.27 (5.39) | 42% | *4.15 (1.01) |
| 5 | MTX (0.3 mg/kg) PO, QD (d18-43) + BTK inhibitor of formula (2) Vehicle PO, QD (d28-43) | -1.16 (0.48) | 51.72 (5.38) | 48.82 (4.96) | 100% | 10.24 (1.66) |

(continued)

| Group | Treatment | Change in Body Weight (g) Day 18-43 | Clinical Arthritis Score AUC Day 18-43 | Clinical Arthritis Score AUC Day 28-43 | Percent Incidence | Histopathology Summed Scores (All Joints) |
|---|---|---|---|---|---|---|
| 6 | MTX (0.5 mg/kg), PO, QD (d18-43) + BTK inhibitor of formula (2) Vehicle PO, QD (d28-43) | -0.83 (0.19) | 46.79 (5.73) | 45.51 (5.56) | 100% | 8.46 (1.36) |
| 7 | MTX (0.3 mg/kg) PO, QD (d18-43) + BTK inhibitor of formula (2) (1 mg/kg) PO, QD (d28-43) | -0.17 (0.21) | *‡33.82 (6.59) | *30.84 (5.88) | 83% | *5.37 (1.35) |
| 8 | MTX (0.5 mg/kg) PO, QD | -0.31 | *‡29.04 | *‡27.59 | 75% | *‡3.88 (1.16) |
| | (d18-43) + BTK inhibitor of formula (2) (1 mg/kg) PO, QD (d28-43) | (0.30) | (6.24) | (5.95) | | |
| 9 | MTX (0.3 mg/kg) PO, QD (d18-43) + BTK inhibitor of formula (2) (5 mg/kg) PO, QD (d28-43) | -0.39 (0.16) | *§15.54 (3.26) | *§12.61 (3.00) | 42% | *§1.39 (0.40) |
| 10 | MTX (0.5 mg/kg) PO, QD (d18-43) + BTK inhibitor of formula (2) (5 mg/kg) PO, QD (d28-43) | -0.21 (0.16) | *§9.74 (3.61) | *§8.16 (3.22) | 17% | *§1.77 (0.68) |
| (SE) = Standard error displayed in parenthesis, AUC = Area Under the Curve<br>*$p$ < 0.05 ANOVA (with Dunnett's post-hoc test) or K-W test (with Dunn's post-hoc test) vs. MTX vehicle + BTK inhibitor of formula (2) vehicle.<br>†$p$ < 0.05 Student's *t*-test vs. MTX Vehicle + BTK inhibitor of formula (2) vehicle.<br>‡$p$ < 0.05 ANOVA (with Sidak's post-hoc test) or K-W test (with Dunn's post-hoc test) vs. MTX vehicle + BTK inhibitor of formula (2) (same dose).<br>§$p$ < 0.05 ANOVA (with Sidak's post-hoc test) or K-W test (with Dunn's post-hoc test) vs. MTX (same dose) + BTK inhibitor of formula (2) vehicle | | | | | | |

Vehicle control mice had body weight loss (measured as percent change from baseline) that peaked at -16.47% on Study Day 30. Disease-induced body weight loss was significantly inhibited as compared to vehicle controls on Day 36 in mice treated with 1 mg/kg the BTK inhibitor of Formula (2), on Days 32-38 and 43 in mice treated with 5 mg/kg the BTK inhibitor of Formula (2), on Days 28-40 in mice treated with 0.3 mg/kg MTX + 1 mg/kg the BTK inhibitor of Formula (2), on Days 26-40 in mice treated with 0.5 mg/kg MTX + 1 mg/kg the BTK inhibitor of Formula (2), on Days 28-42 in mice treated with 0.3 mg/kg MTX + 5 mg/kg the BTK inhibitor of Formula (2), and on Days 30-42 in mice treated with 0.5 mg/kg MTX + 5 mg/kg the BTK inhibitor of Formula (2). Body weight loss was significantly inhibited as compared to the BTK inhibitor of Formula (2) treatment alone on Days 26-32 in mice treated with MTX (0.3 or 0.5 mg/kg) + 1 mg/kg the BTK inhibitor of Formula (2) and on Days 28-30 in mice treated with 0.3 mg/kg MTX + 5 mg/kg the BTK inhibitor of Formula (2). Body weight loss was significantly inhibited as compared to MTX treatment alone on Days 32-42 in mice treated with 0.3 mg/kg MTX + 1 mg/kg the BTK inhibitor of Formula (2), on Days 30-34 in mice treated with 0.5 mg/kg MTX + 1 mg/kg the BTK inhibitor of Formula (2), on Days 30-42 in mice treated with 0.3 mg/kg MTX + 5 mg/kg the BTK inhibitor of Formula (2), and on Days 32-38 in mice treated with 0.5 mg/kg MTX + 5 mg/kg the BTK inhibitor of Formula (2).

At study termination, vehicle control mice had mean absolute body weight loss of -0.94 g. Absolute body weight loss was significantly (66%) inhibited in mice treated with 5 mg/kg BTK inhibitor of Formula (2) alone as compared to vehicle controls.

Daily clinical arthritis scores differed significantly from vehicle controls over time in mice treated with 5 mg/kg

the BTK inhibitor of Formula (2) or 0.5 mg/kg MTX alone and in all combination therapy groups. Clinical arthritis scores were significantly reduced on Study Days 30-43 in mice treated with 5 mg/kg the BTK inhibitor of Formula (2), on Days 25-29 in mice treated with 0.5 mg/kg MTX, on Days 28-43 in mice treated with 0.3 mg/kg MTX + the BTK inhibitor of Formula (2) (1 or 5 mg/kg), and on Days 25-43 in mice treated with 0.5 mg/kg MTX + the BTK inhibitor of Formula (2) (1 or 5 mg/kg). In mice given combination therapy, clinical arthritis scores were significantly reduced on Days 25-27 in mice treated with 0.3 mg/kg MTX + 1 mg/kg the BTK inhibitor of Formula (2) as compared to treatment with 1 mg/kg the BTK inhibitor of Formula (2) alone. Clinical arthritis scores were significantly reduced on Day 28 in mice treated with 0.3 mg/kg MTX + 5 mg/kg the BTK inhibitor of Formula (2) as compared to treatment with 5 mg/kg the BTK inhibitor of Formula (2) alone and on Days 30-43 as compared to treatment with 0.3 mg/kg MTX alone. Clinical arthritis scores were significantly reduced on Days 24-43 in mice treated with 0.5 mg/kg MTX + 1 mg/kg the BTK inhibitor of Formula (2) as compared to treatment with 1 mg/kg the BTK inhibitor of Formula (2) alone. Clinical arthritis scores were significantly reduced on Days 25-29 in mice treated with 0.5 mg/kg MTX + 5 mg/kg the BTK inhibitor of Formula (2) as compared to treatment with 5 mg/kg the BTK inhibitor of Formula (2) alone and on Days 30-43 as compared to treatment with 0.5 mg/kg MTX alone.

The daily arthritis score (average paw score) versus day following immunization are illustrated in FIG. 1, FIG. 2, FIG. 3, and FIG. 4.

The legends for each curve in FIG. 1 correspond to: black=vehicle; blue=MTX at 0.3 mg/kg; red=Formula (2) at 1 mg/kg; pink solid=MTX at 0.3 mg/kg and Formula (2) at 1 mg/kg; pink dashed=theoretical for MTX at 0.3 mg/kg and Formula (2) at 1 mg/kg based on data for MTX at 0.3 mg/kg and Formula (2) at 1 mg/kg.

The legends for each curve in FIG. 2 correspond to: black=vehicle; blue=MTX at 0.5 mg/kg; red=Formula (2) at 1 mg/kg; pink solid=MTX at 0.5 mg/kg and Formula (2) at 1 mg/kg; pink dashed=theoretical for MTX at 0.5 mg/kg and Formula (2) at 1 mg/kg based on data for MTX at 0.5 mg/kg and Formula (2) at 1 mg/kg.

The legends for each curve in FIG. 3 correspond to: black=vehicle; blue=MTX at 0.3 mg/kg; red=Formula (2) at 5 mg/kg; pink solid=MTX at 0.3 mg/kg and Formula (2) at 5 mg/kg; pink dashed=theoretical for MTX at 0.3 mg/kg and Formula (2) at 5 mg/kg based on data for MTX at 0.3 mg/kg and Formula (2) at 5 mg/kg.

The legends for each curve in FIG. 4 correspond to: black=vehicle; blue=MTX at 0.5 mg/kg; red=Formula (2) at 5 mg/kg; pink solid=MTX at 0.5 mg/kg and Formula (2) at 5 mg/kg; pink dashed=theoretical for MTX at 0.5 mg/kg and Formula (2) at 5 mg/kg based on data for MTX at 0.5 mg/kg and Formula (2) at 5 mg/kg.

The data of FIG. 1 to FIG. 4 illustrate that the combination of MTX and Formula (2) results in a reduction of disease incidence, evidenced by lower arthritis scores, compared to MTX and Formula (2) each alone. Furthermore, the data demonstrate a surprising synergistic effect between MTX and Formula (2). This is evidenced by the arthritis score for the combination, which was lower at each concentration combination when compared to the theoretical additive effect of the MTX and Formula (2) based on the data obtained for each active ingredient when separately administers.

Arthritis scores expressed as area under the curve (AUC) for Days 18-43 were significantly reduced as compared to vehicle controls in mice treated with 5 mg/kg the BTK inhibitor of Formula (2) (62% reduction), 0.3 mg/kg MTX + 1 mg/kg the BTK inhibitor of Formula (2) (53%), 0.5 mg/kg MTX + 1 mg/kg the BTK inhibitor of Formula (2) (60%), 0.3 mg/kg MTX + 5 mg/kg the BTK inhibitor of Formula (2) (78%), or 0.5 mg/kg MTX + 5 mg/kg the BTK inhibitor of Formula (2) (86%). Combination treatment with MTX + 1 mg/kg the BTK inhibitor of Formula (2) significantly reduced AUC as compared to treatment with 1 mg/kg the BTK inhibitor of Formula (2) alone, and treatment with MTX + 5 mg/kg the BTK inhibitor of Formula (2) significantly reduced AUC as compared to treatment with MTX (0.3 or 0.5 mg/kg) alone. Results of treatment on clinical scores AUC for Days 28-43 were mostly similar although reductions from treatment with 0.3 mg/kg MTX + 1 mg/kg the BTK inhibitor of Formula (2) were not significant as compared to treatment with 1 mg/kg the BTK inhibitor of Formula (2) alone.

Disease incidence was reduced in mice treated with 5 mg/kg the BTK inhibitor of Formula (2) (42% incidence at termination), 0.3 mg/kg MTX + 1 mg/kg the BTK inhibitor of Formula (2) (83%), 0.5 mg/kg MTX + 1 mg/kg the BTK inhibitor of Formula (2) (75%), 0.3 mg/kg MTX + 5 mg/kg the BTK inhibitor of Formula (2) (42%), or 0.5 mg/kg MTX + 5 mg/kg the BTK inhibitor of Formula (2) (17%) as compared to vehicle controls, which had 100% incidence by Day 28.

The morphologic pathology results are as follows. Vehicle control and Isotype Control animals had histopathology changes, consistent with those seen in type II collagen-induced arthritis, in most joints, with scores ranging from minimal to severe. Microscopic alteration included infiltration of synovium and periarticular tissue with neutrophils and mononuclear inflammatory cells (inflammation), marginal zone pannus and bone resorption and cartilage damage (proteoglycan loss, chondrocyte death and collagen matrix destruction).

All six-joint mean histopathology parameters were significantly reduced in mice treated with 5 mg/kg the BTK inhibitor of Formula (2) (63-82% reductions), 0.3 mg/kg MTX + 1 mg/kg the BTK inhibitor of Formula (2) (55-72%), 0.5 mg/kg MTX + 1 mg/kg the BTK inhibitor of Formula (2) (66-81%), 0.3 mg/kg MTX + 5 mg/kg the BTK inhibitor of Formula (2) (83-97%), or 0.5 mg/kg MTX + 5 mg/kg the BTK inhibitor of Formula (2) (83-96%) as compared to vehicle controls. In mice given combination therapy, all six-joint mean histopathology parameters were significantly reduced in mice treated with 0.5 mg/kg MTX + 1 mg/kg the BTK inhibitor of Formula (2) as compared to treatment with 1 mg/kg the BTK

inhibitor of Formula (2) alone and in mice treated with 0.3 mg/kg MTX + 5 mg/kg the BTK inhibitor of Formula (2) as compared to treatment with 0.3 mg/kg MTX alone. All six-joint mean parameters except cartilage damage scores were significantly reduced in mice treated with 0.5 mg/kg MTX + 5 mg/kg the BTK inhibitor of Formula (2) as compared to treatment with 0.5 mg/kg MTX alone.

Summed six-joint mean histopathology scores were significantly reduced as compared to vehicle controls in mice treated with 5 mg/kg the BTK inhibitor of Formula (2) (73% reduction), 0.3 mg/kg MTX + 1 mg/kg the BTK inhibitor of Formula (2) (64%), 0.5 mg/kg MTX + 1 mg/kg the BTK inhibitor of Formula (2) (74%), 0.3 mg/kg MTX + 5 mg/kg ACP-196 (91%), or 0.5 mg/kg MTX + 5 mg/kg the BTK inhibitor of Formula (2) (88%). Combination treatment with 0.5 mg/kg MTX + 1 mg/kg the BTK inhibitor of Formula (2) significantly reduced AUC as compared to treatment with 1 mg/kg the BTK inhibitor of Formula (2) alone, and treatment with MTX + 5 mg/kg the BTK inhibitor of Formula (2) significantly reduced AUC as compared to treatment with MTX (0.3 or 0.5 mg/kg) alone.

All paw histopathology parameters were significantly reduced in mice treated with 5 mg/kg the BTK inhibitor of Formula (2) (59-82% reductions), 0.3 mg/kg MTX + 1 mg/kg the BTK inhibitor of Formula (2) (53-72%), 0.5 mg/kg MTX + 1 mg/kg the BTK inhibitor of Formula (2) (61-79%), 0.3 mg/kg MTX + 5 mg/kg the BTK inhibitor of Formula (2) (86-97%), or 0.5 mg/kg MTX + 5 mg/kg the BTK inhibitor of Formula (2) (83-97%) as compared to vehicle controls. In mice given combination therapy, all paw histopathology parameters were significantly reduced in mice treated with 0.3 mg/kg MTX + 5 mg/kg the BTK inhibitor of Formula (2) as compared to treatment with 0.3 mg/kg MTX alone and in mice treated with 0.5 mg/kg MTX + 5 mg/kg the BTK inhibitor of Formula (2) as compared to treatment with 0.5 mg/kg MTX alone. All paw parameters except periosteal bone formation scores were significantly reduced in mice treated with 0.5 mg/kg MTX + 1 mg/kg the BTK inhibitor of Formula (2) as compared to treatment with 1 mg/kg the BTK inhibitor of Formula (2) alone.

Summed paw histopathology scores were significantly reduced as compared to vehicle controls in mice treated with 5 mg/kg the BTK inhibitor of Formula (2) (72% reduction), 0.3 mg/kg MTX + 1 mg/kg the BTK inhibitor of Formula (2) (64%), 0.5 mg/kg MTX + 1 mg/kg the BTK inhibitor of Formula (2) (72%), 0.3 mg/kg MTX + 5 mg/kg the BTK inhibitor of Formula (2) (93%), or 0.5 mg/kg MTX + 5 mg/kg the BTK inhibitor of Formula (2) (90%). Combination treatment with 0.5 mg/kg MTX + 1 mg/kg the BTK inhibitor of Formula (2) significantly reduced AUC as compared to treatment with 1 mg/kg the BTK inhibitor of Formula (2) alone, and treatment with MTX + 5 mg/kg the BTK inhibitor of Formula (2) significantly reduced AUC as compared to treatment with MTX (0.3 or 0.5 mg/kg) alone.

Mice treated with 5 mg/kg the BTK inhibitor of Formula (2) had significantly reduced knee inflammation (72% reduction), pannus formation (85%), and bone resorption (90%) as compared to vehicle controls. Mice treated with 0.3 mg/kg MTX + 1 mg/kg the BTK inhibitor of Formula (2) had significantly reduced knee inflammation (60%) and cartilage damage (61%). Mice treated with 0.5 mg/kg MTX + 1 mg/kg the BTK inhibitor of Formula (2) had significantly reduced knee inflammation (80%), pannus formation (92%), cartilage damage (83%), and bone resorption (92%). Mice treated with 0.3 mg/kg MTX + 5 mg/kg ACP- 196 had significantly reduced knee inflammation (77%), pannus formation (98%), cartilage damage (82%), and bone resorption (98%). Mice treated with 0.5 mg/kg MTX + 5 mg/kg ACP- 196 had significantly reduced knee inflammation (84%), pannus formation (85%), cartilage damage (81%), and bone resorption (88%). In mice given combination therapy, periosteal bone formation scores were significantly reduced in mice treated with 0.3 mg/kg MTX + 1 mg/kg the BTK inhibitor of Formula (2) as compared to treatment with 1 mg/kg the BTK inhibitor of Formula (2) alone. All knee parameters were significantly reduced in mice treated with 0.5 mg/kg MTX + 1 mg/kg the BTK inhibitor of Formula (2) as compared to treatment with 1 mg/kg the BTK inhibitor of Formula (2) alone. Mice treated with 0.3 mg/kg MTX + 5 mg/kg the BTK inhibitor of Formula (2) had significantly reduced knee pannus formation, bone resorption, and periosteal bone formation as compared to treatment with 0.3 mg/kg MTX alone.

Summed knee histopathology scores were significantly reduced as compared to vehicle controls in mice treated with 5 mg/kg the BTK inhibitor of Formula (2) (74% reduction), 0.3 mg/kg MTX + 1 mg/kg the BTK inhibitor of Formula (2) (64%), 0.5 mg/kg MTX + 1 mg/kg the BTK inhibitor of Formula (2) (84%), 0.3 mg/kg MTX + 5 mg/kg the BTK inhibitor of Formula (2) (84%), or 0.5 mg/kg MTX + 5 mg/kg the BTK inhibitor of Formula (2) (84%). Combination treatment with 0.5 mg/kg MTX + 1 mg/kg the BTK inhibitor of Formula (2) significantly reduced AUC as compared to treatment with 1 mg/kg the BTK inhibitor of Formula (2) alone.

Paw and six-joint mean periosteal bone widths were significantly reduced as compared to vehicle controls in mice treated 5 mg/kg the BTK inhibitor of Formula (2) (82-83% reductions), 0.3 mg/kg MTX + 1 mg/kg the BTK inhibitor of Formula (2) (68-69%), 0.5 mg/kg MTX + 1 mg/kg the BTK inhibitor of Formula (2) (80%), 0.3 mg/kg MTX + 5 mg/kg the BTK inhibitor of Formula (2) (97%), or 0.5 mg/kg MTX + 5 mg/kg the BTK inhibitor of Formula (2) (93-95%). In mice given combination therapy, knee periosteal bone widths were significantly reduced in mice treated with 0.3 mg/kg MTX + 1 mg/kg the BTK inhibitor of Formula (2) as compared to treatment with 1 mg/kg the BTK inhibitor of Formula (2) alone. Paw, knee, and six-joint mean periosteal bone widths were significantly reduced in mice treated with 0.5 mg/kg MTX + 1 mg/kg the BTK inhibitor of Formula (2) as compared to treatment with 1 mg/kg the BTK inhibitor of Formula (2) alone. Paw, knee, and six-joint mean periosteal bone widths were significantly reduced in mice treated with 0.3 mg/kg MTX + 5 mg/kg the BTK inhibitor of Formula (2) as compared to treatment with 0.3 mg/kg MTX alone. Paw and six-joint mean periosteal bone widths were significantly reduced in mice treated with 0.5 mg/kg MTX + 5 mg/kg the BTK inhibitor of

Formula (2) as compared to treatment with 0.5 mg/kg MTX alone.

Following euthanasia, splenocytes were collected and analyzed for BTK occupancy. The results show that Btk occupancy with the BTK inhibitor of Formula (2) is the same in the presence or absence of MTX. This means that there is a synergistic effect between the combination of the BTK inhibitor of Formula (2) and MTX and the behavior of the drug combination is not due to MTX affecting exposure/clearance of the BTK inhibitor of Formula (2).

Example 2 - Evaluation of Synergy in Combinations of the BTK Inhibitor of Formula (2) and MTX

Combination experiments as described above were performed to determine the synergistic, additive, or antagonistic behavior of drug combinations of BTK Inhibitor of Formula (2) and MTX. Synergistic effects of combination therapy can be evaluated using the Bliss independence or fractional product method as reported in Yan et al., BMC Syst Biol., 2010, 4, 50. The Bliss independence model is defined by the equation Exy = Ex + Ey - (ExEy), where (Exy) is the additive effect of drugs x and y as predicted by their individual effects (Ex and Ey). The Bliss independence method assumes that the two inhibitors act via independent mechanisms. If the actual combined effect of the two inhibitors is equal to Exy, it is an additive effect case and there is no interaction between the two inhibitors. If the actual combined effect is lower than Exy, it is called antagonism. If the actual combined effect is higher than Exy, it is called synergism. Yan et al., BMC Syst Biol., 2010, 4, 50. Combination therapy at all doses resulted in synergistic effects on arthritis scores AUC (Days 28-43) and summed histopathology scores (all joints) as indicated by evaluation of theoretically predicted (expected) and experimentally observed inhibition of score increase.

Combination therapy at all doses resulted in synergistic effects on arthritis scores AUC (Days 28-43) and summed histopathology scores (all joints) as indicated by evaluation of expected inhibition (*i.e.*, theoretically predicted) and experimentally observed inhibition of score increase, as shown in Table 5.

TABLE 5. Theoretical and Observed Synergistic Effects of Combination Therapy

| | | **Clinical Arthritis Score AUC Day 28-43** | | **Histopathology Summer Scores (All Joints)** | |
|---|---|---|---|---|---|
| **Group** | **Treatment** | **Expected Inhibition (%)** | **Observed Inhibition (%)** | **Expected Inhibition (%)** | **Observed Inhibition (%)** |
| 7 | MTX (0.3 mg/kg) PO, QD (d18-43) + BTK inhibitor of formula (2) (1 mg/kg) PO, QD (d28-43) | 39% | 53% | 50% | 64% |
| 8 | MTX (0.5 mg/kg) PO, QD (d18-43) + BTK inhibitor of Formula (2) (1 mg/kg) PO, QD (d28-43) | 43% | 58% | 59% | 74% |
| 9 | MTX (0.3 mg/kg) PO, QD (d18-43) + BTK inhibitor of formula (2) (5 mg/kg) PO, QD (d28-43) | 76% | 81% | 82% | 91% |
| 10 | MTX (0.5 mg/kg) PO, QD (d18-43) + BTK inhibitor of formula (2) (5 mg/kg) PO, QD (d28-43) | 78% | 88% | 85% | 88% |

Example 3 - A Phase 2A, 4-Week, Double-Blind, Proof-of-Concept Efficacy and Safety Study of the BTK Inhibitor of Formula (2) Versus Placebo in Subjects with Active Rheumatoid Arthritis on Background Methotrexate

The primary objective of the study will be to evaluate the efficacy of the BTK inhibitor of Formula (2) in subjects with active rheumatoid arthritis ("RA") despite treatment with methotrexate ("MTX"). The secondary objectives will be to: (i) evaluate the safety and tolerability of Formula (2) when coadministered with methotrexate ("MTX") in subjects with active RA; (ii) evaluate the PK and PD of Formula (2) with coadministration with MTX; and (iii) assess the effect of Formula (2) coadministration with MTX on various immune biomarkers.

The current treatment algorithm for RA includes first treatment with NSAIDS, followed by synthetic DMARDS, such as MTX, once NSAIDS become ineffective. Upon failure to respond to DMARDS and combinations thereof, patients are then treated with anti-tumor necrosis factor (anti-TNF) biologics. Ultimately when patients no longer respond to anti-TNF biologics, administration of rituximab (anti-CD20) or abatacept (CTLA4-Ig) is started. Currently, drug development

of oral tyrosine kinase inhibitors for the treatment of RA is focusing on producing molecules with a better efficacy/safety profile than MTX and a similar efficacy/safety profile to biologics.

The efficacy of rituximab in treatment of patients with RA, relapsing remitting multiple sclerosis, systemic lupus erythematosus, or Sjögren's syndrome has validated B cells as an important target in autoimmune disorders. BTK is a Tec family non-receptor protein kinase, expressed in B cells, myeloid cells, osteoclasts, mast cells and platelets. The function of BTK in signaling pathways activated by the engagement of the BCR has been well established. Buggy, et al., Int. Rev. Immunol. 2012, 31, 119-132. The efficacy of BTK inhibition in other B cell driven diseases, such as B cell malignancies, is represented by the recent approval of ibrutinib (IMBRUYICA™), the first generation BTK inhibitor approved for the treatment of CLL and mantle cell lymphoma. However, the potential benefit of BTK inhibition for the treatment of autoimmune disorders is not limited to its effect on B cell activation. BTK is involved in several biologic processes, many of which affect disease progression in autoimmune disorders. BTK regulates FcγR signaling in myeloid cells and in mast cells it plays a key role in mast cell degranulation following FcεR1 activation. Jongstra-Bilen, et al., J Immunol. 2008, 181, 288-298; Ellmeier, et al., FEBS J. 2011, 278, 1990-2000. BTK regulates RANKL-induced osteoclast differentiation. Shinohara, et al., Cell 2008, 132, 794-806. BTK regulates TLR signaling and BTK inhibition is able to block B-cell activation when B-cells are stimulated via the BCR and TLR9. Kenny, et al., PLoS One, 2013, 8, e74103. BTK affects BCR-induced secretion of pro-inflammatory cytokines and chemokines by B-cells. de Rooij, et al., Blood, 2012, 119, 2590-2594; di Paolo, et al., Nat. Chem. Biol., 2011, 7, 41-50.

RA is a chronic autoimmune disease affecting 1 percent of the general population worldwide. It causes pain, stiffness, swelling, and limitation in the motion and function of multiple joints. If left inadequately treated, RA can produce destruction of one or more joints leading to deformity and permanent disability. As mentioned above, the treatment of RA has traditionally included NSAIDS, corticosteroids, and DMARDS. While these therapies provide some benefit, their efficacy has been limited. Newer, biologically based therapies include molecules that inhibit cytokine activity (TNF inhibitors, IL-1 Ra, or anti-IL-6 R mAb), block T cell-mediated co-stimulation (abatacept), or deplete B cells (rituximab). Newer generation kinase inhibitors (tofacitinib) have provided comparable benefits as the biologically based therapies. These therapies have been effective for moderate-to-severe RA and have slowed disease progression, as determined radiographically, particularly when combined with MTX. However, subjects using these therapies still routinely fail to achieve a response ≥ 50%. Additionally, these therapies can be associated with unfavorable side effects such as increased risk of serious infection.

A multicenter, randomized, double-blind, placebo-controlled, parallel-group clinical trial will be conducted. The on-treatment period will be four weeks with weekly visits to the clinic. There will be also a four-week safety follow-up period after the last dose of Formula (2) or placebo. Subjects meeting the eligibility criteria will be randomized in a 1:1 ratio. For four weeks, Treatment Group 1 will receive Formula (2) 15 mg once per day (QD) while on a stable dose of MTX between 7.5 and 25 mg/week. At the same time, Treatment Group 2 will receive placebo QD while on a stable dose of MTX between 7.5 and 25 mg/week. After the four weeks, enrollment will be paused while a data and safety monitoring board (DSMB) reviews unblinded pharmacokinetics and the safety results of the first 20 subjects treated. Following the review, 50 subjects will be randomized in a 1:1 ratio. Treatment Group 1 will receive Formula (2) 15 mg once per day (QD) while on a stable dose of MTX between 7.5 and 25 mg/week for four weeks. At the same time, Treatment Group 2 will receive placebo QD while on a stable dose of MTX between 7.5 and 25 mg/week for four weeks.

The inclusion criteria for patient eligibility are as follows. Subjects must meet the following criteria: (1) subjects must be able to read and understand the consent form, complete the study-related procedures, and communicate with the study staff; (2) subjects may be men or women and must be 18 to 75 years of age (inclusive); (3) subjects must have a diagnosis of RA according to the 2010 American College of Rheumatology/European League Against Rheumatism (ACR/EULAR) Classification Criteria for ≥ three months before screening, and not before the age of 16 years; (4) subjects with a diagnosis of RA before 2011 must meet ACR 1987 criteria for diagnosis of RA; (5) subjects must have active RA at the time of randomization where active RA is defined as: ≥ three swollen joints out of 28 joint count; ≥ three tender joints out of 28 joint count; > upper limit of normal (ULN) for CRP; ≥ one swollen joint must be in the proximal interphalangeal (PIP)/metacarpophalangeal (MCP)/wrist- where upper extremity with the greatest number of swollen joints in PIP/MCP/wrist will be defined as the index hand and be evaluated via MRI; (6) subjects must have started MTX treatment for ≥ three months before randomization and must be on a stable MTX dose (7.5 to 25 mg/week) for ≥ eight weeks before randomization and remain on a stable dose through the treatment period; (7) Sulfasalazine and hydroxychloroquine are allowed; however, subjects must be on stable dose for ≥ eight weeks before randomization and remain on a stable dose through the treatment period; (8) subjects must be on oral folic or folinic acid supplementation ≥ 5 mg/week; (9) if using oral corticosteroids, subjects must be on a stable dose equivalent to ≤ 10 mg of prednisone/day for ≥ 4 weeks before randomization and remain on a stable dose through the treatment period; (10) if using non-steroidal anti-inflammatory drugs (NSAIDS), subjects must be on a stable dose for ≥ 2 weeks before randomization and remain on a stable dose through the treatment period; (11) subjects must have screening laboratory test result as follows: (i) Hemoglobin ≥ 8.5 g/dL (International System of Units [SI]: ≥ 85 g/L); (ii) White blood cells (WBC) ≥ 3.0 x $10^3$ cells/μL (SI: ≥ 3.0 x $10^9$ cells/L); (iii) Neutrophils ≥ 1.5 x 103 cells/μL; (SI: ≥ 1.5 x109 cells/L); (iv) Platelets ≥ 100 x $10^3$ cells/μL (SI: ≥ 100 x $10^9$

cells/L); (v) Serum transaminase levels not exceeding 2.5 x ULN; an d(vi) Serum creatinine not exceeding 1.5 mg/dL\ (SI: ≤ 25 μmol/L); (12) agreement to use acceptable forms of contraception during the study and for a minimum of 90 days after the last dose of MTX or 30 days after the last dose of Formula (2) /placebo, whichever is longer, if sexually active and able to bear or beget children. Examples of acceptable methods of contraception include condoms, implants, injectables, combined oral contraceptives, intrauterine devices, true sexual abstinence, or sterilized partner. Note that periodic abstinence (eg, calendar, ovulation, symptothermal, postovulation methods or withdrawal) are not acceptable methods of contraception; (13) women of child bearing potential who are sexually active with a male partner must agree to simultaneously use two forms of acceptable methods of contraception (eg, condom and with contraceptives) while on the study and for 30 days after the last dose of Formula (2) /placebo; (14) men must agree to refrain from sperm donation during the study and for 30 days after the last dose of Formula (2) /placebo; (15) are willing and able to adhere to the study visit schedule, and understand and comply with other protocol requirements.

The exclusion criteria for patient eligibility are: (1) prior malignancy, except for adequately treated basal cell or squamous cell skin cancer, in situ cervical cancer, or other cancer from which the subject has been disease free for ≥ 5 years; (2) evidence of tuberculosis (TB), as documented by a specific assay (purified protein derivative [PPD] or QuantiFERON®-TB Gold Test), medical history, or chest radiograph where exceptions include subjects who have documented treatment with isoniazid (INH) for ≥ 6 months, have completed treatment, and have no signs or symptoms of active TB; (3) body mass index (BMI) > 35 kg/m2; (4) subjects unable to ambulate (eg, confined to a bed or wheelchair-bound) or are ACR functional class IV; (5) exposed to a live vaccine within 2 months of randomization; (6) life-threatening illness, medical condition or organ system dysfunction which, in the investigator’s opinion, could compromise the subject’s safety, interfere with the absorption or metabolism of Formula (2), or put the study outcomes at undue risk; (7) any condition that could affect Formula (2) absorption, including gastric restrictions and bariatric surgery, such as gastric bypass; (8) subjects who are pregnant, breast feeding, or planning a pregnancy (both men and women) within six months of randomization (9) subjects who have other inflammatory diseases that might confound the evaluations of benefit from Formula (2) therapy (including but not limited to systemic lupus erythematosus, inflammatory bowel disease, Felty’s syndrome, Lyme disease, psoriasis, or multiple sclerosis) with secondary Sjögren’s syndrome, asthma, or thyroid disease are acceptable; (10) subjects who have taken any investigational drug within the previous 30 days before randomization; (11) subjects with contraindications to whole-body MRI; (12) subjects with acute or chronic severe renal insufficiency (glomerular filtration rate [GFR] < 30 mL/min/1.73$m^2$); (13) any prior BTK therapy; (14) prior nonresponse to a biologic agent or Janus kinase (JAK) inhibitor; (15) use of all other synthetic disease-modifying antirheumatic drugs (DMARDS) such as but not limited to leflunomide, azathioprine, cyclosporine, penicillamine or gold salts within eight weeks of randomization; (15) use of etanercept, anakinra, tofacitinib within four weeks of randomization; (16) use of abatacept, humira, infliximab, or tocilizumab within eight weeks of randomization; (17) subjects who have, or have had, a serious infection during the previous eight weeks before randomization (including, but not limited to, hepatitis, pneumonia, cellulitis, herpes zoster, or pyelonephritis), or have been hospitalized and/or received intravenous (IV) antibiotics for an infection; (18) known history of human immunodeficiency virus (HIV) or hepatitis B virus (HBV) or active infection with hepatitis C virus (HCV) (19) subjects with current signs or symptoms of clinically significant, progressive, or uncontrolled renal, hepatic, hematologic, gastrointestinal, endocrine, pulmonary, cardiac, neurologic, or psychiatric disease; (20) major surgery within 4 weeks before randomization or planned elective surgery during the study duration; (21) history of stroke, intracranial hemorrhage, or myocardial infarction within 6 months before randomization; (22) subjects requiring anticoagulation with warfarin or a vitamin K antagonist; (23) subjects who have, or have had, a substance abuse (drug, chemical, or alcohol) problem within the previous 2 years; (24) subjects who are unable to undergo multiple venipunctures because of poor tolerability or lack of easy venous access; and (25) concurrent participation in another therapeutic clinical trial.

The following efficacy parameters will be used to assess the participants. At the primary endpoint: disease activity score 28 C-reactive protein ("DAS28-CRP") will be accessed at week four. Secondary Endpoints will be assessed as follows: (i) DAS28-CRP at weeks 1, 2, and 3; (ii) American College of Rheumatology ("ACR") ACR20 at weeks 1, 2, 3, 4; (iii) ACR50 at weeks 1, 2, 3, 4; (iv) ACR70 at weeks 1, 2, 3, and 4; (v) individual ACR domains at weeks 1, 2, 3, and 4 such as: swollen joint count ("SJC"), tender joint count ("TJC"), health assessment questionnaire disability index ("HAQ-DI"), physician’s global assessment, subject’s global assessment, subject’s assessment of pain, C-reactive protein ("CRP"), erythrocyte sedimentation rate ("ESR"), ACR-N at weeks 1, 2, 3, and 4, clinical disease activity index ("CDAI") at weeks 1, 2, 3, and 4, SDAI at weeks 1, 2, 3, and 4.

Magnetic resonance imaging (MRI) of the index hand may also be evaluated using the rheumatoid arthritis MRI scoring system ("RAMRIS") between baseline and week 4. A MRI will be performed on the most severely involved hand and wrist at baseline and week 4. Three dimensional GRE with and without gadolinium contrast and STIR images will be acquired. The MRI images will be centrally read by two experienced, independent radiologists who will be blinded to treatment assignment and the sequence of the images. Images will be scored using standard RAMRIS method.

Pharmacokinetic and pharmacodynamic parameters for Formula (2) and MTX and its metabolite will be measured in plasma and urine. Pharmacokinetic parameters will be measured in a subset of subjects (N=20) at select study

centers. The plasma and urine PK of Formula (2), MTX, and 7-hydroxymethotrexate (a metabolite of MTX) will be characterized using non compartmental analysis. The following pharmacokinetic parameters will be calculated, whenever possible, from plasma and/or urine concentrations of analytes: (i) AUC0-last: Area under the plasma concentration-time curve calculated using linear trapezoidal summation from time zero to time last, where "last" is the time of the last measurable concentration; (ii) AUC0-24: Area under the plasma concentration-time curve from zero to 24 hours, calculated using linear trapezoidal summation; (iii) AUC0-inf: Area under the plasma concentration-time curve from zero to infinity, calculated using the formula: AUC0-inf = AUC0-last + Ct / $\lambda z$, where $\lambda z$ is the apparent terminal elimination rate constant; (iv) Cmax: Maximum observed plasma concentration: (iv) Tmax: Time to maximum drug concentration (obtained without interpolation); and (v) t½: Terminal elimination half-life (whenever possible).

Pharmacodynamic parameters will be measured for the occupancy of BTK by Formula (2) in PBMCs with the aid of a biotin-tagged Formula (2) analogue probe. The effect of Formula (2) on biologic markers of B-cell function will also be evaluated, where the biomarker parameters include: (i) matrix metalloproteinase-3 (MMP3): (ii) interleukin-6 (IL-6); (iii) interleukin-8 (IL-8); (iv) C-terminal cross-linked telopeptide (CTX) and N-terminal cross-linked telopeptide (NTX); (v) rheumatoid factor (RF); (vi) osteocalcin; (vii) cartilage oligomeric matrix protein (COMP); and (viii) bone alkaline phosphatase (BAP).

## Claims

**1.** A combination comprising a BTK inhibitor having structure of Formula (2):

Formula (2)

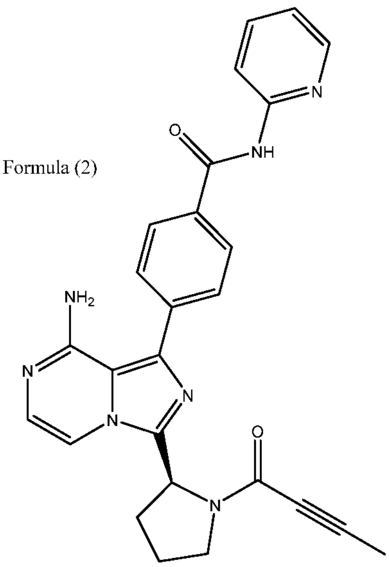

or a pharmaceutically acceptable salt thereof, and methotrexate or a pharmaceutically acceptable salt thereof for use in the treatment of rheumatoid arthritis.

**2.** A composition comprising therapeutically effective amounts of:

(1) a BTK inhibitor having structure of Formula (2):

Formula (2)

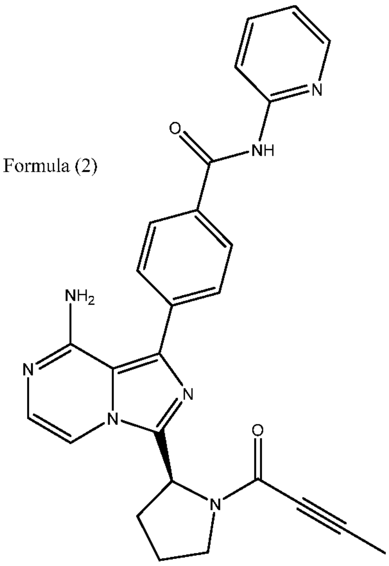

or a pharmaceutically acceptable salt thereof, and
(2) methotrexate or a pharmaceutically acceptable salt thereof,
for use in the treatment of rheumatoid arthritis.

**3.** A kit comprising:

a pharmaceutical composition comprising a BTK inhibitor having structure of Formula (2):

Formula (2)

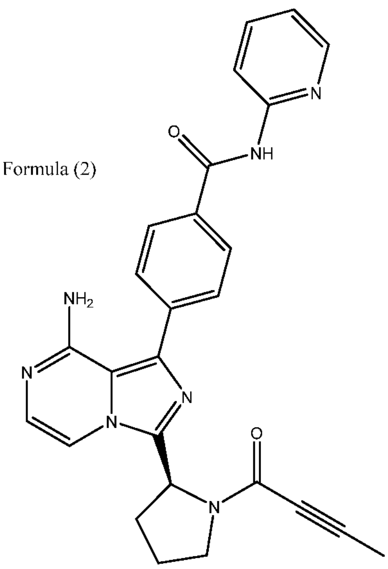

or a pharmaceutically acceptable salt thereof, and
a pharmaceutical composition comprising methotrexate or a pharmaceutically acceptable salt thereof,
for co-administration of the methotrexate and the compound of formula (2), either simultaneously or separately,
for use in the treatment of rheumatoid arthritis.

**Patentansprüche**

**1.** Kombination, umfassend einen BTK-Inhibitor mit der Struktur der Formel (2):

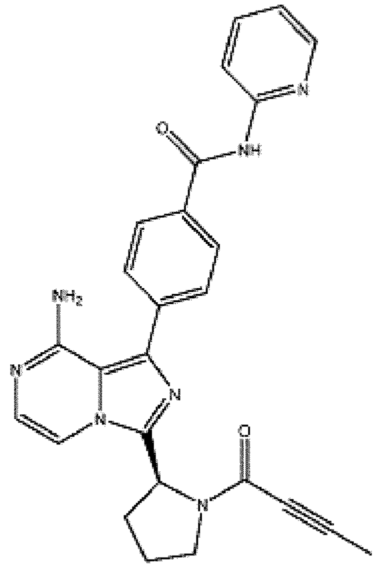

Formel (2)

oder ein pharmazeutisch unbedenkliches Salz davon und Methotrexat oder ein pharmazeutisch unbedenkliches Salz davon zur Verwendung bei der Behandlung von rheumatoider Arthritis.

**2.** Zusammensetzung, umfassend therapeutisch wirksame Mengen von

(1) einem BTK-Inhibitor mit der Struktur der Formel (2):

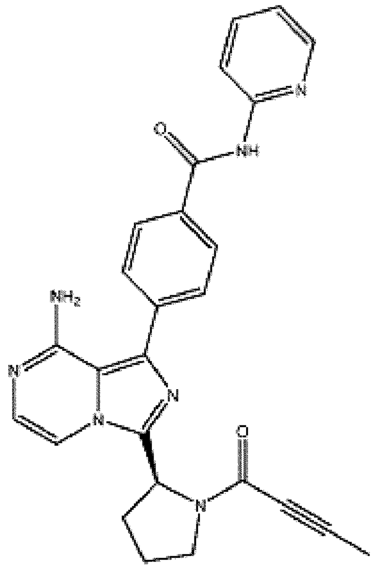

Formel (2)

oder einem pharmazeutisch unbedenklichen Salz davon und
(2) Methotrexat oder einem pharmazeutisch unbedenklichen Salz davon,
zur Verwendung bei der Behandlung von rheumatoider Arthritis.

**3.** Kit, umfassend:

eine pharmazeutische Zusammensetzung, umfassend einen BTK-Inhibitor mit der Struktur der Formel (2) :

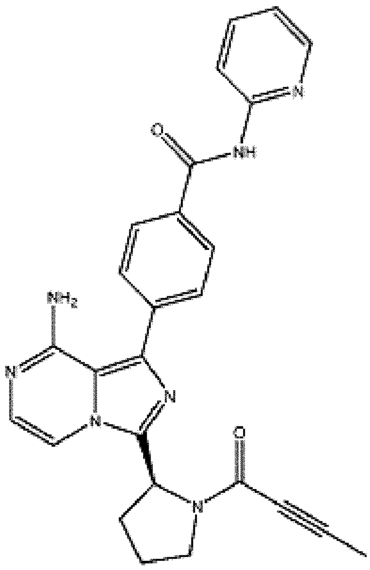

Formel (2)

oder ein pharmazeutisch unbedenkliches Salz davon, und
eine pharmazeutische Zusammensetzung, umfassend Methotrexat oder ein pharmazeutisch unbedenkliches Salz davon,
zur gemeinsamen Verabreichung des Methotrexats und der Verbindung der Formel (2), entweder gleichzeitig oder separat,
zur Verwendung bei der Behandlung von rheumatoider Arthritis.

**Revendications**

1. Combinaison comprenant un inhibiteur de BTK répondant à la structure de Formule (2) :

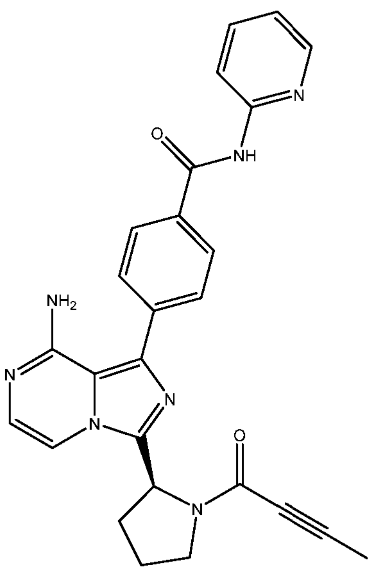

Formule (2)

ou un sel pharmaceutiquement acceptable de celui-ci, et du méthotrexate ou un sel pharmaceutiquement acceptable de celui-ci pour utilisation dans le traitement de la polyarthrite rhumatoïde.

2. Composition comprenant des quantités thérapeutiquement actives :

(1) d'un inhibiteur de BTK répondant à la structure de Formule (2) :

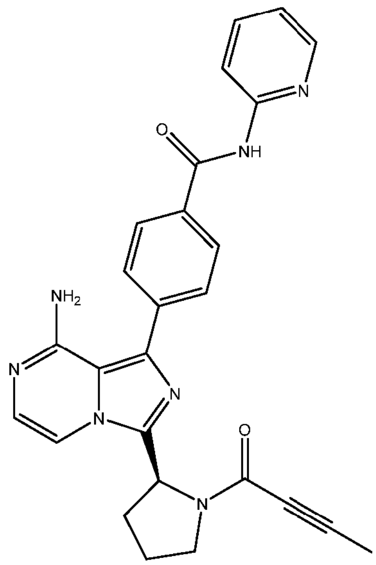

Formule (2)

ou d'un sel pharmaceutiquement acceptable de celui-ci, et
(2) de méthotrexate ou d'un sel pharmaceutiquement acceptable de celui-ci,
pour utilisation dans le traitement de la polyarthrite rhumatoïde.

**3.** Coffret comprenant :

une composition pharmaceutique comprenant un inhibiteur de BTK répondant à la structure de Formule (2) :

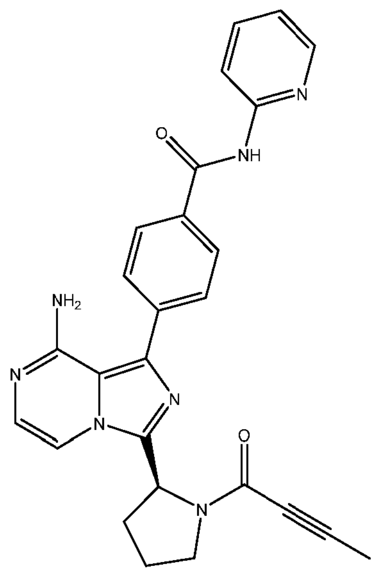

Formule (2)

ou un sel pharmaceutiquement acceptable de celui-ci, et
une composition pharmaceutique comprenant du méthotrexate ou un sel pharmaceutiquement acceptable de celui-ci,
pour co-administration du méthotrexate et du composé de formule (2), soit simultanément soit séparément,
pour utilisation dans le traitement de la polyarthrite rhumatoïde.

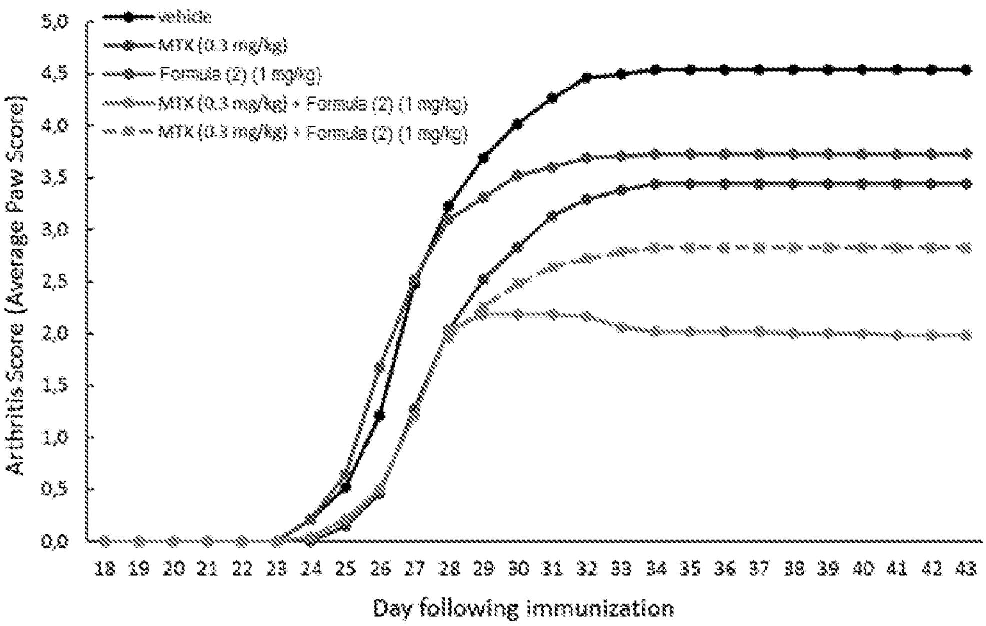

vehicle
MTX (0.3 mg/kg)
Formula (2) (1 mg/kg)
MTX (0.3 mg/kg) + Formula (2) (1 mg/kg)
MTX (0.3 mg/kg) + Formula (2) (1 mg/kg)
Arthritis Score (Average Paw Score)
5,0
4,5
4,0
3,5
3,0
2,5
2,0
1,5
1,0
0,5
0,0
18 19 20 21 22 23 24 25 26 27 28 29 30 31 32 33 34 35 36 37 38 39 40 41 42 43
Day following immunization


FIG. 1

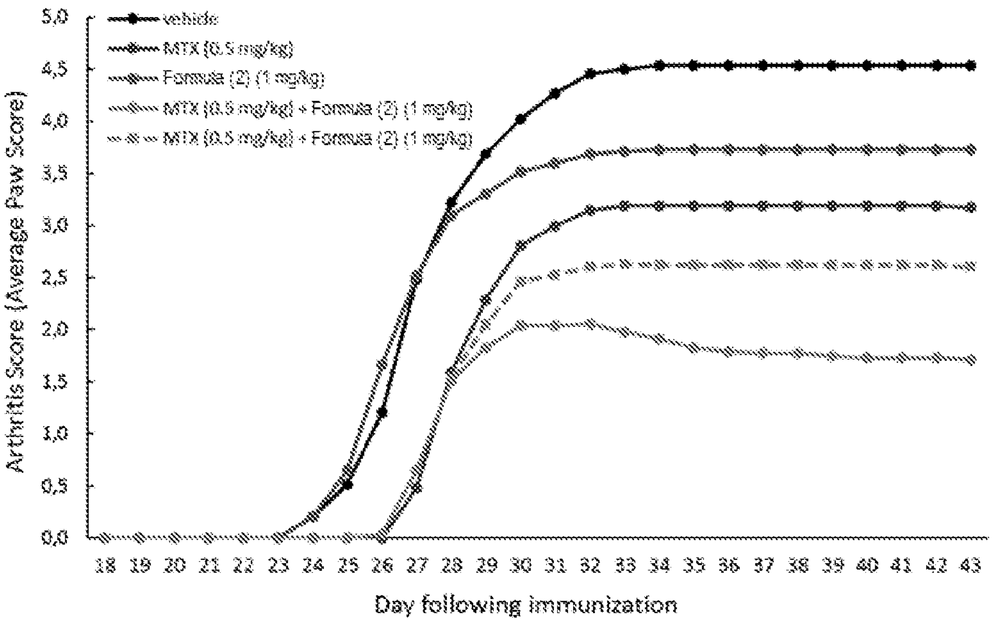
vehicle
MTX (0.5 mg/kg)
Formula (2) (1 mg/kg)
MTX (0.5 mg/kg) + Formula (2) (1 mg/kg)
MTX (0.5 mg/kg) + Formula (2) (1 mg/kg)
Arthritis Score (Average Paw Score)
5,0
4,5
4,0
3,5
3,0
2,5
2,0
1,5
1,0
0,5
0,0
18 19 20 21 22 23 24 25 26 27 28 29 30 31 32 33 34 35 36 37 38 39 40 41 42 43
Day following immunization

FIG. 2

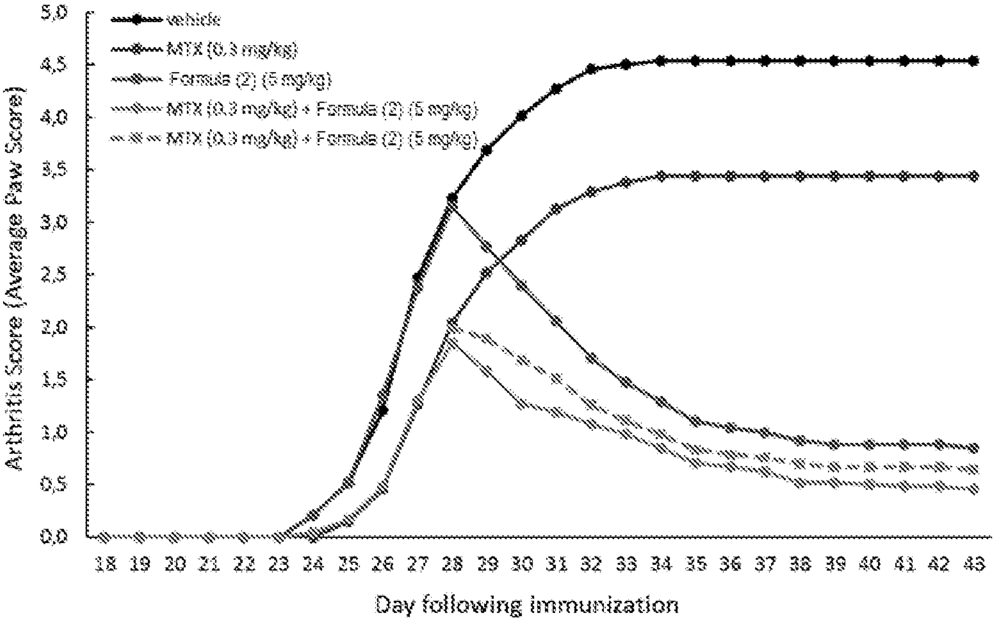
vehicle
MTX (0.3 mg/kg)
Formula (2) (5 mg/kg)
MTX (0.3 mg/kg) + Formula (2) (5 mg/kg)
MTX (0.3 mg/kg) + Formula (2) (5 mg/kg)
Arthritis Score (Average Paw Score)
5,0
4,5
4,0
3,5
3,0
2,5
2,0
1,5
1,0
0,5
0,0
18 19 20 21 22 23 24 25 26 27 28 29 30 31 32 33 34 35 36 37 38 39 40 41 42 43
Day following immunization

FIG. 3

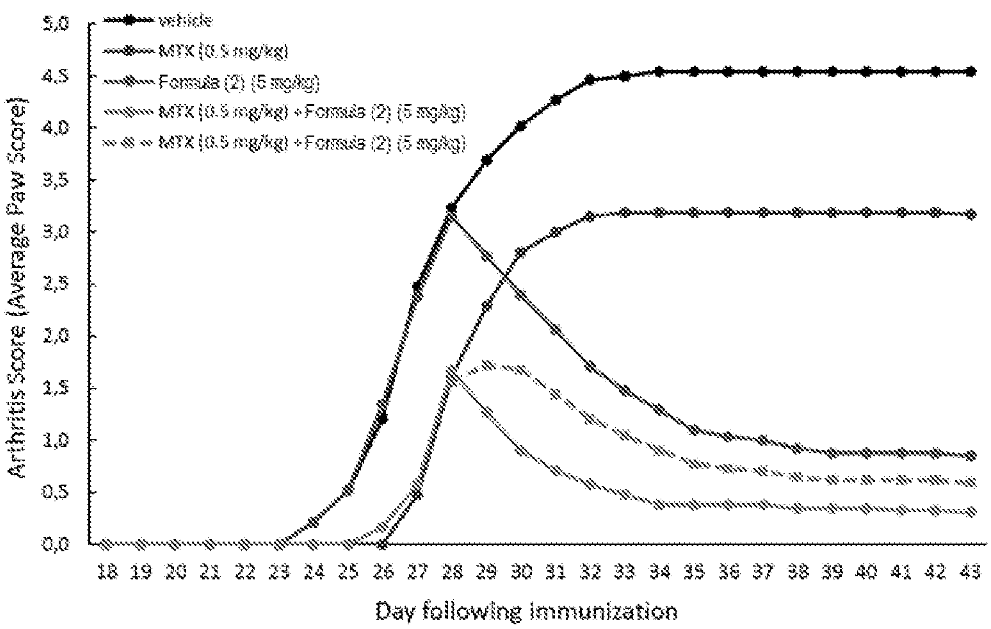
vehicle
MTX (0.5 mg/kg)
Formula (2) (5 mg/kg)
MTX (0.5 mg/kg) + Formula (2) (5 mg/kg)
MTX (0.5 mg/kg) + Formula (2) (5 mg/kg)
Arthritis Score (Average Paw Score)
5,0
4,5
4,0
3,5
3,0
2,5
2,0
1,5
1,0
0,5
0,0
18 19 20 21 22 23 24 25 26 27 28 29 30 31 32 33 34 35 36 37 38 39 40 41 42 43
Day following immunization

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 404097 A
- WO 9311161 A
- US 5714350 A
- US 6350861 B
- US 20040110704 A
- EP 1176195 A
- WO 03035835 A
- WO 9954342 A
- EP 0154316 A
- EP 0401384 A
- WO 2013010868 A
- US 20140155385 A1
- US 2512572 A
- US 5023252 A
- US 4992445 A
- US 5001139 A
- WO 2009088986 A
- WO 2009088880 A
- WO 2011008302 A
- US 8529202 B2
- WO 2006121168 A
- US 8008449 B
- US 20090217401 A1
- US 20130133091 A1
- WO 0109187 A
- US 5545806 A
- US 5625825 A
- US 5545807 A
- WO 0243478 A
- WO 9824884 A
- WO 2008156712 A1
- US 8354509 B
- US 20100266617 A1
- US 20130108651 A1
- US 20130109843 A2
- US 8686119 B2
- US 8287856 B
- US 8580247 B
- US 8168757 B
- US 20090028857 A1
- US 20100285013 A1
- US 20130022600 A1
- US 20110008369 A1
- US 8735553 B1
- US 8907053 B
- US 9096642 B
- US 9044442 B
- US 20150087581 A
- US 20150073024
- US 20150073042 A
- US 20150125491 A
- WO 2015033301 A
- WO 2015036927 A
- WO 201504490 A
- US 20140294898 A
- US 8779108 B
- US 20130034559
- US 20130034559 A
- US 20130034559 A1
- US 8217149 B
- US 20100203056 A1
- US 20130045200 A1
- US 20130045201 A1
- US 20130045202 A1
- US 20140065135 A1
- US 20140341917 A1
- US 7943743 B2


### Non-patent literature cited in the description


- **D'CRUZ ; UCKUN.** *OncoTargets and Therapy,* 2013, vol. 6, 161-176
- **BONNADONNA et al.** *J. Am. Med. Assoc.,* 1995, vol. 273, 542-547
- **BONNADONNA et al.** *N. Engl. J. Med.,* 1995, vol. 332, 901-906
- **HONG et al.** *Cancer,* 1983, vol. 52, 206-210
- **SHIH et al.** *Advan. Enzyme Regul.,* 1998, vol. 38, 135-152
- **SHIH et al.** *Cancer Res,* 1997, vol. 57, 1116-1123
- **XU, D. et al.** *J. Pharmacol. Exp. Th.,* 2012, vol. 341, 90-103
- **JACQUES et al.** Enantiomers, Racemates and Resolutions. Wiley Interscience, 1981
- **E. L. ELIEL.** Stereochemistry of Carbon Compounds. McGraw-Hill, 1962
- **E. L. ELIEL ; S. H. WILEN.** Stereochemistry of Organic Compounds. Wiley-Interscience, 1994
- **T. H. GREENE ; P. G. M. WUTS.** Protective Groups in Organic Synthesis. John Wiley & Sons, 1999

- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **BOLLIGER et al.** *Proc. Natl. Acad. Sci. USA,* 1993, vol. 90, 6444-6448
- **YAMANE-OHNUKI et al.** *Biotechnol. Bioeng.,* 2004, vol. 87, 614-622
- **SHIELDS et al.** *J. Biol. Chem.,* 2002, vol. 277, 26733-26740
- **UMANA et al.** *Nat. Biotech.,* 1999, vol. 17, 176-180
- **TARENTINO et al.** *Biochem.,* 1975, vol. 14, 5516-5523
- **BRUMMELL et al.** *Biochemistry,* 1993, vol. 32, 1180-1187
- **KOBAYASHI et al.** *Protein Eng. 1999,* 1999, vol. 12, 879-884
- **BURKS et al.** *Proc. Natl. Acad. Sci. USA,* 1997, vol. 94, 412-417
- Handbook of Clinical Drug Data. McGraw-Hill, 2002
- Principles of Drug Action. Churchill Livingston, 1990
- **WILLIAMS et al.** *Chem. Biol.,* 2010, vol. 17, 123-34
- **KLEIN et al.** *mAbs,* 2013, vol. 5, 22-33
- **RASTETTER et al.** *Ann. Rev. Med.,* 2004, vol. 55, 477-503
- **PLOSKER ; FIGGETT.** *Drugs,* 2003, vol. 63, 803-43
- **ROBAK.** *Curr. Opin. Investig. Drugs,* 2009, vol. 10, 588-96
- **CHESON.** *J. Clin. Oncol.,* 2010, vol. 28, 3525-30
- **DU et al.** *Mol. Immunol.,* 2009, vol. 46, 2419-2423
- **GOLDENBERG et al.** *Leuk. Lymphoma,* 2010, vol. 51, 747-55
- **KEIR et al.** *Annu. Rev. Immunol.,* 2008, vol. 26, 677-704
- **TOPALIAN et al.** *N. Eng. J. Med.,* 2012, vol. 366, 2443-54
- **WANG et al.** *Cancer Immunol Res.,* 2014, vol. 2, 846-56
- **PAGE et al.** *Ann. Rev. Med.,* 2014, vol. 65, 185-202
- **WEBER et al.** *J. Clin. Oncology,* 2013, vol. 31, 4311-4318
- **CHEN et al.** *EMBO J.,* 1993, vol. 12, 811-820
- **FISHWILD et al.** *Nat. Biotechnology,* 1996, vol. 14, 845-851
- **LONBERG et al.** *Nature,* 1994, vol. 368, 856-859
- **FUERST.** *Oncology Times,* 2014, vol. 36, 35-36
- **ROBERT et al.** *Lancet,* 2014, vol. 384, 1109-17
- **THOMAS et al.** *Exp. Opin. Biol. Ther.,* 2014, vol. 14, 1061-1064
- **BERGER et al.** *Clin. Cancer Res.,* 2008, vol. 14, 3044-51
- **BRAHMER et al.** *J. Clin. Oncol.,* 2014, vol. 32, 5s
- **MCDERMOTT et al.** *Cancer Treatment Rev.,* 2014, vol. 40, 1056-64
- **LORIES et al.** *Ann. Rheum. Dis.,* 2004, vol. 63, 595-598
- **YAN et al.** *BMC Syst Biol.,* 2010, vol. 4, 50
- **YAN et al.** *BMC Syst Biol.,* 2010, vol. 4, 50
- **BUGGY et al.** *Int. Rev. Immunol.,* 2012, vol. 31, 119-132
- **JONGSTRA-BILEN et al.** *J Immunol.,* 2008, vol. 181, 288-298
- **ELLMEIER et al.** *FEBS J.,* 2011, vol. 278, 1990-2000
- **SHINOHARA et al.** *Cell,* 2008, vol. 132, 794-806
- **KENNY et al.** *PLoS One,* 2013, vol. 8, e74103
- **ROOIJ et al.** *Blood,* 2012, vol. 119, 2590-2594
- **DI PAOLO et al.** *Nat. Chem. Biol.,* 2011, vol. 7, 41-50